(19) Europäisches Patentamt
European Patent Office
Office européen des brevets

(11) Veröffentlichungsnummer: **0 464 381 B1**

# (12) EUROPÄISCHE PATENTSCHRIFT

(45) Veröffentlichungstag der Patentschrift: **26.07.95**

(51) Int. Cl.[6]: **C07C 251/40**, A01N 37/50, C07C 259/14, C07D 239/60, C07C 323/62, C07C 255/57, C07D 241/46, C07D 213/79, C07C 255/54, C07D 317/54, C07D 239/34

(21) Anmeldenummer: **91109085.0**

(22) Anmeldetag: **04.06.91**

Die Akte enthält technische Angaben, die nach dem Eingang der Anmeldung eingereicht wurden und die nicht in dieser Patentschrift enthalten sind.

## (54) 2-Methoximinocarbonsäureester.

(30) Priorität: **16.06.90 DE 4019307**

(43) Veröffentlichungstag der Anmeldung:
**08.01.92 Patentblatt 92/02**

(45) Bekanntmachung des Hinweises auf die Patenterteilung:
**26.07.95 Patentblatt 95/30**

(84) Benannte Vertragsstaaten:
**BE CH DE DK ES FR GB GR IT LI NL**

(56) Entgegenhaltungen:
**EP-A- 0 095 683**
**EP-A- 0 253 213**
**EP-A- 0 254 426**
**EP-A- 0 336 494**
**GB-A- 2 226 817**

**TETRAHEDRON LETTERS, Band 22, Nr. 34, 1981, pages 3247-3248, Pergamon Press Ltd, GB; H. NODA et al.: "Biomimetic oxidation of methyl 3,5-dibromo-4-hydroxyphenylpyruvate oxime and related phenols"**

(73) Patentinhaber: **BAYER AG**

**D-51368 Leverkusen (DE)**

(72) Erfinder: **Krämer, Wolfgang, Dr.**
**Rosenkranz 25**
**W-5093 Burscheid 2 (DE)**
Erfinder: **Berg, Dieter, Prof.Dr.**
**Gellertweg 27**
**W-5600 Wuppertal (DE)**
Erfinder: **Dehne, Heinz-Wilhelm, Dr .**
**Krischer Strasse 81**
**W-4019 Monheim (DE)**
Erfinder: **Dutzmann, Stefan, Dr.**
**Kosenberg 10**
**W-4010 Hilden (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 464 381 B1

## Beschreibung

Die Erfindung betrifft neue 2-Methoximinocarbonsäureester, mehrere Verfahren zu ihrer Herstellung und ihre Verwendung als Schädlingsbekämpfungsmittel.

Es ist bekannt, daß bestimmte Carbonsäureester, wie beispielsweise die Verbindung 3-Methoxy-2-(2-methylphenyl)-acrylsäuremethylester fungizide Wirksamkeit besitzen (vergleiche z. B. EP 178 826 EP 254 426 und EP 253 213).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin sind bestimmte 2-Methoximinocarbonsäureester bekannt (vergleiche z. B. Tetrahedron Lett. 23, 3699-3702 [1982]; Tetrahedron Lett. 22, 3247-3248 [1981]; Austral. J. Chem. 34, 765-768 [1981]; Tetrahedron Lett. 21, 2277-2280 [1980]; J. Chem. Soc., Perkin Trans. 1, 1975, 2340-2348; Experientia 31, 756-757 [1975]; J. Chem. Soc., Perkin Trans. 1, 1972, 18-24). Über eine Wirksamkeit dieser vorbekannten Verbindungen als Schädlingsbekämpfungsmittel ist bisher nichts bekannt.

Es wurden neue 2-Methoximinocarbonsäureester der allgemeinen Formel (I),

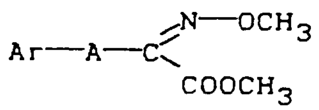

(I)

in welcher

Ar für einen der Reste

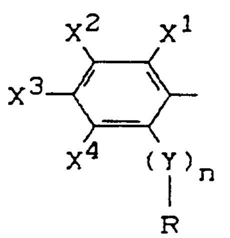 ; 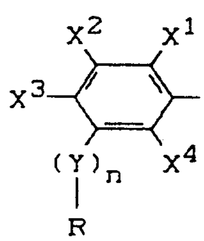 ; 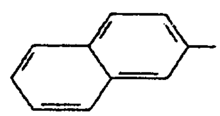 oder

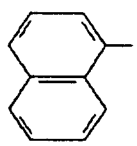

steht und

A für einen der Reste $-CH_2-$ ;

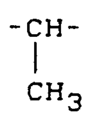 oder 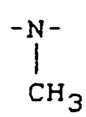

steht,

wobei

Y für einen der Reste

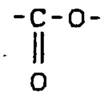 ; 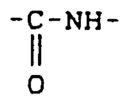 ; 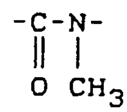 ; 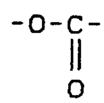 ; 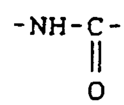 ;

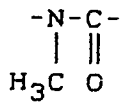 ;

$-O-CH_2-$ ; $-CH_2-O-$ ; $-S-CH_2-$;

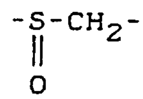 ;

$-SO_2-CH_2-$ ; -O- ; -CH = CH- oder $-CH_2-CH_2-$ steht,

n für eine Zahl 0 oder 1 steht,

R für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 9 gleichen oder verschiedenen Halogenatomen, für geradkettiges oder verzweigtes Alkenyl mit 2 bis 8 Kohlenstoffatomen, für geradkettiges oder verzweigtes Halogenalkenyl mit 2 bis 8 Kohlenstoffatomen und 1 bis 9 gleichen oder verschiedenen Halogenatomen, für gegebenenfalls einfach oder mehrfach, gleich oder verschieden durch Halogen und/oder geradkettiges oder verzweigtes Alkyl mit 1 bis 6 Kohlenstoffatomen substituiertes Cycloalkyl mit 3 bis 7 Kohlenstoffatomen oder für gegebenenfalls einfach oder mehrfach, gleich oder verschieden substituiertes Heteroaryl mit 2 bis 9 Kohlenstoffatomen und 1 bis 5 gleichen oder verschiedenen Heteroatomen - insbesondere Stickstoff, Sauerstoff und/oder Schwefel - steht, wobei als Heteroarylsubstituenten jeweils infrage kommen: Halogen sowie jeweils geradkettiges oder ver-zweigtes Alkyl oder Alkoxy mit jeweils 1 bis 6 Kohlenstoffatomen;

R steht außerdem bevorzugt für gegebenenfalls einfach oder mehrfach, gleich oder verschieden substituiertes Aryl mit 6 bis 10 Kohlenstoffatomen steht, wobei als Arylsubstituenten infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 6 Kohlenstoffatomen und 1 bis 9 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 6 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 6 Kohlenstoffatomen in den einzelnen Alkyl- bzw. Alkenylteilen, wobei als Substituenten in den einzelnen Arylteilen jeweils infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkinyl, Alkenyloxy, Alkinyloxy, Alkenylthio

oder Alkinylthio mit jeweils 2 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 6 Kohlenstoffatomen und 2 bis 9 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 6 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 6 Kohlenstoffatomen in den einzelnen Alkyl-bzw. Alkenylteilen, wobei als Substituenten in den einzelnen Arylteilen jeweils infrage kommen:

Halogen sowie jeweils geradkettiges oder verzweigtes Alkyl oder Alkoxy mit jeweils 1 bis 6 Kohlenstoffatomen;

R steht außerdem für den Fall, daß n = O ist, auch für Fluor, Chlor, Brom oder Iod, und

$X^1$, $X^2$, $X^3$ und $X^4$ unabhängig voneinander jeweils für Wasserstoff, Halogen, Hydroxy oder Methoxy stehen, wobei jedoch für den Fall, daß $X^2$ für Brom steht, $X^3$ nicht gleichzeitig für Hydroxy oder Methoxy steht,

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß die neuen 2-Methoximinocarbonsäureester der allgemeinen Formel (I),

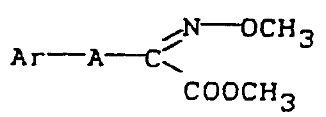

(I)

in welcher

Ar und A die oben angegebenen Bedeutungen haben

nach einem der im Folgenden beschriebenen Verfahren erhält:

(a) Man erhält 2-Methoximinocarbonsäureester der Formel (Ia),

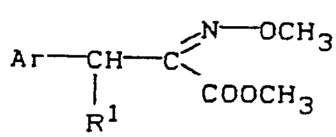

(Ia)

in welcher

$R^1$ für Wasserstoff oder Methyl steht und

Ar die oben angegebene Bedeutung hat,

wenn man Acrylester-Derivate der Formel (II),

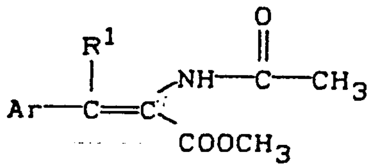

(II)

in welcher

$R^1$ und Ar die oben angegebene Bedeutung haben,

mit einem Säureadditionssalz des O-Methylhydroxylamins gegebenenfalls in Gegenwart eines Verdünnungsmittels sowie gegebenenfalls in Gegenwart eines Reaktionshilfmittels umsetzt;

(b) man erhält 2-Methoximinocarbonsäureester der Formel (Ib),

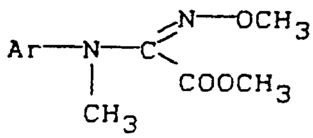

(Ib)

in welcher

Ar die oben angegebene Bedeutung hat,

wenn man zunächst in einer 1. Stufe aromatische Amine der Formel (III),

Ar—NH—$CH_3$ (III)

in welcher

Ar die oben angegebene Bedeutung hat,

mit 2-Chlor-2-hydroximino-essigsäuremethylester der Formel (IV)

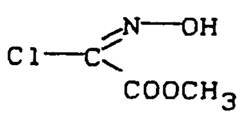

(IV)

gegebenenfalls in Gegenwart eines Verdünnungsmittels und gegebenenfalls in Gegenwart eines Säurebindemittels umsetzt und anschließend in einer 2. Stufe mit einem Methylierungsmittel gegebenenfalls in Gegenwart eines Säurebindemittels an der Hydroximinogruppe methyliert;

(c) man erhalt 2-Methoximinocarbonsäureester der Formel (Ic),

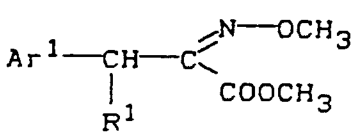

(Ic)

in welcher

$R^1$ für Wasserstoff oder Methyl steht und

$Ar^1$ für einen der Reste

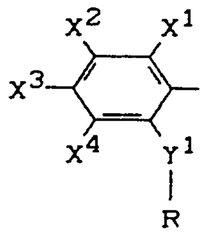 oder 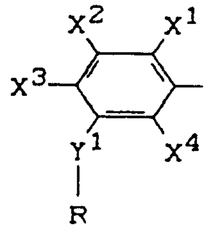

steht, wobei

$Y^1$ jeweils für einen der Reste

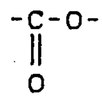 ; 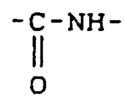 oder 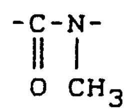

steht und

$X^1$, $X^2$, $X^3$, $X^4$ und R die oben angegebene Bedeutung haben,

alternativ auch, wenn man substituierte Phenol- oder Anilinderivate der Formel (V),

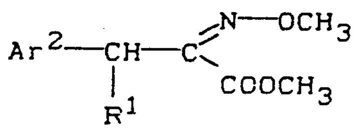 (V)

in welcher

$R^1$ für Wasserstoff oder Methyl steht und

$Ar^2$ für einen der Reste

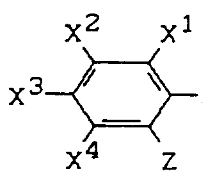 oder 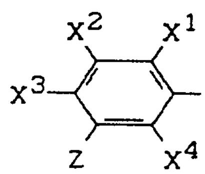

steht, wobei

Z jeweils für Hydroxy, Amino oder N-Methylamino steht und

$X^1$, $X^2$, $X^3$ und $X^4$ die oben angegebene Bedeutung haben,

mit Säurechloriden der Formel (VI),

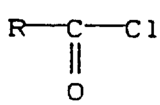 (VI)

in welcher

R die oben angegebene Bedeutung hat,

gegebenenfalls in Gegenwart eines Verdünnungsmittels und gegebenenfalls in Gegenwart eines Säurebindemittels acyliert.

Schließlich wurde gefunden, daß die neuen 2-Methoximinocarbonsäureester der allgemeinen Formel (I) eine sehr stark ausgeprägte Wirksamkeit gegenüber Schädlingen besitzen.

Überraschenderweise zeigen die erfindungsgemäßen 2-Methoximinocarbonsäureester der allgemeinen Formel (I) eine erheblich bessere fungizide Wirksamkeit als die aus dem Stand der Technik bekannten Carbonsäureester, wie beispielsweise die Verbindung 3-Methoxy-2-(2-methylphenyl)-acrylsäure-methylester, welche chemisch und wirkungsmäßig naheliegende Verbindungen sind.

Die erfindungsgemäßen 2-Methoximinocarbonsäureester sind durch die Formel (I) allgemein definiert.

Bei den unter der allgemeinen Formel (I) genannten Definitionen steht

R insbesonders bevorzugt für geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 9 gleichen oder verschiedenen Halogenatomen, für geradkettiges oder verzweigtes Alkenyl mit 2 bis 6 Kohlenstoffatomen, für geradkettiges oder verzweigtes Halogenalkenyl mit 2 bis 6 Kohlenstoffatomen und 1 bis 9 gleichen oder verschiedenen Halogenatomen, für gegebenenfalls ein- bis fünffach gleich oder verschieden durch geradkettiges oder verzweigtes Alkyl mit 1 bis 4 Kohlenstoffatomen und/oder durch Halogen substituiertes Cycloalkyl mit 3 bis 7 Kohlenstoffatomen oder für gegebenenfalls ein- bis dreifach gleich oder verschieden substituiertes Heteroaryl mit 2 bis 9 Kohlenstoffatomen und 1 bis 4 gleichen oder verschiedenen Heteroatomen - insbesondere Stickstoff, Sauerstoff und/oder Schwefel - steht, wobei als Heteroarylsubstituenten jeweils infrage kommen:
Fluor, Chlor, Brom sowie jeweils geradkettiges oder verzweigtes Alkyl oder Alkoxy mit jeweils 1 bis 4 Kohlenstoffatomen;

R steht außerdem insbesondere bevorzugt für gegebenenfalls ein- bis fünffach, gleich oder verschieden substituiertes Aryl mit 6 oder 10 Kohlenstoffatomen, wobei als Arylsubstituenten jeweils infrage kommen:
Fluor, Chlor, Brom, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 4 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 4 Kohlenstoffatomen und 1 bis 9 gleichen oder verschiedenen Halogenatomen, je-weils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 4 Kohlenstoffatomen und 1 bis 7 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 4 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 4 Kohlenstoffatomen in den einzelnen Alkyl- bzw. Alkenylteilen, wobei als Substituenten in den einzelnen Arylteilen jeweils infrage kommen:
Fluor, Chlor, Brom, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 4 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 4 Kohlenstoffatomen und 1 bis 9 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 4 Kohlenstoffatomen und 1 bis 7 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 4 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 4 Kohlenstoffatomen in den einzelnen Alkyl- bzw. Alkenylteilen, wobei als Substituenten in den einzelnen Arylteilen jeweils infrage kommen: Fluor, Chlor, Brom, sowie jeweils geradkettiges oder verzweigtes Alkyl oder Alkoxy mit jeweils 1 bis 4 Kohlenstoffatomen;

R steht außerdem insbesondere bevorzugt für den Fall, daß n = O ist, auch für Chlor oder Brom.

Weiterhin stehen $X^1$, $X^2$, $X^3$ und $X^4$ unabhängig voneinander jeweils bevorzugt für Wasserstoff, Fluor, Chlor, Brom, Iod, Hydroxy oder Methoxy, wobei jedoch für den Fall, daß $X^2$ für Brom steht, $X^3$ nicht gleichzeitig für Hydroxy oder Methoxy steht.

Insbesondere stehen $X^1$, $X^2$, $X^3$ und $X^4$ unabhängig voneinander jeweils für Wasserstoff, Chlor, Brom, Hydroxy oder Methoxy, wobei jedoch für den Fall, daß $X^2$ für Brom steht, $X^3$ nicht gleichzeitig für Hydroxy oder Methoxy steht.

Bei den unter der allgemeinen Formel (I) aufgeführten Definitionen steht Ar vorzugsweise für den Rest

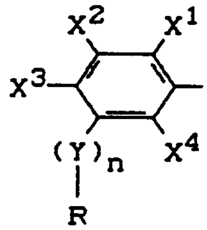

wobei

R, Y, n, $X^1$, $X^2$, $X^3$ und $X^4$ die vorstehend angegebenen Bedeutungen haben können.

Verwendet man beispielsweise 2-Acetamido-3-[3-(4-chlorphenyl)-phenyl]-acrylsäuremethylester und O-Methylhydroxylamin Hydrochlorid als Ausgangsstoffe, so läßt sich der Reaktionablauf des erfindungsgemäßen Verfahrens (a) durch das folgende Formelschema darstellen:

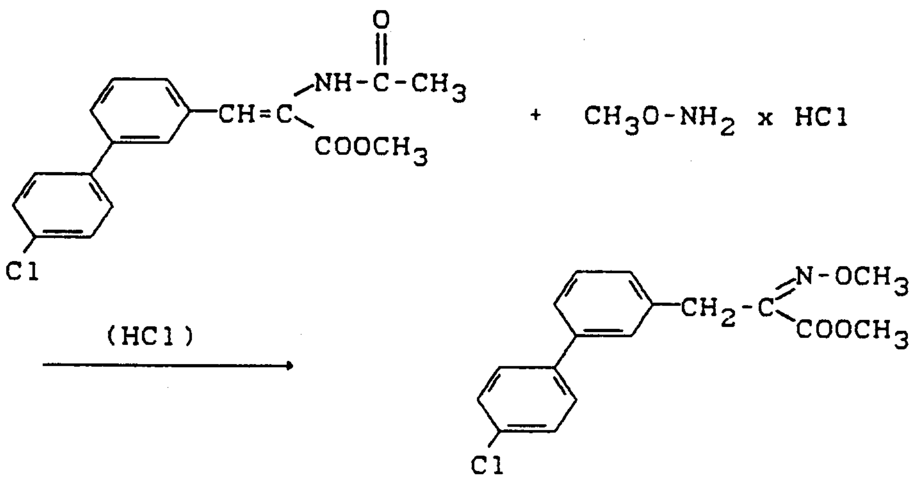


Verwendet man beispielsweise 2-Chlor-2-hydroximinoessigsäuremethylester und N-Methyl-3-trifluormethylanilin als Ausgangsstoffe sowie Dimethylsulfat als Methylierungsmittel, so läßt sich der Reaktionsablauf des erfindungsgemäßen Verfahrens (b) durch das folgende Formelschema darstellen:

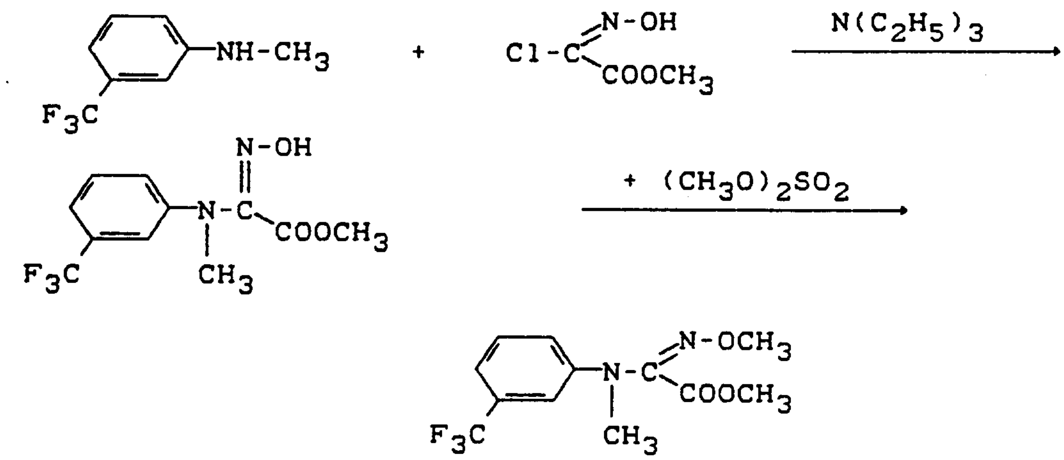


Verwendet man beispielsweise 3-(3-Hydroxyphenyl)-2-methoximino-propionsäuremethylester und 3-Chlorbenzoylchlorid als Ausgangsstoffe, so läßt sich der Reaktionsablauf des erfindungsgemäßen Verfahrens (c) durch das folgende Formelschema darstellen:

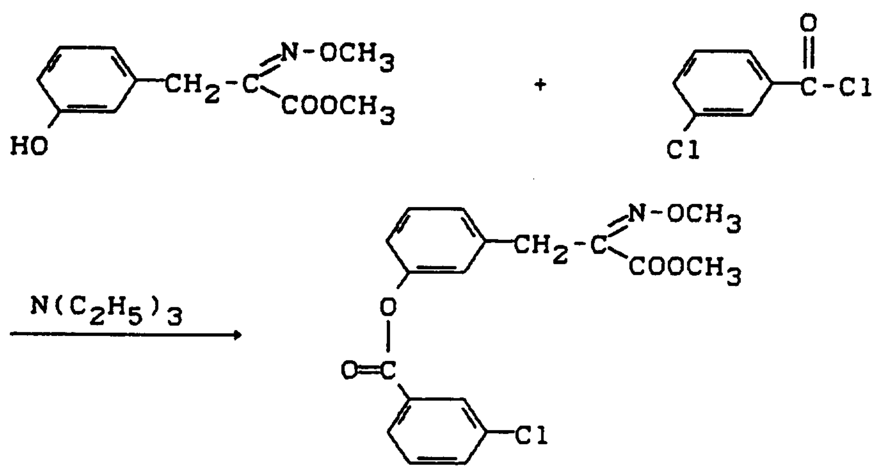


Die zur Durchführung des erfindungsgemäßen Verfahrens (a) als Ausgangsstoffe benötigten Acrylester-Derivate sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diese Substituenten genannt wurden.

$R^1$ steht vorzugsweise für Wasserstoff oder Methyl. Die Acrylester-Derivate der Formel (II) sind bekannt (vergleiche z. B. Synthesis 1989, 414-418; Jpn. Kokai Tokkyo Koho JP 61218558; J. Labelled Comp. Radiopharm. 21, 263-284 [1984]; Liebigs Ann. Chem. 1984, 1205-1215) oder erhältlich in Analogie zu bekannten Verfahren (vergleiche z. B. Organic Reactions Vol. III, Azlactones S. 198-239) beispielsweise wenn man aromatische Aldehyde oder Ketone der Formel (VII),

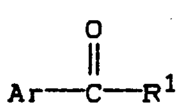

(VII)

in welcher

$R^1$ und Ar die oben angegebene Bedeutung haben,

mit N-Acetylglycin in Gegenwart von Acetanhydrid und Natriumacetat sowie gegebenenfalls in Gegenwart von Essigsäure bei Temperaturen zwischen 20 °C und 160° C umsetzt und dann in einer 2. Stufe die so erhältlichen Azlactone der Formel (VIII),

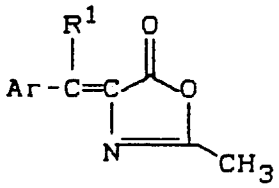

(VIII)

in welcher

R[1] und Ar die oben angegebene Bedeutung haben,

in Methanol gegebenenfalls in Gegenwart einer Base, wie beispielsweise Natriumhydroxid oder Natriummethylat bei Temperaturen zwischen 0 °C und 50 °C umsetzt.

Aromatische Aldehyde und Ketone der Formel (VII) sind allgemein bekannte Verbindungen der organischen Chemie oder erhältlich in Analogie zu allgemein bekannten Verfahren.

Die zur Durchführung des erfindungsgemäßen Verfahrens (b) als Ausgangsstoffe benötigten aromatischen Amine sind durch die Formel (III) allgemein definiert. In dieser Formel (III) steht Ar vorzugsweise für diejenigen Reste, die bereits im Zusammenhang mit der Beschreibung der erfindungsgemäßen Stoffe der Formel (I) als bevorzugt für diese Substituenten genannt wurden.

Die aromatischen Amine der Formel (III) sind ebenfalls allgemein bekannte Verbindungen der organischen Chemie.

Der zur Durchführung des erfindungsgemäßen Verfahrens (b) weiterhin als Ausgangsverbindung benötigte 2-Chlor-2-hydroximino-essigsäuremethylester der Formel (IV) ist ebenfalls bekannt (vergleiche DE 19 63 061; US 3 584 032).

Die zur Durchführung des erfindungsgemäßen Verfahrens (c) als Ausgangsstoffe benötigten substituierten Phenol- und Anilinderivate sind durch die Formel (V) allgemein definiert, In dieser Formel (V) steht $R^1$ vorzugsweise für Wasserstoff oder Methyl und $Ar^2$ für einen der Reste

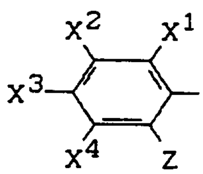

oder

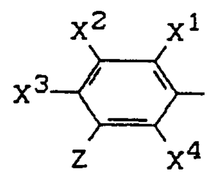

,

wobei

Z jeweils für Hydroxy, Amino oder N-Methylamino steht und

$X^1$, $X^2$, $X^3$ und $X^4$ vorzugsweise für diejenigen Reste stehen, die bereits im Zusammenhang mit der Beschreibung der erfindungsgemäßen Stoffe der Formel (I) als bevorzugt für diese Substituenten genannt wurden.

Die substituierten Phenol- und Anilinderivate der Formel (V) sind noch nicht bekannt und ebenfalls Gegenstand der Erfindung.

Man erhält sie, wenn man Azlactone der Formel (IX),

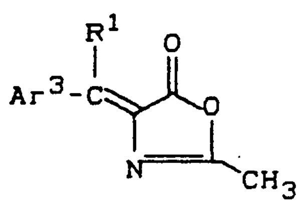

(IX)

in welcher

$R^1$ die oben angegebene Bedeutung hat und

$Ar^3$ für einen der Reste

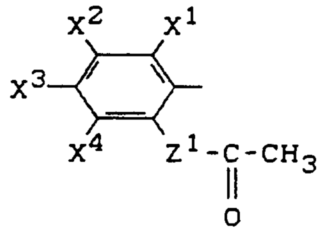

oder

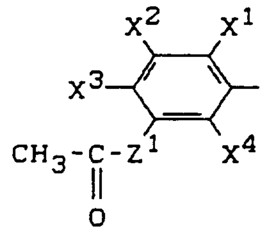

steht, wobei

$Z^1$ jeweils für Sauerstoff, für eine NH-Gruppe oder für eine

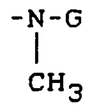

Gruppe

steht und

$X^1$, $X^2$, $X^3$ und $X^4$ die oben angegebene Bedeutung haben,

mit Methanol in Gegenwart einer Base wie beispielsweise Natriumhydroxid und anschließend in Gegenwart einer Säure wie beispielsweise Salzsäure bei Temperaturen zwischen 0 °C und 50 °C umsetzt und anschließend die so erhältlichen Acrylester-Derivate der Formel (X),

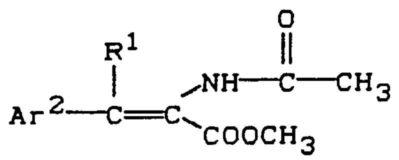

(X)

in welcher

$R^1$ die oben angegebene Bedeutung hat und

$Ar^2$ für einen der Reste

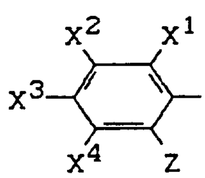

oder

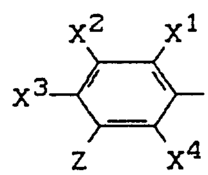

steht, wobei

Z jeweils für Hydroxy, Amino oder N-Methylamino steht und

$X^1$, $X^2$, $X^3$ und $X^4$ die oben angegebene Bedeutung haben,

mit einem Säureadditionssalz des O-Methylhydroxylamins, wie beispielsweise dem O-Methylhydroxylaminhydrochlorid, gegebenenfalls in Gegenwart eines Verdünnungsmittels wie beispielsweise Methanol und gegebenenfalls in Gegenwart eines Reaktionshilfsmittels wie beispielsweise Schwefelsäure in Analogie zur Durchführung des erfindungsgemäßen Verfahrens (a) bei Temperaturen zwischen 20 °C und 60 °C umsetzt.

Azlactone der Formel (IX) sind bekannt (vergleiche z. B. Takeda Kenkyushoho 43, 53-76 [1984]; Izv. Akad. Nauk SSSR, Ser. Khim. 1984, 1090-1094; Izv. Akad. Nauk SSSR, Ser. Khim. 1980, 823-829; Tetrahedron Lett. 1979, 737-740; Bull. Soc. Chim. Belg., 83 (3/4), 117-132 [1974]; DE 22 43 665; BE 715 588; ZA 6803 083) oder erhältlich in Analogie zu bekannten Verfahren (vergleiche z. B. Organic Reactions Vol. III, Azlactones, S. 108-239).

Die zur Durchführung des erfindungsgemäßen Verfahrens (c) weiterhin als Ausgangsstoffe benötigten Säurechloride sind durch die Formel (VI) allgemein definiert. In dieser Formel (VI) steht R vorzugsweise für

diejenigen Reste, die bereits im Zusammenhang mit der Beschreibung der erfindungsgemäßen Stoffe der Formel (I) als bevorzugt für diesen Substituenten genannt wurden.

Die Säurechloride (VI) sind allgemein bekannte Verbindungen der organischen Chemie.

Als Verdünnungsmittel zur Durchführung des erfindungsgemäßen Verfahrens (a) kommen polare organische Lösungsmittel oder wässrige Systeme infrage. Mit besonderem Vorzug verwendet man Alkohole, wie beispielsweise Methanol, Ethanol oder Propanol, deren Gemische mit Wasser oder reines Wasser als Verdünnungsmittel.

Als Reaktionshilfsmittel zur Durchführung des erfindungsgemäßen Verfahren (a) kommen starke Mineralsäuren wie Salzsäure, Bromwasserstoffsäure oder Schwefelsäure infrage.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 100 °C, vorzugsweise bei Temperaturen zwischen 20 °C und 60 °C.

Zur Durchführung des erfindungsgemäßen Verfahrens (a) setzt man pro Mol an Acrylester-Derivat der Formel (II) im allgemeinen 1.0 bis 3.0 Mol, vorzugsweise 1.0 bis 1.5 Mol an O-Methylhydroxylamin und gegebenenfalls 1.0 bis 3.0 Mol, vorzugsweise 1.0 bis 1.5 Mol an Reaktionshilfsmittel ein.
Die Reaktionsdurchführung, Aufarbeitung und Isolierung der Reaktionsprodukte erfolgt nach allgemein üblichen Methoden (vergleiche auch die Herstellungsbeispiele).

Als Verdünnungsmittel zur Durchführung des erfindungsgemäßen Verfahrens (b) kommen sowohl für die 1. Stufe als auch für die 2. Stufe inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Tetrachlorethan, Chloroform, Tetrachlorkohlenstoff, Ether wie Diethylether, Dioxan, Tetrahydrofuran oder Ethylenglykoldimethyl- oder -diethylether, Ketone wie Aceton oder Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Das erfindungsgemäße Verfahren (b) wird vorzugsweise in Gegenwart eines geeigneten Säurebindemittels durchgeführt. Als solche kommen sowohl für die 1. Stufe als auch für die 2. Stufe alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 1. Stufe des erfindungsgemäßen Verfahrens (b) in einem größeren Bereich variiert werden. Im allgemeinen arbeitet man bei Temperaturen zwischen -20 °C und 100 °C, vorzugsweise bei Temperaturen zwischen 0 °C und 80 °C.

Zur Durchführung der 1. Stufe des erfindungsgemäßen Verfahrens (b) setzt man pro Mol an aromatischem Amin der Formel (III) im allgemeinen 1.0 bis 1.5 Mol, vorzugsweise 1.0 bis 1.2 Mol an 2-Chlor-2-hydroximinoessigsäuremethylester der Formel (IV) und gegebenenfalls 1.0 bis 1.5 Mol, vorzugsweise 1.0 bis 1.2 Mol an Säurebindemittel ein.
Die Reaktionsdurchführung, Aufarbeitung und Isolierung der Reaktionsprodukte erfolgt nach allgemein üblichen Methoden.

Als Methylierungsmittel zur Durchführung der 2. Stufe des erfindungsgemäßen Verfahrens (b) kommen alle üblicherweise verwendbaren Methylierungsmittel infrage. Vorzugsweise verwendet man Dimethylsulfat, p-Toluolsulfonsäuremethylester, Methyliodid oder Methylbromid. Besonders geeignet ist Dimethylsulfat.

Die Reaktionstemperaturen können bei der Durchführung der 2. Stufe des erfindungsgemäßen Verfahrens (b) in einem größeren Bereich variiert werden. Im allgemeinen arbeitet man bei Temperaturen zwischen -20 °C und 100 °C, vorzugsweise bei Temperaturen zwischen 0 °C und 80 °C.

Zur Durchführung der 2. Stufe des erfindungsgemäßen Verfahrens (b) setzt man pro Mol an Hydroximinoausgangsverbindung im allgemeinen 1.0 bis 1.5 Mol, vorzugsweise 1.0 bis 1.2 Mol an Methylierungsmittel und gegebenenfalls 1.0 bis 1.5 Mol, vorzugsweise 1.0 bis 1.2 Mol an Säurebindemittel ein.
Die Reaktionsdurchführung, Aufarbeitung und Isolierung der Reaktionsprodukte erfolgt nach allgemein üblichen Methoden (vergleiche auch die Herstellungsbeispiele).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Ketone wie Aceton oder

Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Das erfindungsgemäße Verfahren (c) wird vorzugsweise in Gegenwart eines geeigneten Säurebindemittels durchgeführt. Als solche kommen alle üblichen anorganischen oder organischen Basen infrage. Hierzu gehören beispielsweise Alkalimetallhydroxide wie Natriumhydroxid oder Kaliumhydroxid, Alkalimetallcarbonate wie Natriumcarbonat, Kaliumcarbonat oder Natriumhydrogencarbonat, sowie tertiäre Amine wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und 100 °C, vorzugsweise bei Temperaturen zwischen 0 °C und 60 °C.

Zur Durchführung des erfindungsgemäßen Verfahrens (c) setzt man pro Mol an Phenol- oder Anilinderivat der Formel (V) im allgemeinen 1.0 bis 1.5 Mol, vorzugsweise 1.0 bis 1.2 Mol an Säurechlorid der Formel (VI) und gegebenenfalls 1.0 bis 1.5 Mol, vorzugsweise 1.0 bis 1.2 Mol an Säurebindemittel ein.

Die Reaktionsdurchführung, Aufarbeitung und Isolierung der Reaktionsprodukte erfolgt nach allgemein üblichen Methoden (vergleiche auch die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten wie beispielsweise gegen den Erreger des echten Getreidemehltaus (Erysiphe graminis) an Gerste oder Weizen oder gegen den Erreger der Netzfleckenkrankheit der Gerste (Pyrenophora teres) oder gegen den Erreger der Braunfleckenkrankheit (Cochliobolus sativus) an Gerste oder Weizen

oder gegen den Erreger der Braunspelzigkeit des Weizens (Leptosphaeria nodorum) oder gegen den Erreger des Getreideschneeschimmels (Fusarium nivale) oder zur Bekämpfung von Reiskrankheiten, wie beispielsweise gegen den Erreger der Reisfleckenkrankheit (Pyricularia oryzae) oder zur Bekämpfung von Krankheiten im Obst- und Gemüseanbau, wie beispielsweise gegen den Erreger des Apfelschorfs (Venturia inaequalis) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele:

Beispiel 1:

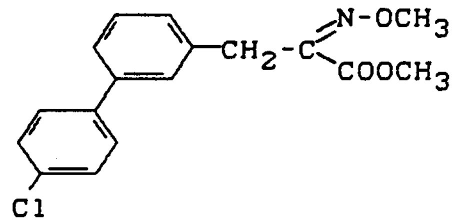

(Verfahren a)

Zu einer Suspension von 130 g (0.395 Mol) 2-Acetamido-3-[3-(4-Chlorphenyl)-phenyl]-acrylsäuremethylester in 500 ml Methanol gibt man 36 g (0.435 Mol) O-Methylhydroxylamin-hydrochlorid und 43.5 ml (0.435 Mol) konzentrierte Salzsäure, erhitzt für 38 Stunden auf Rückflußtemperatur, engt dann im Vakuum ein, nimmt den Rückstand in 1 l Dichlormethan auf, wäscht dreimal mit jeweils 500 ml Wasser, trocknet über Natriumsulfat, entfernt das Lösungsmittel im Vakuum und reinigt den Rückstand durch Chromatographie an Kieselgel (Laufmittel: Dichlormethan).

Man erhält 104.2 g (83 % der Theorie) an 3-[3-(4-Chlorphenyl)-phenyl]-2-methoximinopropionsäuremethylester als Öl vom Brechungsindex $n_D^{23}$ 1.5919.

Herstellung der Ausgangsverbindung

Beispiel II-1

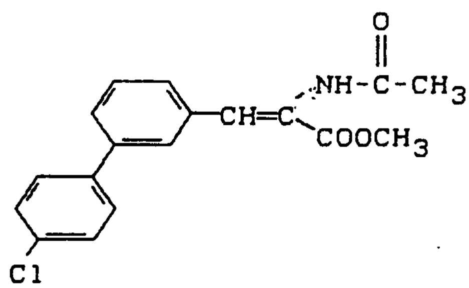

Zu einer Suspension von 130 g (0.437 Mol) 4-[3-(4-Chlorphenyl)-benzyliden]-2-methyl-1,3-oxazolin-5-on in 500 ml Methanol gibt man 1.7 ml (0.022 Mol) 50prozentige wässrige Natronlauge und rührt dann 18 Stunden bei 20 °C. Zur Aufarbeitung wird der ausgefallene Niederschlag abgesaugt, mit 200 ml Diethylether gewaschen und getrocknet.

Man erhält 130 g (90 % der Theorie) an 2-Acetamido-3-[3-(4-chlorphenyl)-phenyl]-acrylsäuremethylester vom Schmelzpunkt 168 °C.

Beispiel VIII-1

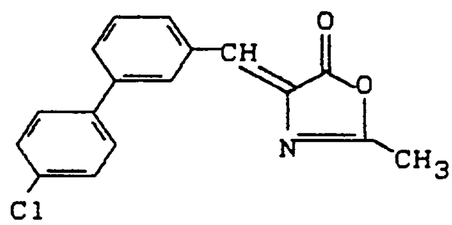

Zu einer Suspension von 179 g (0.827 Mol) 3-(4-Chlorphenyl)-benzaldehyd in 1 l Acetanhydrid gibt man 117,1 g (1 Mol) N-Acetylglycin und 82 g (1 Mol) Natriumacetat und rührt 18 Stunden bei 130 °C. Zur Aufarbeitung wird das Lösungsmittel im Wasserstrahlvakuum abdestilliert, der Rückstand in 2 l Dichlormethan aufgenommen, dreimal mit jeweils 1 l Wasser gewaschen, über Natriumsulfat getrocknet und im Vakuum eingeengt. Der Rückstand wird durch Verrühren mit 300 ml Acetonitril kristallisiert, abgesaugt und getrocknet.

Man erhält 115.9 g (47 % der Theorie) an 4-[3-(4-Chlorphenyl)-benzyliden]-2-methyl-1,3-oxazolin-5-on vom Schmelzpunkt 150 °C.

Beispiel 2

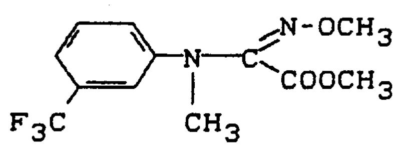

(Verfahren b)

Zu 0.5 g (0.0018 Mol) 2-(Hydroximino)-2-[N-methyl-N-(3-trifluormethylphenyl)-amino]-essigsäuremethylester in 30 ml Tetrahydrofuran gibt man 0.3 g (0.0018 Mol) Kaliumcarbonat und 0.2 ml (0.0018 Mol) Dimethylsulfat, rührt 16 Stunden bei 20 °C, saugt dann ausgefallenen Niederschlag ab, engt das Filtrat im Wasserstrahlvakuum ein und chromatographiert den Rückstand über Kieselgel (Laufmittel: Toluol/Essigester 1:1).

Man erhält 0.5 g (100 % der Theorie) an 2-Methoximino-2-[N-methyl-N-(3-trifluormethylphenyl)-amino]-essigsäuremethylester als Öl.

$^1$H-NMR ($CDCl_3$/Tetramethylsilan):

$\delta$ = 4.12 (3H,s); 3.66 (3H,s); 3.22 (3H,s)ppm

Herstellung der Ausgangsverbindung

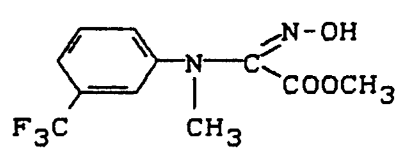

Zu 6.8 g (0.039 Mol) N-Methyl-3-trifluormethylanilin in 50 ml Toluol gibt man 5.4 ml (0.039 Mol) Triethylamin und anschließend bei 5 °C unter Rühren tropfenweise eine Lösung von 5.4 g (0.039 Mol) 2-Chlor-2-hydroximinoessigsäuremethylester in 17 g Toluol. Man läßt die Mischung 16 Stunden bei Raumtemperatur stehen, wäscht dann zweimal mit jeweils 50 ml Wasser, trocknet über Natriumsulfat, engt im

Vakuum ein und chromatographiert den Rückstand an Kieselgel (Laufmittel: Toluol/Essigester 1:1).

Man erhält 0.5 g (4.6 % der Theorie) an 2-Hydroximino-2-[N-methyl-N-(3-trifluormethylphenyl)-amino]-essigsäuremethylester vom Schmelzpunkt 131 °C.

Beispiel 3

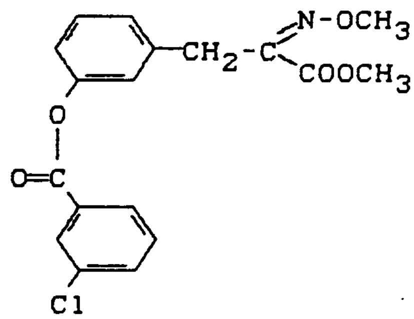

(Verfahren c)

Zu 4.4 g (0.02 Mol) 3-(3-Hydroxyphenyl)-2-methoximinopropionsäuremethylesterund 2.8 ml (0.02 Mol) Triethylamin in 20 ml Essigester gibt man tropfenweise innerhalb von 10 Minuten bei Raumtemperatur 3.5 g (0.02 Mol) 3-Chlorbenzoylchlorid in 10 ml Essigester, wobei die Temperatur der Reaktionsmischung auf 30 °C ansteigt. Man rührt eine Stunde bei Raumtemperatur, wäscht dann zweimal mit jeweils 100 ml Wasser, trocknet über Natriumsulfat, engt im Vakuum ein, kristallisiert den Rückstand durch Verrühren mit 20 ml n-Hexan, saugt ab und trocknet.

Man erhält 5.2 g (72 % der Theorie) an 3-[3-(3-Chlorbenzoyloxy)-phenyl]-2-methoximinopropionsäuremethylester vom Schmelzpunkt 65 °C.

Herstellung der Ausgangsverbindung

Beispiel V-1

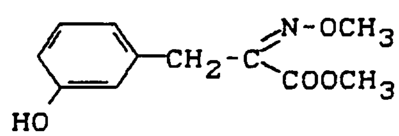

Zu 142.9 g (0.61 Mol) 3-(3-Hydroxyphenyl)-2-acetamidoacrylsäuremethylester in 600 ml Methanol gibt man 50.6 g (0.61 Mol) O-Methylhydroxylaminhydrochlorid und dann tropfenweise unter Rühren 61 g (0.61 Mol) konzentrierte Schwefelsäure, wobei die Temperatur der Reaktionsmischung auf 35 °C ansteigt. Man rührt 30 Minuten bei Raumtemperatur und erhitzt dann für 18 Stunden auf Rückflußtemperatur. Zur Aufarbeitung wird das Lösungsmittel im Wasserstrahlvakuum abdestilliert, der Rückstand in 600 ml Dichlormethan aufgenommen, zweimal mit jeweils 300 ml Wasser gewaschen, über Natriumsulfat getrocknet, im Vakuum eingeengt, der Rückstand an Kieselgel chromatographiert (Laufmittel: Dichlormethan/Methanol 100:2.5) und anschließend aus 150 ml n-Hexan-Diisopropylether (9:1) kristallisiert.

Man erhält 77.9 g (57 % der Theorie) an 3-(3-Hydroxyphenyl)-2-methoximinopropionsäuremethylester vom Schmelzpunkt 75 °C.

Beispiel X-1

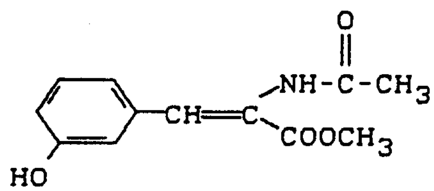

Zu 124.7 g (0.51 Mol) 4-(3-Acetoxybenzyliden)-2-methyl-2-oxazolin-5-on (vergleiche DE 22 43 665) in 600 ml Methanol gibt man 0.44 ml (0.005 Mol) 50prozentige Natronlauge, rührt dann 3 Stunden bei 65 °C, gibt weitere 4 ml (0.045 Mol) 50prozentige Natronlauge zu und rührt 16 Stunden bei 20 °C. Zur Aufarbeitung wird 5 ml (0.05 Mol) konzentrierte Salzsäure zugegeben, das Lösungsmittel abdestilliert, 500 ml Wasser zugegeben und der ausgefallene Niederschlag abgesaugt.

Man erhält 102.7 g (86 % der Theorie) an 3-(3-Hydroxyphenyl)-2-acetamidoacrylsäuremethylester vom Schmelzpunkt 115 °C.

In entsprechender Weise und gemäß den allgemeinen Angaben zur Herstellung erhält man die folgenden 2-Methoximinocarbonsäureester der allgemeinen Formel (I):

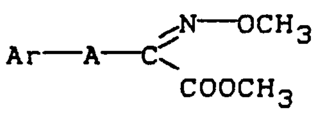

(I)

| Bsp.-Nr. | Ar | A | physikalische Eigenschaften |
|---|---|---|---|
| 4 | 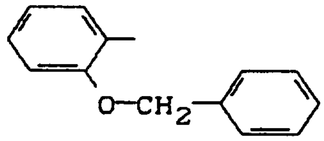 | $-CH_2-$ | Fp.: 55° C |
| 5 | 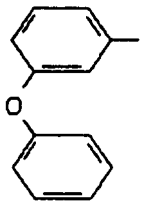 | $-CH_2-$ | $n_D^{23}$ : 1.5620 |
| 6 | 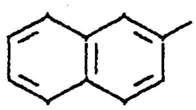 | $-CH_2-$ | Fp.: 60° C |
| 7 | 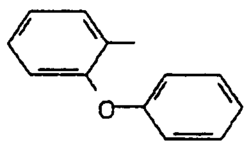 | $-CH_2-$ | $n_D^{23}$ : 1.5609 |
| 8 | 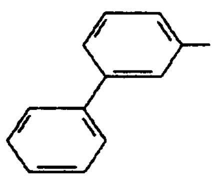 | $-CH_2-$ | $n_D^{20}$ : 1.5822 |
| 9 | 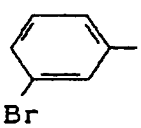 | $-CH_2-$ | $n_D^{20}$ : 1.5468 |
| 10 | 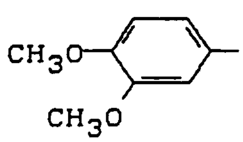 | $-CH_2-$ | Fp.: 70° C |

| Bsp.-Nr. | Ar | A | physikalische Eigenschaften |
|---|---|---|---|
| 11 | 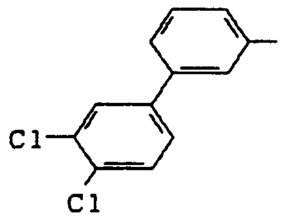 | $-CH_2-$ | Fp.: 35° C |
| 12 | 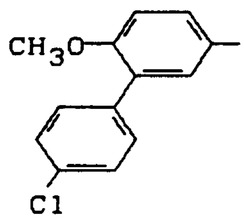 | $-CH_2-$ | Fp.: 62° C |
| 13 | 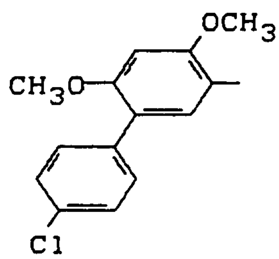 | $-CH_2-$ | Fp.: 120° C |
| 14 | 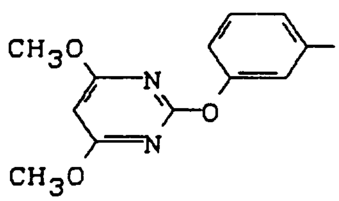 | $-CH_2-$ | Fp.: 106° C |
| 15 | 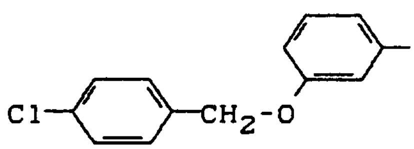 | $-CH_2-$ | $n_D^{20}$ : 1.5641 |
| 16 | 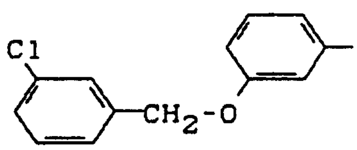 | $-CH_2-$ | $n_D^{20}$ : 1.5682 |

| Bsp.-Nr. | Ar | A | physikalische Eigenschaften |
|---|---|---|---|
| 17 | 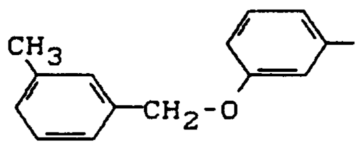 | $-CH_2-$ | $n_D^{23}$ : 1.5579 |
| 18 | 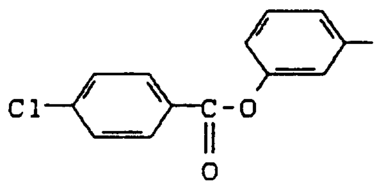 | $-CH_2-$ | Fp.: 90° C |
| 19 | 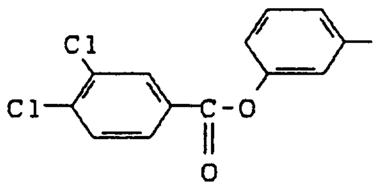 | $-CH_2-$ | Fp.: 104° C |
| 20 | 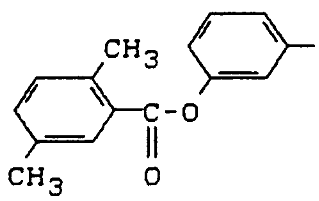 | $-CH_2-$ | Fp.: 56° C |
| 21 | 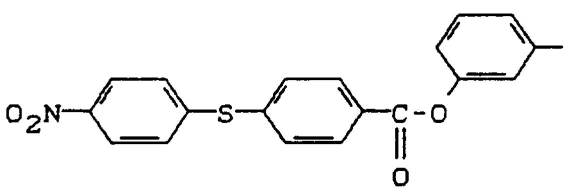 | $-CH_2-$ | $n_D^{20}$ : 1.6261 |
| 22 | 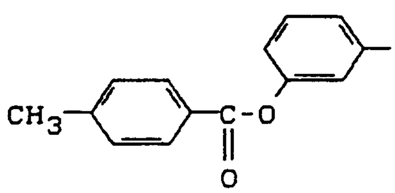 | $-CH_2-$ | Fp.: 110° C |

| Bsp.-Nr. | Ar | A | physikalische Eigenschaften |
|---|---|---|---|
| 23 | 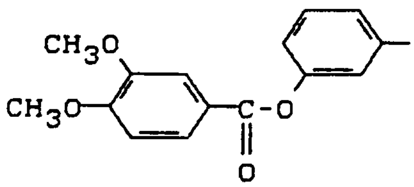 | $-CH_2-$ | Fp.: 100° C |
| 24 | 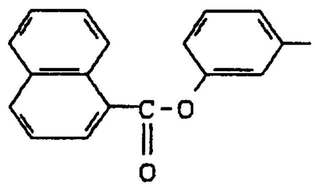 | $-CH_2-$ | Fp.: 60° C |
| 25 | 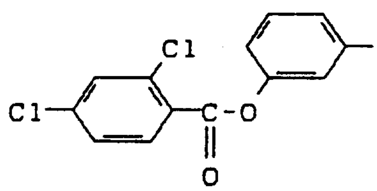 | $-CH_2-$ | Fp.: 70° C |
| 26 | 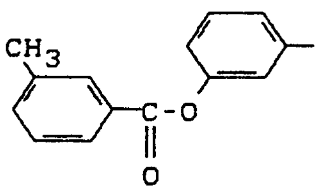 | $-CH_2-$ | Fp.: 68° C |
| 27 | 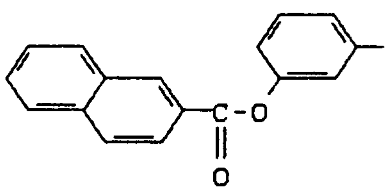 | $-CH_2-$ | Fp.: 96° C |
| 28 | 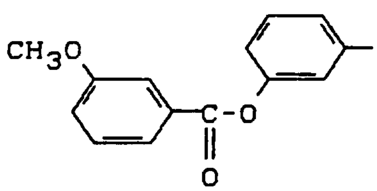 | $-CH_2-$ | $n_D^{20}$ : 1.5592 |

| Bsp.-Nr. | Ar | A | physikalische Eigenschaften |
|---|---|---|---|
| 29 | 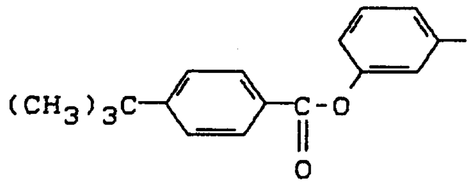 | $-CH_2-$ | $n_D^{20}$ : 1.5448 |
| 30 | 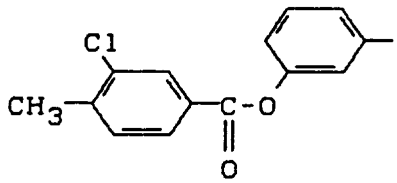 | $-CH_2-$ | Fp.: 86° C |
| 31 | 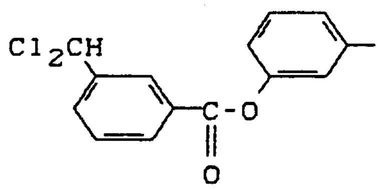 | $-CH_2-$ | $n_D^{20}$ : 1.5699 |
| 32 | 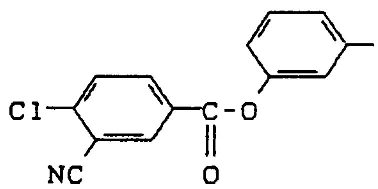 | $-CH_2-$ | Fp.: 100° C |
| 33 | 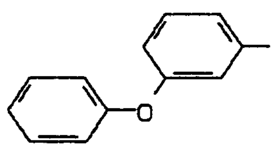 | -N-<br>$CH_3$ | $n_D^{20}$ : 1.5753 |
| 34 | 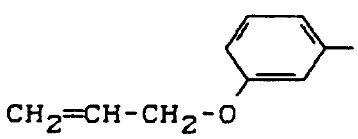 | $-CH_2-$ | $n_D^{23}$ : 1.5261 |
| 35 | 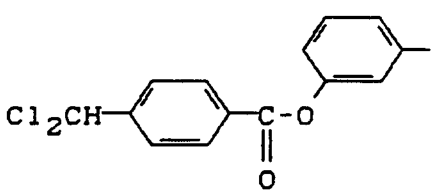 | $-CH_2-$ | Fp.: 60° C |

| Bsp.-Nr. | Ar | A | physikalische Eigenschaften |
|---|---|---|---|
| 36 | 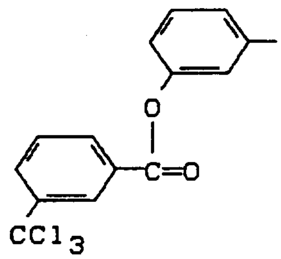 | $-CH_2-$ | $n_D^{20}$ : 1.5738 |
| 37 | 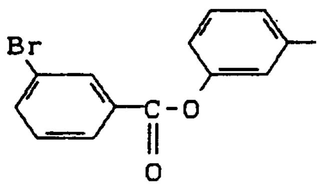 | $-CH_2-$ | Fp.: 92° C |
| 38 | 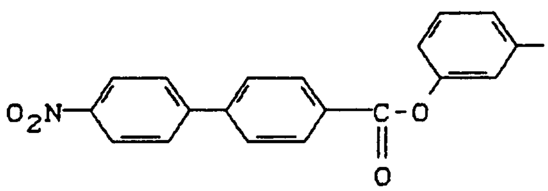 | $-CH_2-$ | Fp.: 130° C |
| 39 | 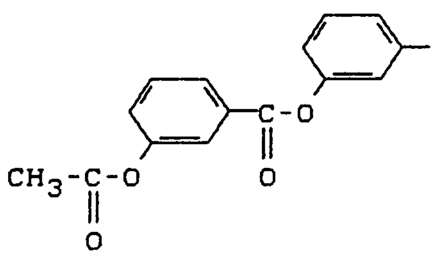 | $-CH_2-$ | Fp.: 96° C |
| 40 | 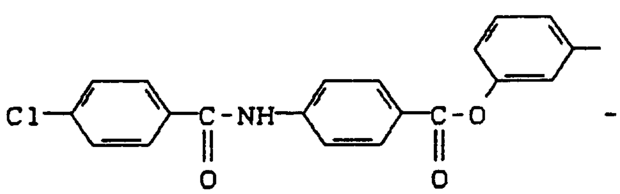 | $-CH_2-$ | Fp.: 154° C |

| Bsp.-Nr. | Ar | A | physikalische Eigenschaften |
|---|---|---|---|
| 41 | 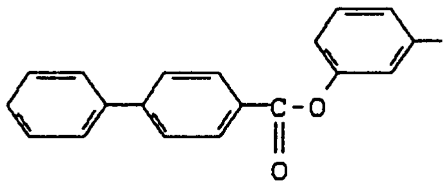 | $-CH_2-$ | Fp: 95° C |
| 42 | 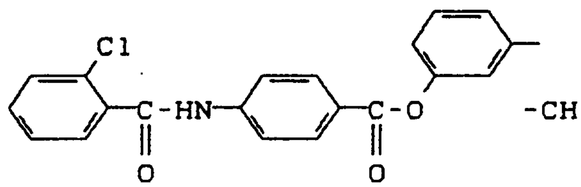 | $-CH_2-$ | Fp: 142° C |
| 43 | 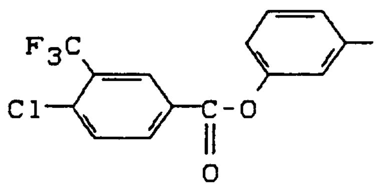 | $-CH_2-$ | Fp: 58° C |
| 44 | 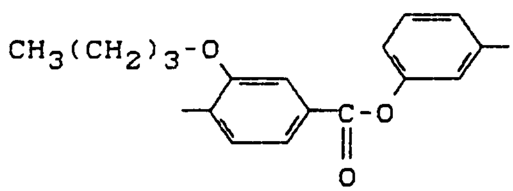 | $-CH_2-$ | Fp: 38° C |
| 45 | 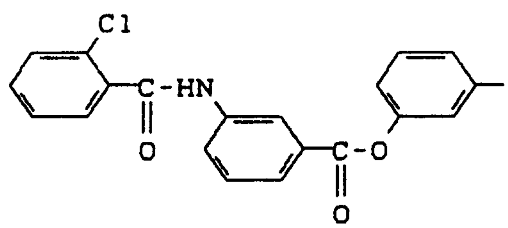 | $-CH_2-$ | Fp: 60° C |
| 46 | 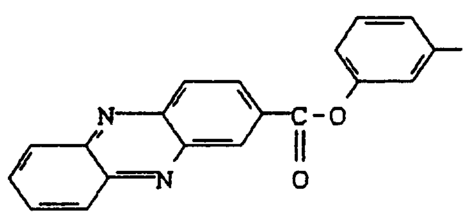 | $-CH_2-$ | Fp: 178° C |

| Bsp.-Nr. | Ar | A | physikalische Eigenschaften |
|---|---|---|---|
| 47 | 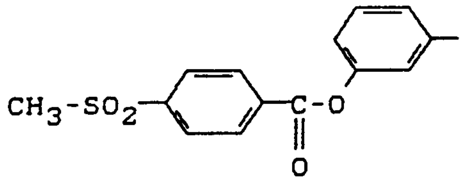 | $-CH_2-$ | Fp: 160° C |
| 48 | 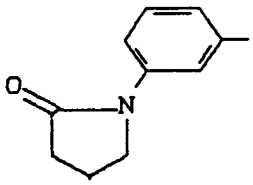 | -N-<br>$CH_3$ | Fp: 88° -92° C |
| 49 | 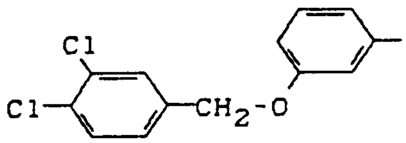 | $-CH_2-$ | Fp: 78° C |
| 50 | 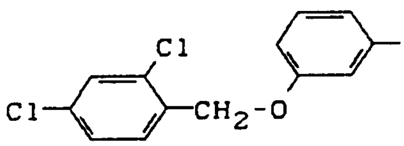 | $-CH_2-$ | $n_D^{20}$ : 1.5695 |
| 51 | 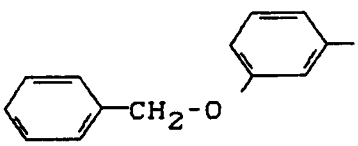 | $-CH_2-$ | $n_D^{20}$ : 1.5471 |
| 52 | 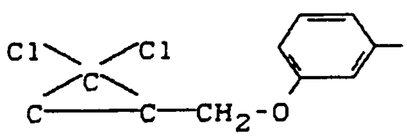 | $-CH_2-$ | $n_D^{20}$ : 1.5363 |
| 53 | 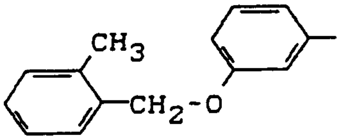 | $-CH_2-$ | $n_D^{20}$ : 1.5598 |

| Bsp.-Nr. | Ar | A | physikalische Eigenschaften |
|---|---|---|---|
| 54 | 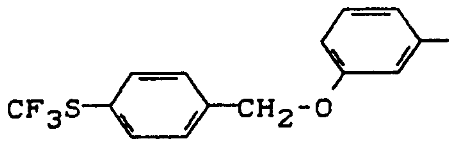 | $-CH_2-$ | $n_D^{20}$ : 1.5394 |
| 55 | 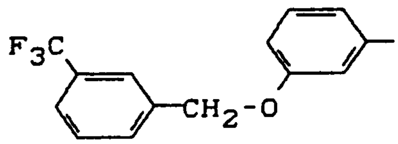 | $-CH_2-$ | $n_D^{20}$ : 1.5214 |
| 56 | 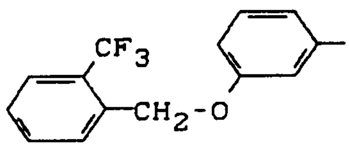 | $-CH_2-$ | $n_D^{20}$ : 1.5233 |
| 57 | 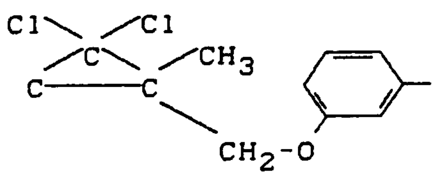 | $-CH_2$ | $n_D^{20}$ : 1.5324 |
| 58 | 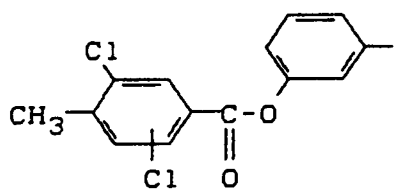 | $-CH_2-$ | $n_D^{20}$ : 1.5711 (Isomerengemisch) |
| 59 | 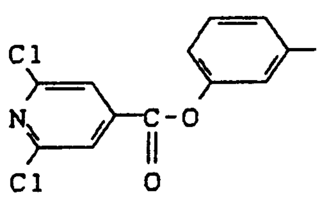 | $-CH_2-$ | Fp: 120° C |

| Bsp.-Nr. | Ar | A | physikalische Eigenschaften |
|---|---|---|---|
| 60 | 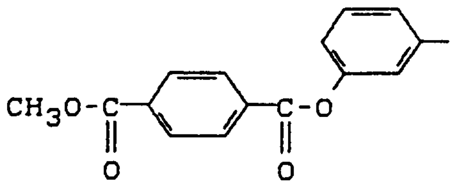 | $-CH_2-$ | Fp: 192° C |
| 61 | 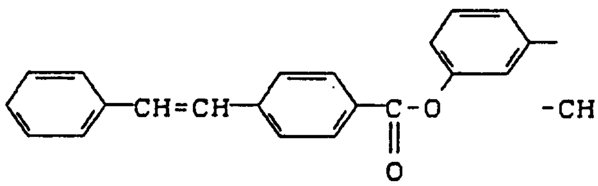 | $-CH_2-$ | Fp: 106° C |
| 62 | 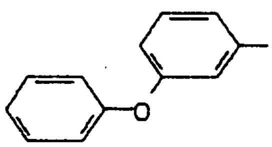 | $-N(CH_3)-$ | |
| 63 | 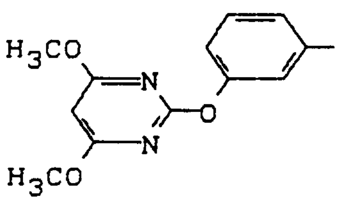 | $-CH_2-$ | Fp: 72° C |
| 64 | 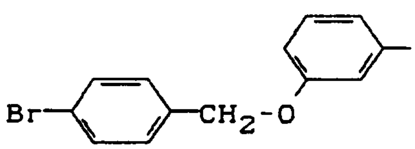 | $-CH_2$ | Fp: 68° C |
| 65 | 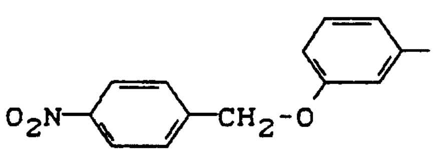 | $-CH_2$ | Fp: 78° C |

| Bsp.-Nr. | Ar | A | physikalische Eigenschaften |
|---|---|---|---|
| 66 | 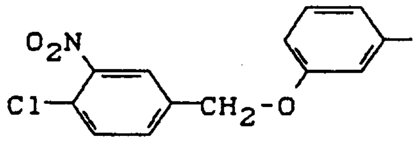 | $-CH_2-$ | Fp: 71° C |
| 67 | 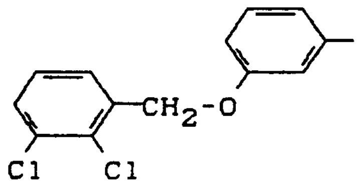 | $-CH_2-$ | Fp: 50° C |
| 68 | 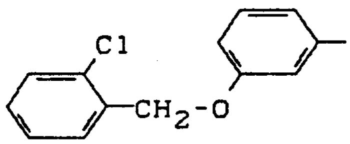 | $-CH_2-$ | $n_D^{20}$ : 1.5679 |
| 69 | 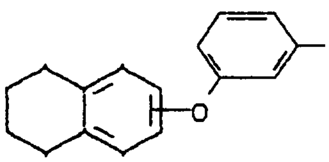 | $-CH_2-$ | $n_D^{20}$ : 1.5748 (Isomerengemisch) |
| 70 | 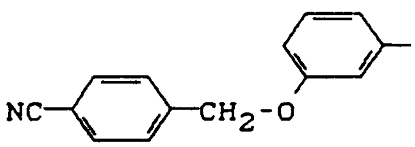 | $-CH_2-$ | Fp: 70° C |
| 71 | 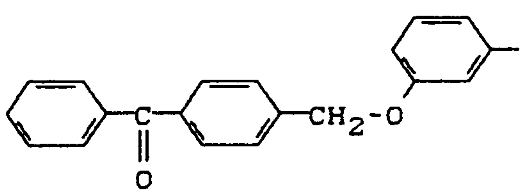 | $-CH_2-$ | $n_D^{20}$ :1.5978 |

| Bsp.-Nr. | Ar | A | physikalische Eigenschaften |
|---|---|---|---|
| 72 | 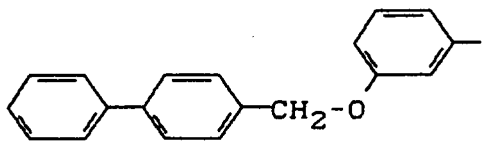 | $-CH_2-$ | Fp: 80° C |
| 73 | 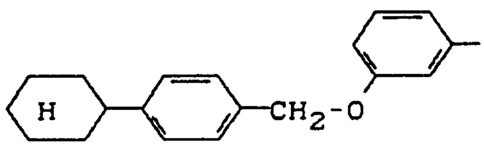 | $-CH_2-$ | $n_D^{20}$ : 1.5598 |
| 74 | 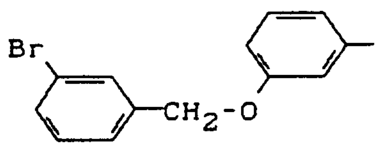 | $-CH_2-$ | $n_D^{20}$ : 1.5807 |
| 75 | 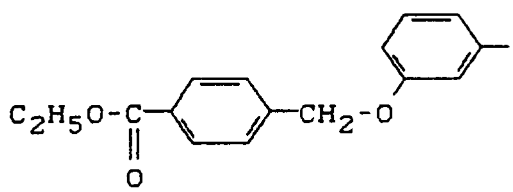 | $-CH_2-$ | Fp: 58° C |
| 76 | 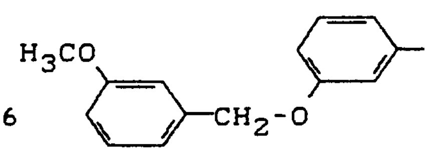 | $-CH_2-$ | $n_D^{20}$ : 1.5648 |
| 77 | 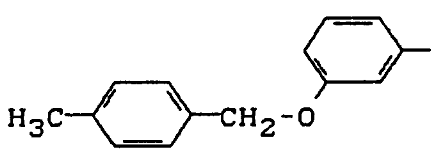 | $-CH_2-$ | Fp: 70° C |

| Bsp.-Nr. | Ar | A | physikalische Eigenschaften |
|---|---|---|---|
| 78 | 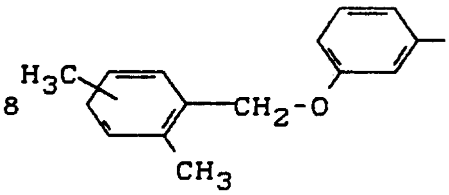 | $-CH_2-$ | $n_D^{20}$ : 1.5603 (Isomrengemisch) |
| 79 | 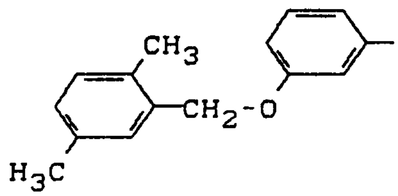 | $-CH_2-$ | $n_D^{20}$ : 1.5579 |
| 80 | 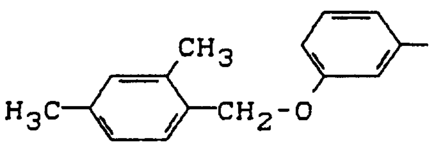 | $-CH_2-$ | Fp: 58° C |
| 81 | 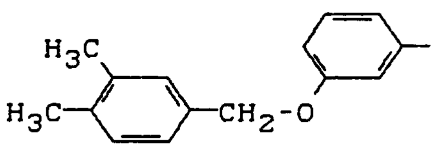 | $-CH_2-$ | $n_D^{20}$ : 1.5599 |
| 82 | 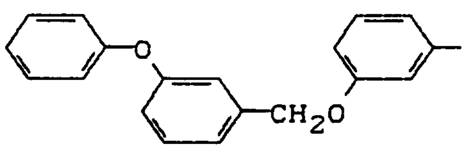 | $-CH_2-$ | $n_D^{20}$ : 1.5830 |
| 83 | 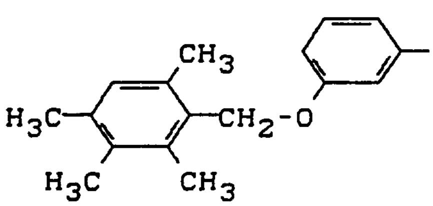 | $-CH_2-$ | Fp: 86° C |

| Bsp.-Nr. | Ar | A | physikalische Eigenschaften |
|---|---|---|---|
| 84 | 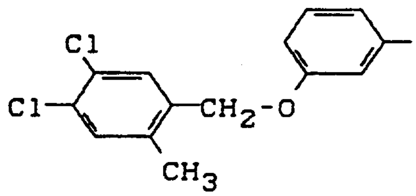 | $-CH_2-$ | Fp: 78° C |
| 85 | 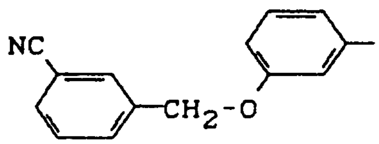 | $-CH_2-$ | Fp: 84° C |
| 86 | 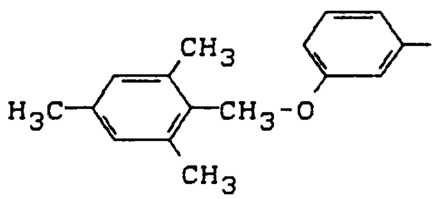 | $-CH_2-$ | $n_D^{20}$ : 1,5710 |
| 87 | 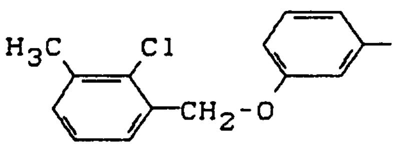 | $-CH_2-$ | Fp: 86° C |
| 88 | 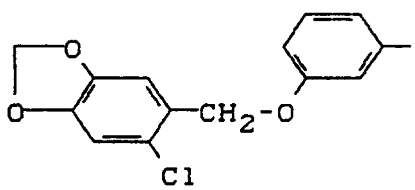 | $-CH_2-$ | Fp: 76° C |
| 89 | 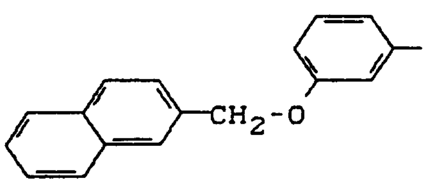 | $-CH_2$ | Fp: 84° C |

| Bsp.-Nr. | Ar | A | physikalische Eigenschaften |
|---|---|---|---|
| 90 | 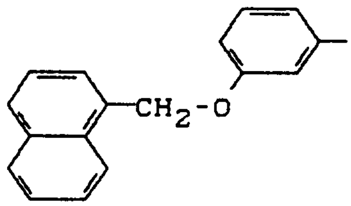 | $-CH_2$ | $n_D^{20}$ : 1.6059 |
| 91 | 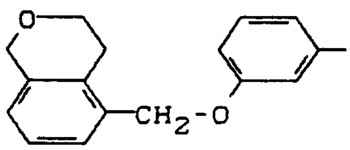 | $-CH_2-$ | $n_D^{20}$ : 1.5707 |
| 92 | 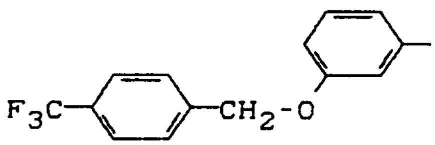 | $-CH_2$ | Fp: 60° C |
| 93 | 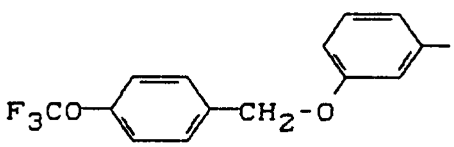 | $-CH_2-$ | $n_D^{20}$ : 1.5162 |
| 94 | 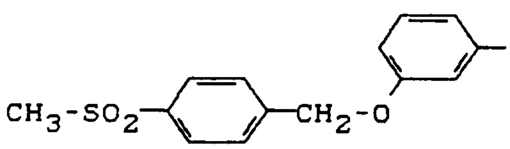 | $-CH_2-$ | Fp: 80° C |
| 95 | 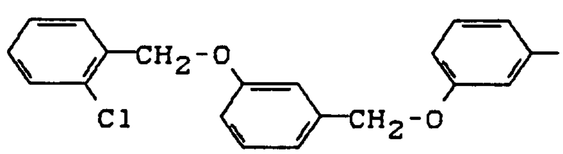 | $-CH_2-$ | $n_D^{20}$ : 1.5871 |

| Bsp.-Nr. | Ar | A | physikalische Eigenschaften |
|---|---|---|---|
| 96 | 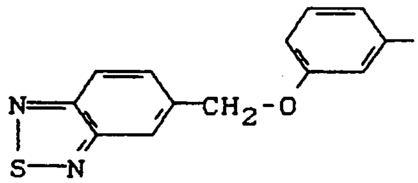 | $-CH_2-$ | Fp: 72° C |
| 97 | 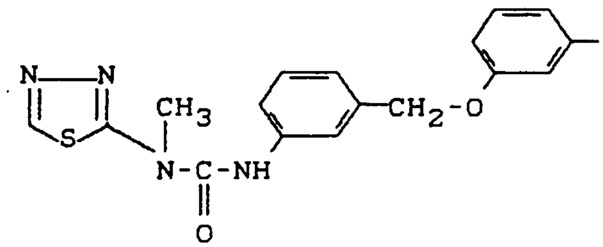 | $-CH_2-$ | Fp: 110° C |
| 98 | 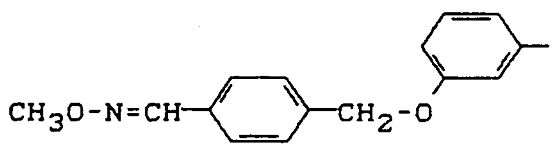 | $-CH_2-$ | Fp: 74° C |
| 99 | 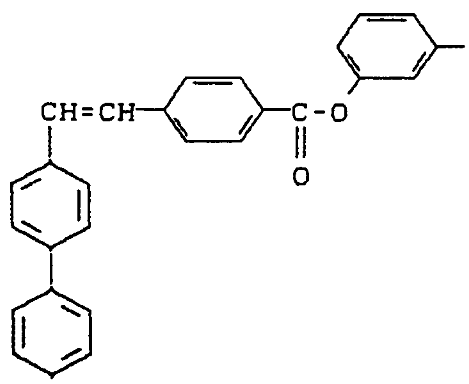 | $-CH_2-$ | Fp: 196° C |
| 100 | 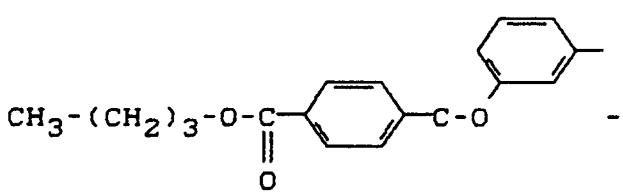 | $-CH_2-$ | Fp: 130° C |

| Bsp.-Nr. | Ar | A | physikalische Eigenschaften |
|---|---|---|---|
| 101 | 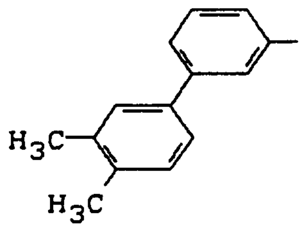 | $-CH_2-$ | Fp: 80° C |

| Bsp.-Nr. | Ar | A | physikalische Eigenschaften |
|---|---|---|---|
| 102 | 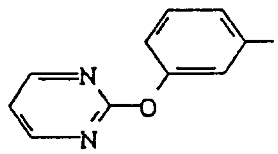 | $-CH_2-$ | Fp.: 72° C |
| 103 | 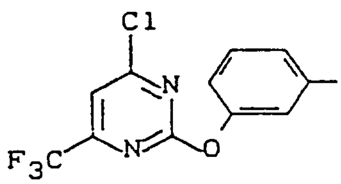 | $-CH_2-$ | Fp.: 68° C |
| 104 | 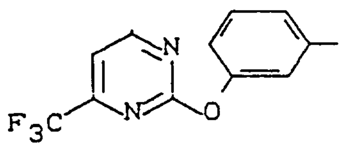 | $-CH_2-$ | Fp.: 90° C |
| 105 | 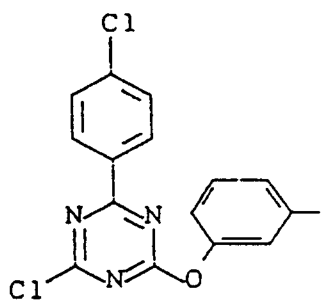 | $-CH_2-$ | Fp.: 120° C |
| 106 | 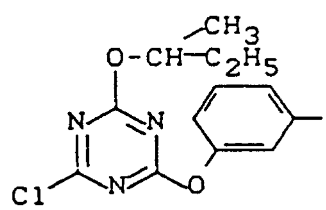 | $-CH_2-$ | $n_D^{20}$ : 1.5396 |
| 107 | 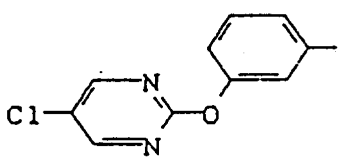 | $-CH_2-$ | Fp.: 104° C |

| Bsp.-Nr. | Ar | A | physikalische Eigenschaften |
|---|---|---|---|
| 108 | 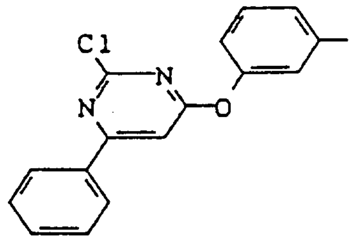 | $-CH_2-$ | Fp.: 180° C |
| 109 | 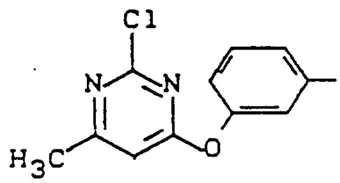 | $-CH_2-$ | Fp.: 94° C |
| 110 | 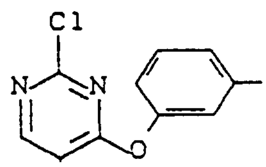 | $-CH_2-$ | Fp.: 82° C |

Anwendungsbeispiele:

In den folgenden Anwendungsbeispielen wurde die nachstehend aufgeführte Verbindung als Vergleichsubstanz eingesetzt:

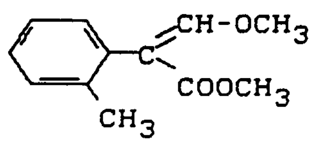

(A)

3-Methoxy-2-(2-methylphenyl)-acrylsäuremethylester (bekannt aus EP 178 826).

Beispiel A

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 9, 10 und 12.

Beispiel B

Erysiphe-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 1, 4, 5, 6, 8 und 11.

Beispiel C

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 1, 4, 5 und 11.

Beispiel D

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1 und 11.

## Patentansprüche

### Patentansprüche für folgende Vertragsstaaten : BE, CH, DE, DK, FR, GB, GR, IT, LI, NL

1. 2-Methoximinocarbonsäureester der allgemeinen Formel (I)

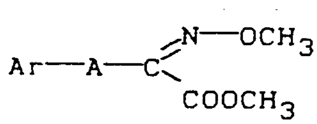

(I)

in welcher

Ar für einen der Reste

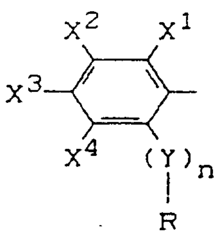 ; 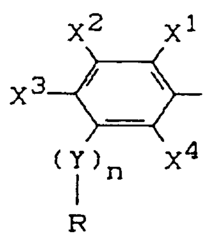 ; 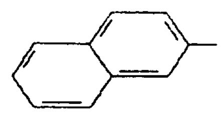 oder

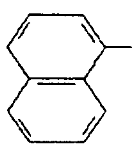

steht und

A für einen der Reste $-CH_2-$ ;

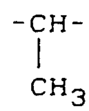 oder 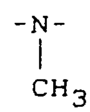

steht, wobei

Y für einen der Reste

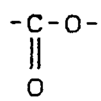 ; 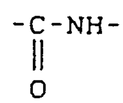 ; 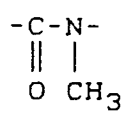 ; 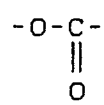 ; 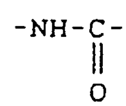 ;

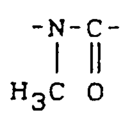 ;

$-O-CH_2-$ ; $-CH_2-O-$ ; $-S-CH_2-$;

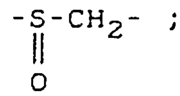

$-SO_2-CH_2-$ ; -O- ; -CH=CH- oder $-CH_2-CH_2-$ steht,

n für eine Zahl 0 oder 1 steht,

R für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 9 gleichen oder verschiedenen Halogenatomen, für geradkettiges oder verzweigtes Alkenyl mit 2 bis 8 Kohlenstoffatomen, für geradkettiges oder verzweigtes Halogenalkenyl mit 2 bis 8 Kohlenstoffatomen und 1 bis 9 gleichen oder verschiedenen Halogenatomen, für gegebenenfalls einfach oder mehrfach, gleich oder verschieden durch Halogen und/oder geradkettiges oder verzweigtes Alkyl mit 1 bis 6 Kohlenstoffatomen substituiertes Cycloalkyl mit 3 bis 7 Kohlenstoffatomen oder für gegebenenfalls einfach oder mehrfach, gleich oder verschieden substituiertes Heteroaryl mit 2 bis 9 Kohlenstoffatomen und 1 bis 5 gleichen oder verschiedenen Heteroatomen - insbesondere Stickstoff, Sauerstoff und/oder Schwefel - steht, wobei als Heteroarylsubstituenten jeweils infrage kommen: Halogen sowie jeweils geradkettiges oder ver-zweigtes Alkyl oder Alkoxy mit jeweils 1 bis 6 Kohlenstoffatomen;

R außerdem für gegebenenfalls einfach oder mehrfach, gleich oder verschieden substituiertes Aryl mit 6 bis 10 Kohlenstoffatomen steht, wobei als Arylsubstituenten infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 6 Kohlenstoffatomen und 1 bis 9 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 6 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 6 Kohlenstoffatomen in den einzelnen Alkyl- bzw. Alkenylteilen, wobei als Substituenten in den einzelnen Arylteilen jeweils infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschiedenen Halogenatomen, jeweils geradkettiges oder Verzweigtes Halogenalkenyl, Halogenalkinyl, Halogenalkenyloxy, Halogenalkinyloxy, Halogenalkenylthio oder Halogenalkinylthio mit jeweils 2 bis 6 Kohlenstoffatomen und 2 bis 9 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)amino, Aminocarbonyl,

N-Alkyl-aminocarbonyl, N,N-Dialkylaminocarbonyl oder Alkoximinoalkyl mit jeweils 1 bis 6 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 6 Kohlenstoffatomen in den einzelnen Alkyl- bzw. Alkenylteilen, wobei als Substituenten in den einzelnen Arylteilen jeweils infrage kommen:

Halogen sowie jeweils geradkettiges oder verzweigtes Alkyl oder Alkoxy mit jeweils 1 bis 6 Kohlenstoffatomen;

R außerdem für den Fall, daß n = 0 ist, auch für Fluor, Chlor, Brom oder Iod steht und

$X^1$, $X^2$, $X^3$ und $X^4$ unabhängig voneinander jeweils für Wasserstoff, Fluor, Chlor, Brom, Iod, Hydroxy oder Methoxy stehen, wobei jedoch für den Fall, daß $X^2$ für Brom steht, $X^3$ nicht gleichzeitig für Hydroxy oder Methoxy steht.

**2.** 2-Methoximinocarbonsäureester der allgemeinen Formel (I) gemäß Anspruch 1, in welcher

Ar für einen Rest

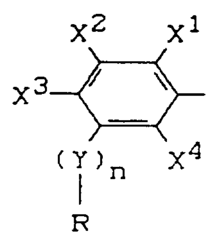

steht und

A für einen der Reste $-CH_2-$ ;

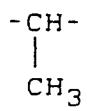 oder 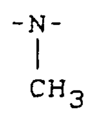

steht, wobei

Y für einen der Reste

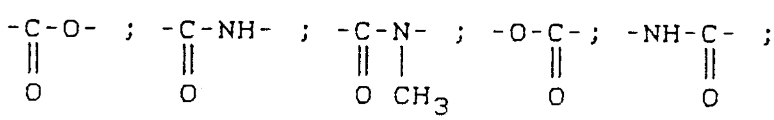

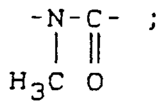

$-O-CH_2$ ; $-CH_2-O-$ ; $-S-CH_2-$;

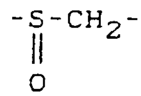 ;

$-SO_2-CH_2-$ ; -O- ; -CH=CH- oder $-CH_2-CH_2-$ steht,

n für eine Zahl 0 oder 1 steht,

R für geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 9 gleichen oder verschiedenen Halogenatomen, für geradkettiges oder verzweigtes Alkenyl mit 2 bis 6 Kohlenstoffatomen, für geradkettiges oder verzweigtes Halogenalkenyl mit 2 bis 6 Kohlenstoffatomen und 1 bis 9 gleichen oder verschiedenen Halogenatomen, für gegebenenfalls ein- bis fünffach gleich oder verschieden durch geradkettiges oder verzweigtes Alkyl mit 1 bis 4 Kohlenstoffatomen und/oder durch Halogen substituiertes Cycloalkyl mit 3 bis 7 Kohlenstoffatomen oder für gegebenenfalls ein- bis dreifach gleich oder verschieden substituiertes Heteroaryl mit 2 bis 9 Kohlenstoffatomen und 1 bis 4 gleichen oder verschiedenen Heteroatomen - insbesondere Stickstoff, Sauerstoff und/oder Schwefel - steht, wobei als Heteroarylsubstituenten jeweils infrage kommen: Fluor, Chlor, Brom sowie jeweils geradkettiges oder verzweigtes Alkyl oder Alkoxy mit jeweils 1 bis 4 Kohlenstoffatomen;

R außerdem für gegebenenfalls ein- bis fünffach, gleich oder verschieden substituiertes Aryl mit 6 oder 10 Kohlenstoffatomen steht, wobei als Arylsubstituenten jeweils infrage kommen: Fluor, Chlor, Brom, Cyano, Nitro, jeweils geradkettiges oder verzweigtes Alkyl, Alkoxy, Alkylthio,Alkylsulfinyl oder Alkylsulfonyl mit jeweils 1 bis 4 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 4 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 4 Kohlenstoffatomen und 1 bis 9 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 4 Kohlenstoffatomen und 1 bis 7 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 4 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 4 Kohlenstoffatomen in den einzelnen Alkylbzw. Alkenylteilen, wobei als Substituenten in den einzelnen Arylteilen jeweils infrage kommen:
Fluor, Chlor, Brom, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 4 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 4 Kohlenstoffatomen und 1 bis 9 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 4 Kohlenstoffatomen und 1 bis 7 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 4 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 4 Kohlenstoffatomen in den einzelnen Alkyl- bzw. Alkenylteilen, wobei als Substituenten in den einzelnen Arylteilen jeweils infrage kommen:
Fluor, Chlor, Brom, sowie jeweils geradkettiges oder verzweigtes Alkyl oder Alkoxy mit jeweils 1 bis 4 Kohlenstoffatomen;

R außerdem für den Fall, daß n = O ist, auch für Chlor oder Brom steht und

$X^1$, $X^2$, $X^3$ und $X^4$ unabhängig voneinander jeweils für Wasserstoff, Chlor, Brom, Hydroxy oder Methoxy stehen, wobei jedoch für den Fall, daß $X^2$ für Brom steht, $X^3$ nicht gleichzeitig für Hydroxy oder Methoxy steht.

**3.** Verfahren zur Herstellung von 2-Methoximinocarbonsäureestern der allgemeinen Formel (I)

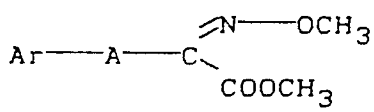
(I)

in welcher
Ar und A die in Anspruch 1 angegebene Bedeutung haben
dadurch gekennzeichnet, daß man
(a) zun Erhalt von 2-Methoximinocarbonsäureester der Formel (Ia),

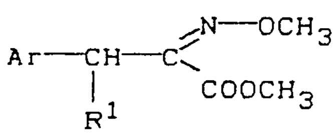
(Ia)

in welcher
$R^1$ für Wasserstoff oder Methyl steht und
Ar die oben angegebene Bedeutung hat,
Acrylester-Derivate der Formel (II),

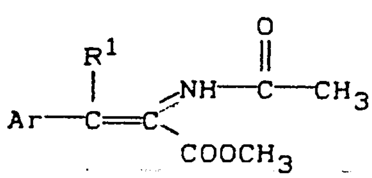
(II)

in welcher
$R^1$ und Ar die oben angegebene Bedeutung haben,
mit einem Säureadditionssalz des O-Methylhydroxylamins gegebenenfalls in Gegenwart eines Verdünnungsmittels sowie gegebenenfalls in Gegenwart eines Reaktionshilfmittels umsetzt;
oder daß man

(b) zum Erhalt von 2-Methoximinocarbonsäureestern der Formel (Ib),

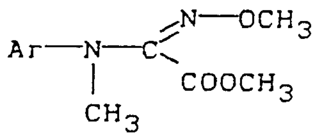 (Ib)

in welcher
Ar die oben angegebene Bedeutung hat,
zunächst in einer 1. Stufe aromatische Amine der Formel (III),

$Ar—NH—CH_3$ (III)

in welcher
Ar die oben angegebene Bedeutung hat,
mit 2-Chlor-2-hydroximino-essigsäuremethylester der Formel (IV)

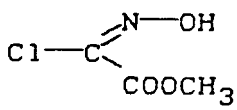 (IV)

gegebenenfalls in Gegenwart eines Verdünnungsmittels und gegebenenfalls in Gegenwart eines Säurebindemittels umsetzt und anschließend in einer 2. Stufe mit einem Methylierungsmittel gegebenenfalls in Gegenwart eines Säurebindemittels an der Hydroximinogruppe methyliert;
oder daß man
(c) zum Erhalt von 2-Methoximinocarbonsäureestern der Formel (Ic),

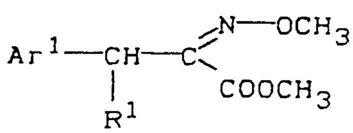 (Ic)

in welcher
$R^1$ für Wasserstoff oder Methyl steht und
$Ar^1$ für einen der Reste

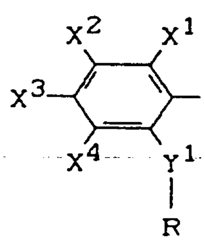 oder 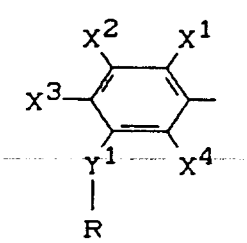

steht, wobei
$Y^1$ jeweils für einen der Reste

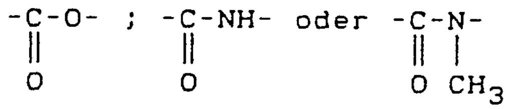
; oder

steht und

$X^1$, $X^2$, $X^3$, $X^4$ und R die oben angegebene Bedeutung haben,

substituierte Phenol-oder Anilinderivate der Formel (V),

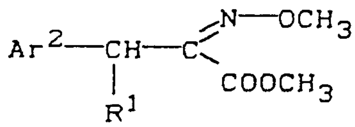
(V)

in welcher

$R^1$ für Wasserstoff oder Methyl steht und

$Ar^2$ für einen der Reste

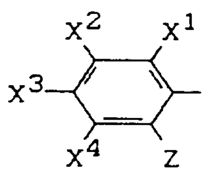
oder
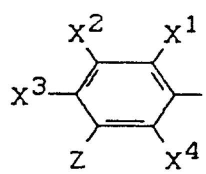

steht,

wobei

Z jeweils für Hydroxy, Amino oder N-Methylamino steht und

$X^1$, $X^2$, $X^3$ und $X^4$ die oben angegebene Bedeutung haben,

mit Säurechloriden der Formel (VI),

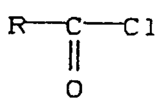
(VI)

in welcher

R die oben angegebene Bedeutung haben,

gegebenenfalls in Gegenwart eines Verdünnungsmittels und gegebenenfalls in Gegenwart eines Säurebindemittels acyliert.

**4.** Schädlingsbekämpfungsmittel, gekennzeichnet durch einen Gehalt an mindestens einem 2-Methoximinocarbonsäureester der allgemeinen Formel (I) nach Anspruch 1.

**5.** Verfahren zur Bekämpfung von phytopathogenen Pilzen, dadurch gekennzeichnet, daß man 2-Methoximinocarbonsäureester der allgemeinen Formel (I) nach Anspruch 1 auf phytopathogene Pilze und/oder deren Lebensraum einwirken läßt.

**6.** Verwendung von 2-Methoximinocarbonsäureestern der allgemeinen Formel (I) nach Anspruch 1 zur Bekämpfung von Schädlingen, insbesondere von phytopathogenen Pilzen.

**7.** Verfahren zur Herstellung von Schädlingsbekämpfungsmitteln, dadurch gekennzeichnet, daß man 2-Methoximinocarbonsäureester der allgemeinen Formel (I) nach Anspruch 1 mit Streckmitteln und/oder

oberflächenaktiven Mitteln vermischt.

**8.** Verfahren zur Herstellung von fungiziden Mitteln, dadurch gekennzeichnet, daß man 2-Methoximinocarbonsäureester der allgemeinen Formel (I) nach Anspruch 1 mit Streckmitteln und/oder oberflächenaktiven Mitteln vermischt.

**9.** Verbindungen der Formel (V)

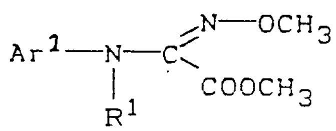

(V)

in welcher

| | |
|---|---|
| $R^1$ | für Wasserstoff oder Methyl steht und |
| $Ar^2$ | für einen der Reste |

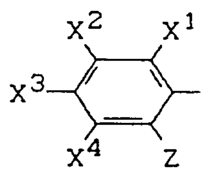

oder

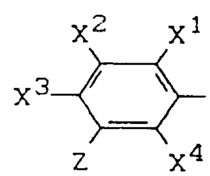

steht, wobei

| | |
|---|---|
| Z | jeweils für Hydroxy, Amino oder N-Methylamino steht und |
| $X^1$, $X^2$, $X^3$ und $X^4$ | die oben angegebene Bedeutung haben, |

**Patentansprüche für folgenden Vertragsstaat : ES**

**1.** Verfahren zur Herstellung von 2-Methoximinocarbonsäureestern der allgemeinen Formel (I)

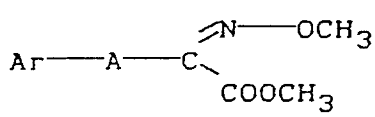

(I)

in welcher

| | |
|---|---|
| Ar | für einen der Reste |

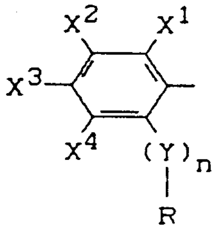 ; 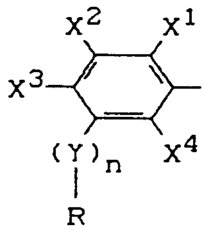 ; 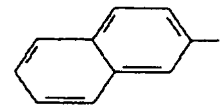 oder

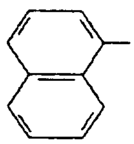

A steht und für einen der Reste $-CH_2-$ ;

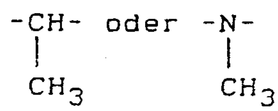

Y steht, wobei für einen der Reste

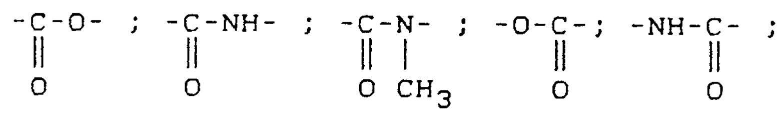

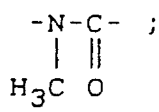

$-O-CH_2$ ; $-CH_2-O-$ ; $-S-CH_2-$;

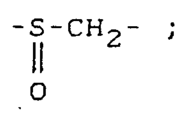

$-SO_2-CH_2-$ ; -O- ; -CH=CH- oder $-CH_2-CH_2-$ steht,

n für eine Zahl 0 oder 1 steht,

R für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 9 gleichen oder verschiedenen Halogenatomen, für geradkettiges oder verzweigtes Alkenyl mit 2 bis 8 Kohlenstoffatomen, für geradkettiges oder verzweigtes Halogenalkenyl mit 2 bis 8 Kohlenstoffatomen und 1 bis 9 gleichen oder verschiedenen Halogenatomen, für gegebenenfalls einfach oder mehrfach, gleich oder verschieden durch Halogen und/oder geradkettiges oder verzweigtes Alkyl mit 1 bis 6 Kohlenstoffatomen substituiertes Cycloalkyl mit 3 bis 7 Kohlenstoffatomen oder für gegebenenfalls einfach oder mehrfach, gleich oder verschieden substituiertes Heteroaryl mit 2 bis 9 Kohlenstoffatomen und 1 bis

5 gleichen oder verschiedenen Heteroatomen - insbesondere Stickstoff, Sauerstoff und/oder Schwefel - steht, wobei als Heteroarylsubstituenten jeweils infrage kommen: Halogen sowie jeweils geradkettiges oder ver-zweigtes Alkyl oder Alkoxy mit jeweils 1 bis 6 Kohlenstoffatomen;

R außerdem für gegebenenfalls einfach oder mehrfach, gleich oder verschieden substituiertes Aryl mit 6 bis 10 Kohlenstoffatomen steht, wobei als Arylsubstituenten infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 6 Kohlenstoffatomen und 1 bis 9 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 6 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 6 Kohlenstoffatomen in den einzelnen Alkyl- bzw. Alkenylteilen, wobei als Substituenten in den einzelnen Arylteilen jeweils infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 6 Kohlenstoffatomen und 2 bis 9 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)amino, Aminocarbonyl, N-Alkyl-aminocarbonyl, N,N-Dialkylaminocarbonyl oder Alkoximinoalkyl mit jeweils 1 bis 6 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 6 Kohlenstoffatomen in den einzelnen Alkyl- bzw. Alkenylteilen, wobei als Substituenten in den einzelnen Arylteilen jeweils infrage kommen:

Halogen sowie jeweils geradkettiges oder verzweigtes Alkyl oder Alkoxy mit jeweils 1 bis 6 Kohlenstoffatomen;

R außerdem für den Fall, daß n = 0 ist, auch für Fluor, Chlor, Brom oder Iod steht und

$X^1$, $X^2$, $X^3$ und $X^4$ unabhängig voneinander jeweils für Wasserstoff, Fluor, Chlor, Brom, Iod, Hydroxy oder Methoxy stehen, wobei jedoch für den Fall, daß $X^2$ für Brom steht, $X^3$ nicht gleichzeitig für Hydroxy oder Methoxy steht,

dadurch gekennzeichnet, daß man

(a) zun Erhalt von 2-Methoximinocarbonsäureester der Formel (Ia),

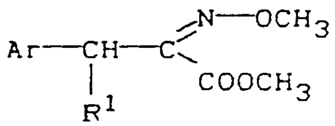

(Ia)

in welcher

R[1] für Wasserstoff oder Methyl steht und

Ar die oben angegebene Bedeutung hat,

Acrylester-Derivate der Formel (II),

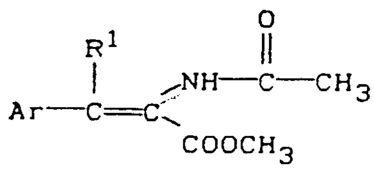

(II)

in welcher

R[1] und Ar die oben angegebene Bedeutung haben,

mit einem Säureadditionssalz des O-Methylhydroxylamins gegebenenfalls in Gegenwart eines Verdünnungsmittels sowie gegebenenfalls in Gegenwart eines Reaktionshilfmittels umsetzt;

oder daß man

(b) zum Erhalt von 2-Methoximinocarbonsäureestern der Formel (Ib),

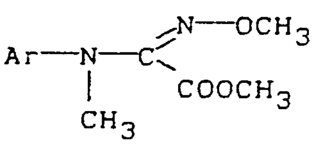

(Ib)

in welcher

Ar die oben angegebene Bedeutung hat,

zunächst in einer 1. Stufe aromatische Amine der Formel (III),

$Ar—NH—CH_3$ (III)

in welcher

Ar die oben angegebene Bedeutung hat,

mit 2-Chlor-2-hydroximino-essigsäuremethylester der Formel (IV)

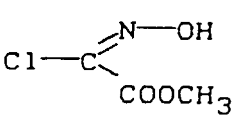

(IV)

gegebenenfalls in Gegenwart eines Verdünnungsmittels und gegebenenfalls in Gegenwart eines Säurebindemittels umsetzt und anschließend in einer 2. Stufe mit einem Methylierungsmittel gegebenenfalls in Gegenwart eines Säurebindemittels an der Hydroximinogruppe methyliert;

oder daß man

(c) zum Erhalt von 2-Methoximinocarbonsäureestern der Formel (Ic),

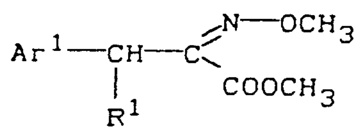

(Ic)

in welcher

R[1] für Wasserstoff oder Methyl steht und

Ar[1] für einen der Reste

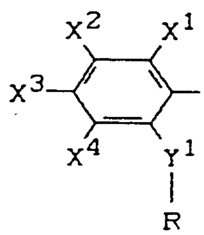

oder

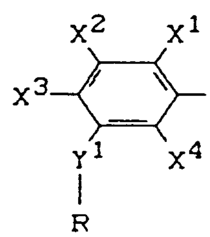

steht, wobei

Y[1] jeweils für einen der Reste

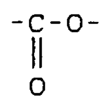

;

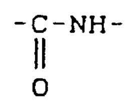

oder

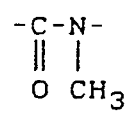

steht und

X[1], X[2], X[3], X[4] und R die oben angegebene Bedeutung haben,

substituierte Phenol-oder Anilinderivate der Formel (V),

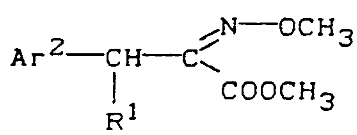

(V)

in welcher

R[1] für Wasserstoff oder Methyl steht und

Ar[2] für einen der Reste

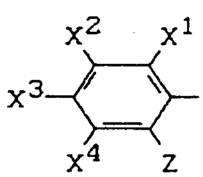

oder

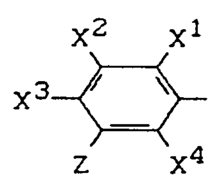

steht,

wobei

Z jeweils für Hydroxy, Amino oder N-Methylamino steht und

X[1], X[2], X[3] und X[4] die oben angegebene Bedeutung haben,

mit Säurechloriden der Formel (VI),

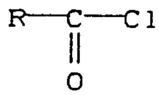 (VI)

in welcher

R die oben angegebene Bedeutung haben,

gegebenenfalls in Gegenwart eines Verdünnungsmittels und gegebenenfalls in Gegenwart eines Säurebindemittels acyliert.

**2.** Verfahren gemäß Anspruch 1, in welcher

Ar für einen Rest

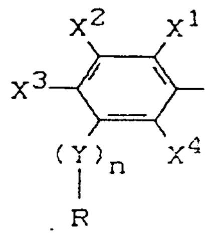

steht und

A für einen der Reste $-CH_2-$ ;

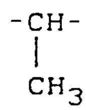 oder 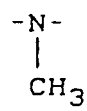

steht, wobei

Y für einen der Reste

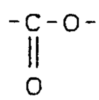 ; 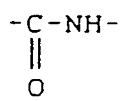 ; 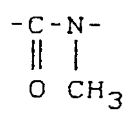 ; 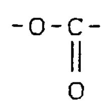 ; 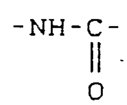 ;

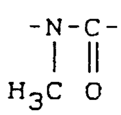 ;

$-O-CH_2$ ; $-CH_2-O-$ ; $-S-CH_2-$;

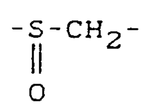 ;

$-SO_2-CH_2-$ ; -O- ; -CH = CH- oder $-CH_2-CH_2-$ steht,

n für eine Zahl 0 oder 1 steht,

R für geradkettiges oder verzweigtes Alkyl mit 1 bis 6 Kohlenstoffatomen, für geradkettiges oder verzweigtes Halogenalkyl mit 1 bis 6 Kohlenstoffatomen

und 1 bis 9 gleichen oder verschiedenen Halogenatomen, für geradkettiges oder verzweigtes Alkenyl mit 2 bis 6 Kohlenstoffatomen, für geradkettiges oder verzweigtes Halogenalkenyl mit 2 bis 6 Kohlenstoffatomen und 1 bis 9 gleichen oder verschiedenen Halogenatomen, für gegebenenfalls ein- bis fünffach gleich oder verschieden durch geradkettiges oder verzweigtes Alkyl mit 1 bis 4 Kohlenstoffatomen und/oder durch Halogen substituiertes Cycloalkyl mit 3 bis 7 Kohlenstoffatomen oder für gegebenenfalls ein- bis dreifach gleich oder verschieden substituiertes Heteroaryl mit 2 bis 9 Kohlenstoffatomen und 1 bis 4 gleichen oder verschiedenen Heteroatomen - insbesondere Stickstoff, Sauerstoff und/oder Schwefel - steht, wobei als Heteroarylsubstituenten jeweils infrage kommen: Fluor, Chlor, Brom sowie jeweils geradkettiges oder verzweigtes Alkyl oder Alkoxy mit jeweils 1 bis 4 Kohlenstoffatomen;

R außerdem für gegebenenfalls ein- bis fünffach, gleich oder verschieden substituiertes Aryl mit 6 oder 10 Kohlenstoffatomen steht, wobei als Arylsubstituenten jeweils infrage kommen:

Fluor, Chlor, Brom, Cyano, Nitro, jeweils geradkettiges oder verzweigtes Alkyl, Alkoxy, Alkylthio,Alkylsulfinyl oder Alkylsulfonyl mit jeweils 1 bis 4 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 4 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 4 Kohlenstoffatomen und 1 bis 9 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 4 Kohlenstoffatomen und 1 bis 7 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 4 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 4 Kohlenstoffatomen in den einzelnen Alkylbzw. Alkenylteilen, wobei als Substituenten in den einzelnen Arylteilen jeweils infrage kommen:

Fluor, Chlor, Brom, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 4 Kohlenstoffatomen, jeweils geradkettiges oder verzweigtes Halogenalkyl, Halogenalkoxy, Halogenalkylthio, Halogenalkylsulfinyl, Halogenalkylsulfonyl mit jeweils 1 bis 4 Kohlenstoffatomen und 1 bis 9 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 4 Kohlenstoffatomen und 1 bis 7 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 4 Kohlenstoffatomen in den einzelnen Alkylteilen sowie jeweils gegebenenfalls einfach oder mehrfach, gleich oder verschieden substituiertes Aryl, Aryloxy, Arylthio, Arylcarbonyl, Arylcarbonyloxy, Arylcarbonylamino, Arylcarbonyl-(N-alkyl)amino, Arylaminocarbonyl, N-Aryl-N-alkylaminocarbonyl, Arylalkyl oder Arylalkenyl mit jeweils 6 bis 10 Kohlenstoffatomen im Arylteil und gegebenenfalls bis zu 4 Kohlenstoffatomen in den einzelnen Alkyl- bzw. Alkenylteilen, wobei als Substituenten in den einzelnen Arylteilen jeweils infrage kommen:

Fluor, Chlor, Brom, sowie jeweils geradkettiges oder verzweigtes Alkyl oder

Alkoxy mit jeweils 1 bis 4 Kohlenstoffatomen;

R außerdem für den Fall, daß n = O ist, auch für Chlor oder Brom steht und

$X^1$, $X^2$, $X^3$ und $X^4$ unabhängig voneinander jeweils für Wasserstoff, Chlor, Brom, Hydroxy oder Methoxy stehen, wobei jedoch für den Fall, daß $X^2$ für Brom steht, $X^3$ nicht gleichzeitig für Hydroxy oder Methoxy steht.

**3.** Schädlingsbekämpfungsmittel, gekennzeichnet durch einen Gehalt an mindestens einem 2-Methoximinocarbonsäureester der allgemeinen Formel (I) nach Anspruch 1.

**4.** Verfahren zur Bekämpfung von phytopathogenen Pilzen, dadurch gekennzeichnet, daß man 2-Methoximinocarbonsäureester der allgemeinen Formel (I) nach Anspruch 1 auf phytopathogene Pilze und/oder deren Lebensraum einwirken läßt.

**5.** Verwendung von 2-Methoximinocarbonsäureestern der allgemeinen Formel (I) nach Anspruch 1 zur Bekämpfung von Schädlingen, insbesondere von phytopathogenen Pilzen.

**6.** Verfahren zur Herstellung von Schädlingsbekämpfungsmitteln, dadurch gekennzeichnet, daß man 2-Methoximinocarbonsäureester der allgemeinen Formel (I) nach Anspruch 1 mit Streckmitteln und/oder oberflächenaktiven Mitteln vermischt.

**7.** Verfahren zur Herstellung von fungiziden Mitteln, dadurch gekennzeichnet, daß man 2-Methoximinocarbonsäureester der allgemeinen Formel (I) nach Anspruch 1 mit Streckmitteln und/oder oberflächenaktiven Mitteln vermischt.

**8.** Verfahren zur Herstellung von Verbindungen der Formel (V)

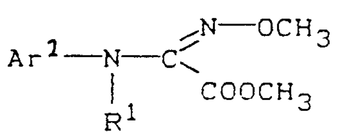

(V)

in welcher

$R^1$ für Wasserstoff oder Methyl steht und

$Ar^2$ für einen der Reste

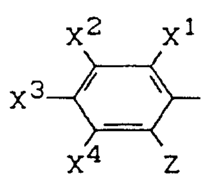

oder

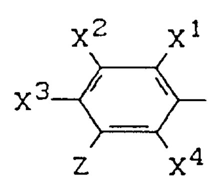

steht, wobei

Z jeweils für Hydroxy, Amino oder N-Methylamino steht und

$X^1$, $X^2$, $X^3$ und $X^4$ die in Anspruch 1 angegebene Bedeutung haben,

dadurch gekennzeichnet, daß

man Azlactone der Formel (IX),

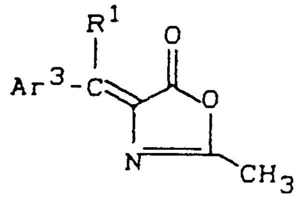

(IX)

in welcher

$R^1$ die oben angegebene Bedeutung hat und

$Ar^3$ für einen der Reste

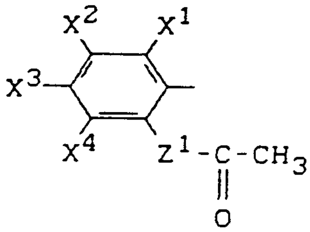

oder

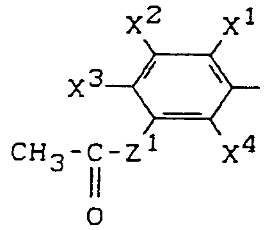

steht, wobei

$Z^1$ jeweils für Sauerstoff, für eine NH-Gruppe oder für eine

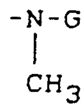
Gruppe

steht und

$X^1$, $X^2$, $X^3$ und $X^4$ die oben angegebene Bedeutung haben,

mit Methanol in Gegenwart einer Base und anschließend in Gegenwart einer Säure bei Temperaturen zwischen 0 °C und 50 °C umsetzt und anschließend die so erhältlichen Acrylester-Derivate der Formel (X),

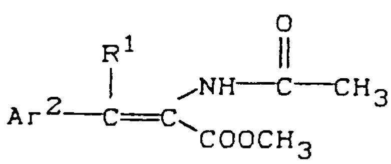

(X)

in welcher

$R^1$ die oben angegebene Bedeutung hat und

$Ar^2$ für einen der Reste

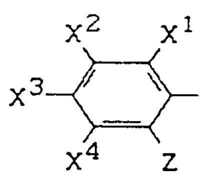

oder

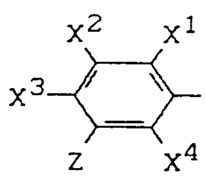

steht, wobei

Z jeweils für Hydroxy, Amino oder N-Methylamino steht und

$X^1$, $X^2$, $X^3$ und $X^4$ die oben angegebene Bedeutung haben,

mit einem Säureadditionssalz des O-Methylhydroxylamins, gegebenenfalls in Gegenwart eines Verdünnungsmittels und gegebenenfalls in Gegenwart eines Reaktionshilfsmittels in Analogie zur Durchführung des erfindungsgemäßen Verfahrens (a) bei Temperaturen zwischen 20 °C und 60 °C umsetzt.

## Claims

**Claims for the following Contracting States : BE, CH, DE, DK, FR, GB, GR, IT, LI, NL,**

**1.** 2-Methoximinocarboxylic esters of the general formula (I)

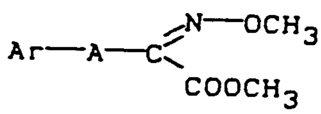

(I)

in which

Ar represents one of the radicals

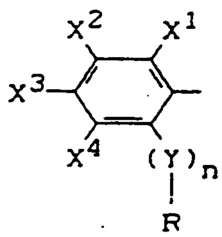 ; 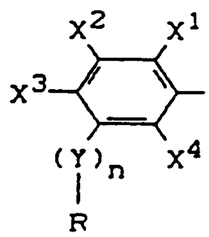 ; 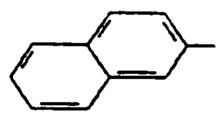 or

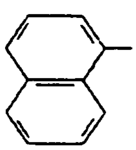

and

A represents one of the radicals $-CH_2-$;

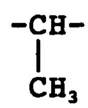 or 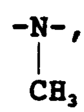,

where

Y represents one of the radicals

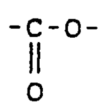 ; 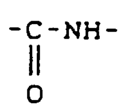 ; 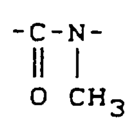 ; 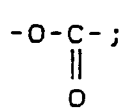 ; 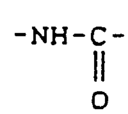 ;

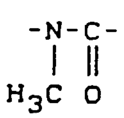 ;

$-O-CH_2-$ ; $-CH_2-O-$ ; $-S-CH_2-$;

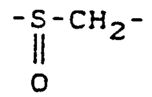 ;

$-SO_2-CH_2-$ ; -O- ; -CH=CH- or $-CH_2-CH_2-$,

n represents the number 0 or 1,

R represents straight-chain or branched alkyl having 1 to 8 carbon atoms, straight-chain or branched halogenoalkyl having 1 to 8 carbon atoms and 1 to 9 identical or different halogen atoms, straight-chain or branched alkenyl having 2 to 8 carbon atoms, straight-chain or branched halogenoalkenyl having 2 to 8 carbon atoms and 1 to 9 identical or different halogen atoms, or represents cycloalkyl which has 3 to 7 carbon atoms and which is optionally monosubstituted or polysubstituted by identical or different substituents from the series comprising halogen and/or straight-chain or branched alkyl having 1 to 6 carbon atoms, or represents heteroaryl which has 2 to 9 carbon atoms and 1 to 5 identical or different hetero atoms - in particular nitrogen, oxygen and/or sulphur - and which is optionally monosubstituted or polysubstituted by identical or different substituents, suitable heteroaryl substituents in each case being: halogen and in each case straight-chain or branched alkyl or alkoxy each of which has 1 to 6 carbon atoms;

R furthermore represents aryl which has 6 to 10 carbon atoms and which is optionally monosubstituted or polysubstituted by identical or different substituents, suitable aryl substituents being:

halogen, cyano, nitro, in each case straight-chain or branched alkyl, alkoxy, alkylthio, alkylsulphinyl or alkylsulphonyl each of which has 1 to 6 carbon atoms, in each case straight-chain or branched alkenyl, alkinyl, alkenyloxy, alkinyloxy, alkenylthio or alkinylthio each of which has 2 to 6 carbon atoms, in each case straight-chain or branched halogenoalkyl, halogenoalkoxy, halogenoalkylthio, halogenoalkylsulphinyl and halogenoalkylsulphonyl each of which has 1 to 6 carbon atoms and 1 to 13 identical or different halogen atoms, in each case straight-chain or branched halogenoalkenyl, halogenoalkinyl, halogenoalkenyloxy, halogenoalkinyloxy, halogenoalkenylthio or halogenoalkinylthio each of which has 2 to 6 carbon atoms and 1 to 9 identical or different halogen atoms, in each case straight-chain or branched alkylcarbonyl, alkoxycarbonyl, alkylcarbonyloxy, alkylcarbonylamino, alkylcarbonyl-(N-alkyl)-amino, aminocarbonyl, N-alkyl-aminocarbonyl, N,N-dialkylaminocarbonyl or alkoximinoalkyl each of which has 1 to 6 carbon atoms in the individual alkyl moieties, and aryl, aryloxy, arylthio, arylcarbonyl, arylcarbonyloxy, arylcarbonylamino, arylcarbonyl-(N-alkyl)amino, arylaminocarbonyl, N-aryl-N-alkylaminocarbonyl, arylalkyl or arylalkenyl each of which has 6 to 10 carbon atoms in the aryl moiety and, if appropriate, up to 6 carbon atoms in the individual alkyl or alkenyl moieties and each of which is optionally monosubstituted or polysubstituted by identical or different substituents, suitable substituents in the individual aryl moieties in each case being:

halogen, cyano, nitro, in each case straight-chain or branched alkyl, alkoxy, alkylthio, alkylsulphinyl, or alkylsulphonyl each of which has 1 to 6 carbon atoms, in each case straight-chain or branched alkenyl, alkinyl, alkenyloxy, alkinyloxy, alkenylthio or alkinylthio each of which has 2 to 6 carbon atoms, in each case straight-chain or branched halogenoalkyl, halogenoalkoxy, halogenoalkylthio, halogenoalkylsulphinyl and halogenoalkylsulphonyl each of which has 1 to 6 carbon atoms and 1 to 13 identical or different halogen atoms, in each case straight-chain or branched halogenoalkenyl, halogenoalkinyl, halogenoalkenyloxy, halogenoalkinyloxy, halogenoalkenylthio or halogenoalkinylthio each of which has 2 to 6 carbon atoms and 2 to 9 identical or different halogen atoms, in each case straight-chain or branched alkylcarbonyl, alkoxycarbonyl, alkylcarbonyloxy, alkylcarbonylamino, alkylcarbonyl(N-alkyl)-amino,

aminocarbonyl, N-alkyl-aminocarbonyl, N,N-dialkylaminocarbonyl or alkoximinoalkyl each of which has 1 to 6 carbon atoms in the individual alkyl moieties, and aryl, aryloxy, arylthio, arylcarbonyl, arylcarbonyloxy, arylcarbonylamino, arylcarbonyl(N-alkyl)-amino, arylaminocarbonyl, N-aryl-N-alkylaminocarbonyl, arylalkyl or arylalkenyl each of which has 6 to 10 carbon atoms in the aryl moiety and if appropriate up to 6 carbon atoms in the individual alkyl or alkenyl moieties and each of which is optionally monosubstituted or polysubstituted by identical or different substituents, suitable substituents in the individual aryl moieties in each case being:
halogen and in each case straight-chain or branched alkyl or alkoxy each of which has 1 to 6 carbon atoms;

R in the event that n is 0, furthermore also represents fluorine, chlorine, bromine or iodine and

$X^1$, $X^2$, $X^1$ and $X^4$ independently of one another in each case represent hydrogen, fluorine, chlorine, bromine, iodine, hydroxyl or methoxy, but where, in the event that $X^2$ represents bromine, $X^3$ does not simultaneously represent hydroxyl or methoxy.

**2.** 2-Methoximinocarboxylic esters of the general formula (I), according to Claim 1, in which

Ar represents a radical

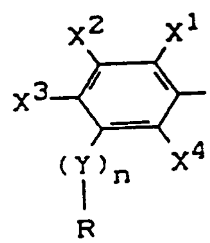

and

A represents one of the radicals $-CH_2-$;

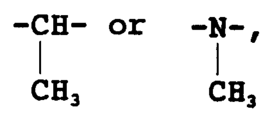

where

Y represents one of the radicals

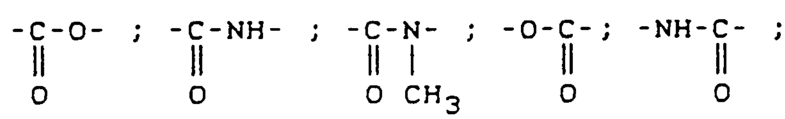

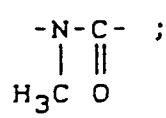

$-O-CH_2$ ; $-CH_2-O-$ ; $-S-CH_2-$;

$$-\underset{\underset{O}{\|}}{S}-CH_2- \ ;$$

$-SO_2-CH_2-$ ; -O- ; -CH = CH- or $-CH_2-CH_2-$ ,

n represents the number 0 or 1,

R represents straight-chain or branched alkyl having 1 to 6 carbon atoms, straight-chain or branched halogenoalkyl having 1 to 6 carbon atoms and 1 to 9 identical or different halogen atoms, straight-chain or branched alkenyl having 2 to 6 carbon atoms, straight-chain or branched halogenoalkenyl having 2 to 6 carbon atoms and 1 to 9 identical or different halogen atoms, cycloalkyl which has 3 to 7 carbon atoms and which is optionally monosubstituted to pentasubstituted by identical or different substituents from the series comprising straight-chain or branched alkyl having 1 to 4 carbon atoms and/or halogen, or heteroaryl which has 2 to 9 carbon atoms and 1 to 4 identical or different hetero atoms - in particular nitrogen, oxygen and/or sulphur - and which is optionally monosubstituted to trisubstituted by identical or different substituents, suitable heteroaryl substituents in each case being:
fluorine, chlorine, bromine and in each case straight-chain or branched alkyl or alkoxy each of which has 1 to 4 carbon atoms;

R furthermore represents aryl which has 6 or 10 carbon atoms and which is optionally monosubstituted to pentasubstituted by identical or different substituents, suitable aryl substituents in each case being:
fluorine, chlorine, bromine, cyano, nitro, in each case straight-chain or branched alkyl, alkoxy, alkylthio, alkylsulphinyl or alkylsulphonyl each of which has 1 to 4 carbon atoms, in each case straight-chain or branched alkenyl, alkinyl, alkenyloxy, alkinyloxy, alkenylthio or alkinylthio each of which has 2 to 4 carbon atoms, in each case straight-chain or branched halogenoalkyl, halogenoalkoxy, halogenoalkylthio, halogenoalkylsulphinyl and halogenoalkylsulphonyl each of which has 1 to 4 carbon atoms and 1 to 9 identical or different halogen atoms, in each case straight-chain or branched halogenoalkenyl, halogenoalkinyl, halogenoalkenyloxy, halogenoalkinyloxy, halogenoalkenylthio or halogenoalkinylthio each of which has 2 to 4 carbon atoms and 1 to 7 identical or different halogen atoms, in each case straight-chain or branched alkylcarbonyl, alkoxycarbonyl, alkylcarbonyloxy, alkylcarbonylamino, alkylcarbonyl-(N-alkyl)-amino, aminocarbonyl, N-alkyl-aminocarbonyl, N,N-dialkylaminocarbonyl or alkoximinoalkyl each of which has 1 to 4 carbon atoms in the individual alkyl moieties, and aryl, aryloxy, arylthio, arylcarbonyl, arylcarbonyloxy, arylcarbonylamino, arylcarbonyl-(N-alkyl)amino, arylaminocarbonyl, N-aryl-N-alkylaminocarbonyl, arylalkyl or arylalkenyl each of which has 6 to 10 carbon atoms in the aryl moiety and if appropriate up to 4 carbon atoms in the individual alkyl or alkenyl moieties and each of which is optionally monosubstituted or polysubstituted by identical or different substituents, suitable substituents in the individual aryl moieties in each case being:
fluorine, chlorine, bromine, cyano, nitro, in each case straight-chain or branched alkyl, alkoxy, alkylthio, alkylsulphinyl or alkylsulphonyl each of which has 1 to 4 carbon atoms, in each case straight-chain or branched alkenyl, alkinyl, alkenyloxy, alkinyloxy, alkenylthio or alkinylthio each of which has 2 to 4 carbon atoms, in each case straight-chain or branched halogenoalkyl, halogenoalkoxy, halogenoalkylthio, halogenoalkylsulphinyl and halogenoalkylsulphonyl each of which has 1 to 4 carbon atoms and 1 to 9 identical or different halogen atoms, in each case straight-chain or branched halogenoalkenyl, halogenoalkinyl, halogenoalkenyloxy, halogenoalkinyloxy, halogenoalkenylthio or halogenoalkinylthio each of which has 2 to 4 carbon atoms and 1 to 7 identical or different halogen atoms, in each case straight-chain or branched alkylcarbonyl, alkoxycarbonyl, alkylcarbonyloxy, alkylcarbonylamino,

alkylcarbonyl-(N-alkyl)-amino, aminocarbonyl, N-alkyl-aminocarbonyl, N,N-dialkylaminocarbonyl or alkoximinoalkyl each of which has 1 to 4 carbon atoms in the individual alkyl moieties, and aryl, aryloxy, arylthio, arylcarbonyl, arylcarbonyloxy, arylcarbonylamino, arylcarbonyl-(N-alkyl)amino, arylaminocarbonyl, N-aryl-N-alkylaminocarbonyl, arylalkyl or arylalkenyl each of which has 6 to 10 carbon atoms in the aryl moiety and if appropriate up to 4 carbon atoms in the individual alkyl or alkenyl moieties and each of which is optionally monosubstituted or polysubstituted by identical or different substituents, suitable substituents in the individual aryl moieties in each case being:
fluorine, chlorine, bromine and in each case straight-chain or branched alkyl or alkoxy each of which has 1 to 4 carbon atoms;

R in the event that n is 0, furthermore also represents chlorine or bromine and

$X^1$, $X^2$, $X^3$ and $X^4$ independently of one another in each case represent hydrogen, chlorine, bromine, hydroxyl or methoxy, but where, in the event that $X^2$ represents bromine, $X^3$ does not simultaneously represent hydroxyl or methoxy.

**3.** Process for the preparation of 2-methoximinocarboxylic esters of the general formula (I)

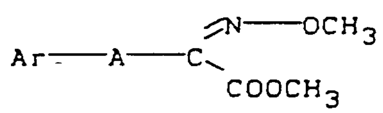

(I)

in which
Ar and A have the meaning given in Claim 1,
characterised in that
(a) to obtain 2-methoximinocarboxylic esters of the formula (Ia),

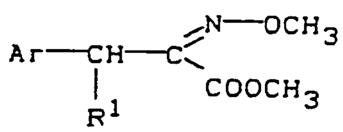

(Ia)

in which
$R^1$ represents hydrogen or methyl and
Ar has the abovementioned meaning,
acrylic ester derivatives of the formula (II),

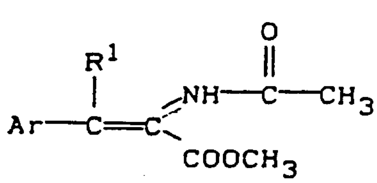

(II)

in which
$R^1$ and Ar have the abovementioned meaning,
are reacted with an acid addition salt of O-methylhydroxylamine, if appropriate in the presence of a diluent and if appropriate in the presence of a reaction auxiliary;
or in that

(b) to obtain 2-methoximinocarboxylic esters of the formula (Ib),

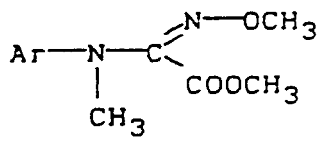

(Ib)

in which
Ar has the abovementioned meaning,
aromatic amines of the formula (III),

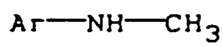

(III)

in which
Ar has the abovementioned meaning,
are first reacted in a 1st step,
with methyl 2-chloro-2-hydroximinoacetate of the formula (IV)

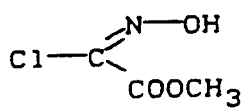

(IV)

if appropriate in the presence of a diluent and if appropriate in the presence of an acid-binding agent, and, in a 2nd step, the product is subsequently methylated on the hydroximino group using a methylation agent, if appropriate in the presence of an acid-binding agent;
or in that
(c) to obtain 2-methoximinocarboxylic esters of the formula (Ic),

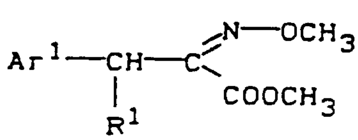

(Ic)

in which
$R^1$ represents hydrogen or methyl and
$Ar^1$ represents one of the radicals

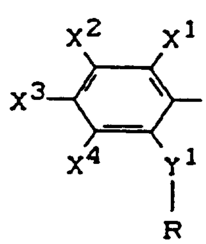

or

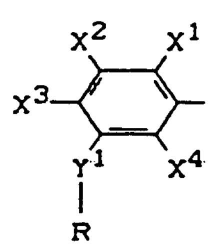

where
$Y^1$ in each case represents one of the radicals

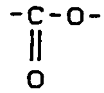 ; 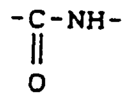 or 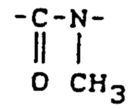

and

$X^1$, $X^2$, $X^3$, $X^4$ and R have the abovementioned meaning,

substituted phenol or aniline derivatives of the formula (V),

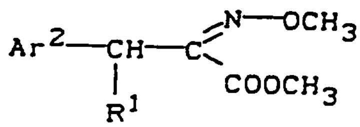 (V)

in which

$R^1$ represents hydrogen or methyl and

$Ar^2$ represents one of the radicals

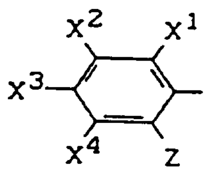 or 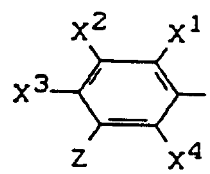

where

Z in each case represents hydroxyl, amino or N-methylamino and

$X^1$, $X^2$, $X^3$ and $X^4$ have the abovementioned meaning,

are acylated with acid chlorides of the formula (VI),

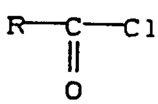 (VI)

in which

R has the abovementioned meaning,

if appropriate in the presence of a diluent and if appropriate in the presence of an acid-binding agent.

**4.** Pesticides, characterised in that they contain at least one 2-methoximinocarboxylic ester of the general formula (I) according to Claim 1.

**5.** Method of combating phytopathogenic fungi, characterised in that 2-methoximinocarboxylic esters of the general formula (I) according to Claim 1 are allowed to act on phytopathogenic fungi and/or their environment.

**6.** Use of 2-methoximinocarboxylic esters of the general formula (I) according to Claim 1, for combating pests, in particular phytopathogenic fungi.

**7.** Process for the preparation of pesticides, characterised in that 2-methoximinocarboxylic esters of the general formula (I) according to Claim 1 are mixed with extenders and/or surface-active agents.

**8.** Process for the preparation of fungicidal agents, characterised in that 2-methoximinocarboxylic esters of the general formula (I) according to Claim 1 are mixed with extenders and/or surface-active agents.

**9.** Compounds of the formula (V)

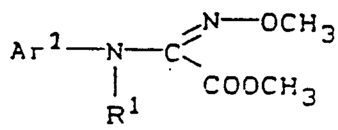

in which

| | |
|---|---|
| $R^1$ | represents hydrogen or methyl and |
| $Ar^2$ | represents one of the radicals |

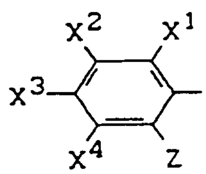

or

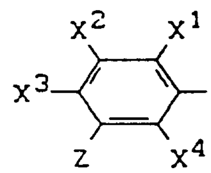

where

| | |
|---|---|
| Z | in each case represents hydroxyl, amino or N-methylamino and |
| $X^1$, $X^2$, $X^3$ and $X^4$ | have the abovementioned meaning. |

**Claims for the following Contracting State : ES**

**1.** Process for the preparation of 2-methoximinocarboxylic esters of the general formula (I)

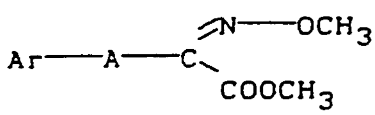

(I)

in which

| | |
|---|---|
| Ar | represents one of the radicals |

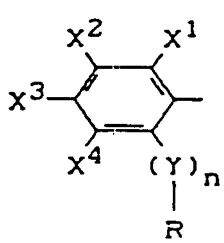

;

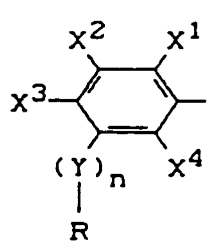

;

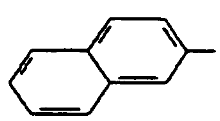

or

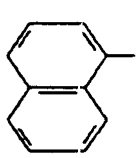

and

A represents one of the radicals $-CH_2-$;

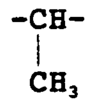 or 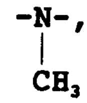,

where

Y represents one of the radicals

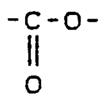 ; 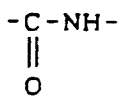 ; 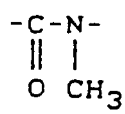 ; 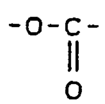 ; 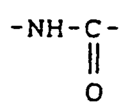 ;

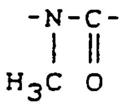 ;

$-O-CH_2$ ; $-CH_2-O-$ ; $-S-CH_2-$;

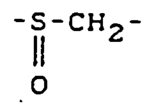 ;

$-SO_2-CH_2-$ ; $-O-$ ; $-CH=CH-$ or $-CH_2-CH_2-$ ,

n represents the number 0 or 1,

R represents straight-chain or branched alkyl having 1 to 8 carbon atoms, straight-chain or branched halogenoalkyl having 1 to 8 carbon atoms and 1 to 9 identical or different halogen atoms, straight-chain or branched alkenyl having 2 to 8 carbon atoms, straight-chain or branched halogenoalkenyl having 2 to 8 carbon atoms and 1 to 9 identical or different halogen atoms, or represents cycloalkyl which has 3 to 7 carbon atoms and which is optionally monosubstituted or polysubstituted by identical or different substituents from the series comprising halogen and/or straight-chain or branched alkyl having 1 to 6 carbon atoms, or represents heteroaryl which has 2 to 9 carbon atoms and 1 to 5 identical or different hetero atoms - in particular nitrogen, oxygen and/or sulphur - and which is optionally monosubstituted or polysubstituted by identical or different substituents, suitable heteroaryl substituents in each case being: halogen and in each case straight-chain or branched alkyl or alkoxy each of which has 1 to 6 carbon atoms;

R furthermore represents aryl which has 6 to 10 carbon atoms and which is optionally monosubstituted or polysubstituted by identical or different substituents, suitable aryl substituents being:
halogen, cyano, nitro, in each case straight-chain or branched alkyl, alkoxy, alkylthio, alkylsulphinyl or alkylsulphonyl each of which has 1 to 6 carbon atoms, in each case straight-chain or branched alkenyl, alkinyl, alkenyloxy, alkinyloxy, alkenylthio or alkinylthio each of which has 2 to 6 carbon atoms, in each case straight-chain or branched halogenoalkyl, halogenoalkoxy, halogenoalkylthio, halogenoalkylsulphinyl and halogenoalkylsulphonyl each of which has 1 to 6 carbon atoms and 1 to 13 identical or different halogen atoms, in each case straight-chain or branched halogenoalkenyl, halogenoalkinyl, halogenoalkenyloxy, halogenoalkinyloxy, halogenoalkenylthio or halogenoalkinylthio each of which has 2 to 6 carbon atoms and 1 to 9 identical or different halogen atoms, in each case straight-chain or branched alkylcarbonyl, alkoxycarbonyl, alkylcarbonyloxy, alkylcarbonylamino, alkylcarbonyl-(N-alkyl)-amino, aminocarbonyl, N-alkyl-aminocarbonyl, N,N-dialkylaminocarbonyl or alkoximinoalkyl each of which has 1 to 6 carbon atoms in the individual alkyl moieties, and aryl, aryloxy, arylthio, arylcarbonyl, arylcarbonyloxy, arylcarbonylamino, arylcarbonyl-(N-alkyl)amino, arylaminocarbonyl, N-aryl-N-alkylaminocarbonyl, arylalkyl or arylalkenyl each of which has 6 to 10 carbon atoms in the aryl moiety and, if appropriate, up to 6 carbon atoms in the individual alkyl or alkenyl moieties and each of which is optionally monosubstituted or polysubstituted by identical or different substituents, suitable substituents in the individual aryl moieties in each case being:

halogen, cyano, nitro, in each case straight-chain or branched alkyl, alkoxy, alkylthio, alkylsulphinyl, or alkylsulphonyl each of which has 1 to 6 carbon atoms, in each case straight-chain or branched alkenyl, alkinyl, alkenyloxy, alkinyloxy, alkenylthio or alkinylthio each of which has 2 to 6 carbon atoms, in each case straight-chain or branched halogenoalkyl, halogenoalkoxy, halogenoalkylthio, halogenoalkylsulphinyl and halogenoalkylsulphonyl each of which has 1 to 6 carbon atoms and 1 to 13 identical or different halogen atoms, in each case straight-chain or branched halogenoalkenyl, halogenoalkinyl, halogenoalkenyloxy, halogenoalkinyloxy, halogenoalkenylthio or halogenoalkinylthio each of which has 2 to 6 carbon atoms and 2 to 9 identical or different halogen atoms, in each case straight-chain or branched alkylcarbonyl, alkoxycarbonyl, alkylcarbonyloxy, alkylcarbonylamino, alkylcarbonyl-(N-alkyl)-amino, aminocarbonyl, N-alkyl-aminocarbonyl, N,N-dialkylaminocarbonyl or alkoximinoalkyl each of which has 1 to 6 carbon atoms in the individual alkyl moieties, and aryl, aryloxy, arylthio, arylcarbonyl, arylcarbonyloxy, arylcarbonylamino, arylcarbonyl-(N-alkyl)-amino, arylaminocarbonyl, N-aryl-N-alkylaminocarbonyl, arylalkyl or arylalkenyl each of which has 6 to 10 carbon atoms in the aryl moiety and if appropriate up to 6 carbon atoms in the individual alkyl or alkenyl moieties and each of which is optionally monosubstituted or polysubstituted by identical or different substituents, suitable substituents in the individual aryl moieties in each case being:

halogen and in each case straight-chain or branched alkyl or alkoxy each of which has 1 to 6 carbon atoms;

R in the event that n is 0, furthermore also represents fluorine, chlorine, bromine or iodine and

$X^1$, $X^2$, $X^3$ and $X^4$ independently of one another in each case represent hydrogen, fluorine, chlorine, bromine, iodine, hydroxyl or methoxy, but where, in the event that $X^2$ represents bromine, $X^3$ does not simultaneously represent hydroxyl or methoxy,

characterised in that

(a) to obtain 2-methoximinocarboxylic esters of the formula (Ia),

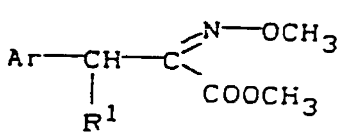

(Ia)

in which

$R^1$ represents hydrogen or methyl and

Ar has the abovementioned meaning,

acrylic ester derivatives of the formula (II),

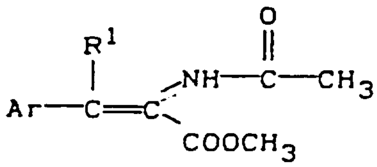

(II)

in which

R[1] and Ar have the abovementioned meaning,

are reacted with an acid addition salt of O-methylhydroxylamine, if appropriate in the presence of a diluent and if appropriate in the presence of a reaction auxiliary;

or in that

(b) to obtain 2-methoximinocarboxylic esters of the formula (Ib),

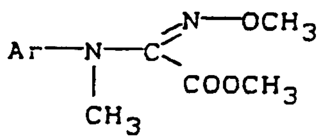

(Ib)

in which

Ar has the abovementioned meaning,

aromatic amines of the formula (III),

$Ar{-}NH{-}CH_3$ (III)

in which

Ar has the abovementioned meaning,

are first reacted in a 1st step,

with methyl 2-chloro-2-hydroximinoacetate of the formula (IV)

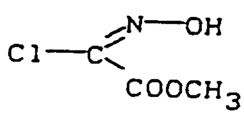

(IV)

if appropriate in the presence of a diluent and if appropriate in the presence of an acid-binding agent, and, in a 2nd step, the product is subsequently methylated on the hydroximino group using a methylation agent, if appropriate in the presence of an acid-binding agent;

or in that

(c) to obtain 2-methoximinocarboxylic esters of the formula (Ic),

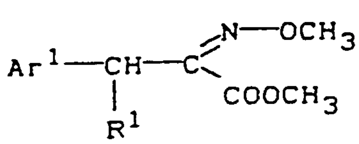

(Ic)

in which

R[1] represents hydrogen or methyl and

Ar[1] represents one of the radicals

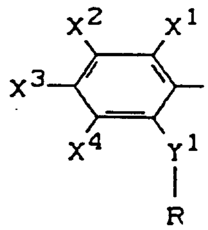

or

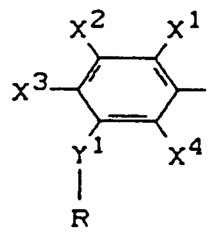

where

Y[1] in each case represents one of the radicals

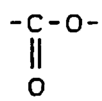

;

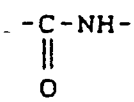

or

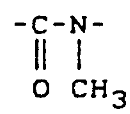

and

$X^1$, $X^2$, $X^3$, $X^4$ and R have the abovementioned meaning,

substituted phenol or aniline derivatives of the formula (V),

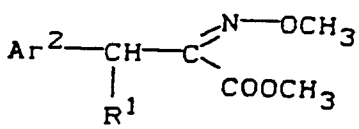

(V)

in which

$R^1$ represents hydrogen or methyl and

$Ar^2$ represents one of the radicals

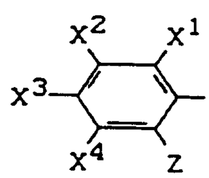

or

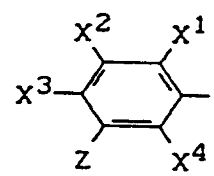

where

Z in each case represents hydroxyl, amino or N-methylamino and

$X^1$, $X^2$, $X^3$ and $X^4$ have the abovementioned meaning,

are acylated with acid chlorides of the formula (VI),

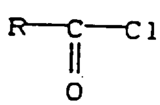

(VI)

in which

R has the abovementioned meaning,

if appropriate in the presence of a diluent and if appropriate in the presence of an acid-binding agent.

**2.** Process according to Claim 1, in which

Ar represents a radical

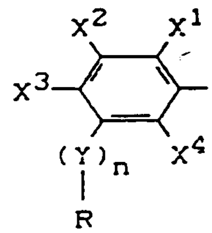

and

A represents one of the radicals $-CH_2-$;

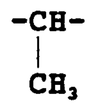 or 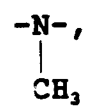

where

Y represents one of the radicals

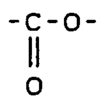 ; 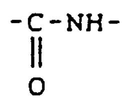 ; 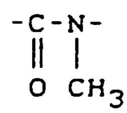 ; 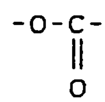 ; 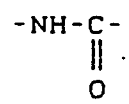 ;

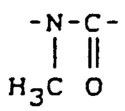 ;

$-O-CH_2$ ; $-CH_2-O-$ ; $-S-CH_2-$;

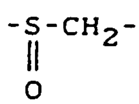 ;

$-SO_2-CH_2-$ ; $-O-$ ; $-CH=CH-$ or $-CH_2-CH_2-$,

n represents the number 0 or 1,

R represents straight-chain or branched alkyl having 1 to 6 carbon atoms, straight-chain or branched halogenoalkyl having 1 to 6 carbon atoms and 1 to 9 identical or different halogen atoms, straight-chain or branched alkenyl having 2 to 6 carbon atoms, straight-chain or branched halogenoalkenyl having 2 to 6 carbon atoms and 1 to 9 identical or different halogen atoms, cycloalkyl which has 3 to 7 carbon atoms and which is optionally monosubstituted to pentasubstituted by identical or different substituents from the series comprising straight-chain or branched alkyl having 1 to 4 carbon atoms and/or halogen, or heteroaryl which has 2 to 9 carbon atoms and 1 to 4 identical or different hetero atoms - in particular nitrogen, oxygen and/or sulphur - and which is optionally monosubstituted to trisubstituted by identical or different substituents, suitable heteroaryl substituents in each case being:

fluorine, chlorine, bromine and in each case straight-chain or branched alkyl or alkoxy each of which has 1 to 4 carbon atoms;

R furthermore represents aryl which has 6 or 10 carbon atoms and which is

optionally monosubstituted to pentasubstituted by identical or different substituents, suitable aryl substituents in each case being:
fluorine, chlorine, bromine, cyano, nitro, in each case straight-chain or branched alkyl, alkoxy, alkylthio, alkylsulphinyl or alkylsulphonyl each of which has 1 to 4 carbon atoms, in each case straight-chain or branched alkenyl, alkinyl, alkenyloxy, alkinyloxy, alkenylthio or alkinylthio each of which has 2 to 4 carbon atoms, in each case straight-chain or branched halogenoalkyl, halogenoalkoxy, halogenoalkylthio, halogenoalkylsulphinyl and halogenoalkylsulphonyl each of which has 1 to 4 carbon atoms and 1 to 9 identical or different halogen atoms, in each case straight-chain or branched halogenoalkenyl, halogenoalkinyl, halogenoalkenyloxy, halogenoalkinyloxy, halogenoalkenylthio or halogenoalkinylthio each of which has 2 to 4 carbon atoms and 1 to 7 identical or different halogen atoms, in each case straight-chain or branched alkylcarbonyl, alkoxycarbonyl, alkylcarbonyloxy, alkylcarbonylamino, alkylcarbonyl-(N-alkyl)-amino, aminocarbonyl, N-alkyl-aminocarbonyl, N,N-dialkylaminocarbonyl or alkoximinoalkyl each of which has 1 to 4 carbon atoms in the individual alkyl moieties, and aryl, aryloxy, arylthio, arylcarbonyl, arylcarbonyloxy, arylcarbonylamino, arylcarbonyl(N-alkyl)amino, arylaminocarbonyl, N-aryl-N-alkylaminocarbonyl, arylalkyl or arylalkenyl each of which has 6 to 10 carbon atoms in the aryl moiety and if appropriate up to 4 carbon atoms in the individual alkyl or alkenyl moieties and each of which is optionally monosubstituted or polysubstituted by identical or different substituents, suitable substituents in the individual aryl moieties in each case being:
fluorine, chlorine, bromine, cyano, nitro, in each case straight-chain or branched alkyl, alkoxy, alkylthio, alkylsulphinyl or alkylsulphonyl each of which has 1 to 4 carbon atoms, in each case straight-chain or branched alkenyl, alkinyl, alkenyloxy, alkinyloxy, alkenylthio or alkinylthio each of which has 2 to 4 carbon atoms, in each case straight-chain or branched halogenoalkyl, halogenoalkoxy, halogenoalkylthio, halogenoalkylsulphinyl and halogenoalkylsulphonyl each of which has 1 to 4 carbon atoms and 1 to 9 identical or different halogen atoms, in each case straight-chain or branched halogenoalkenyl, halogenoalkinyl, halogenoalkenyloxy, halogenoalkinyloxy, halogenoalkenylthio or halogenoalkinylthio each of which has 2 to 4 carbon atoms and 1 to 7 identical or different halogen atoms, in each case straight-chain or branched alkylcarbonyl, alkoxycarbonyl, alkylcarbonyloxy, alkylcarbonylamino, alkylcarbonyl-(N-alkyl)-amino, aminocarbonyl, N-alkyl-aminocarbonyl, N,N-dialkylaminocarbonyl or alkoximinoalkyl each of which has 1 to 4 carbon atoms in the individual alkyl moieties, and aryl, aryloxy, arylthio, arylcarbonyl, arylcarbonyloxy, arylcarbonylamino, arylcarbonyl-(N-alkyl)amino, arylaminocarbonyl, N-aryl-N-alkylaminocarbonyl, arylalkyl or arylalkenyl each of which has 6 to 10 carbon atoms in the aryl moiety and if appropriate up to 4 carbon atoms in the individual alkyl or alkenyl moieties and each of which is optionally monosubstituted or polysubstituted by identical or different substituents, suitable substituents in the individual aryl moieties in each case being:
fluorine, chlorine, bromine and in each case straight-chain or branched alkyl or alkoxy each of which has 1 to 4 carbon atoms;

R in the event that n is 0, furthermore also represents chlorine or bromine and

$X^1$, $X^2$, $X^3$ and $X^4$ independently of one another in each case represent hydrogen, chlorine, bromine, hydroxyl or methoxy, but where, in the event that $X^2$ represents bromine, $X^3$ does not simultaneously represent hydroxyl or methoxy.

**3.** Pesticides, characterised in that they contain at least one 2-methoximinocarboxylic ester of the general formula (I) according to claim 1.

**4.** Method of combating phytopathogenic fungi, characterised in that 2-methoximinocarboxylic esters of the general formula (I) according to Claim 1 are allowed to act on phytopathogenic fungi and/or their environment.

**5.** Use of 2-methoximinocarboxylic esters of the general formula (I) according to Claim 1, for combating pests, in particular phytopathogenic fungi.

**6.** Process for the preparation of pesticides, characterised in that 2-methoximinocarboxylic esters of the general formula (I) according to Claim 1 are mixed with extenders and/or surface-active agents.

**7.** Process for the preparation of fungicidal agents, characterised in that 2-methoximinocarboxylic esters of the general formula (I) according to Claim 1 are mixed with extenders and/or surface-active agents.

**8.** Process for the preparation of compounds of the formula (V)

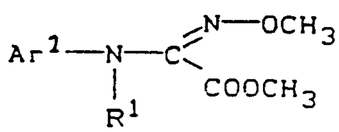

(V)

in which

| | |
|---|---|
| $R^1$ | represents hydrogen or methyl and |
| $Ar^2$ | represents one of the radicals |

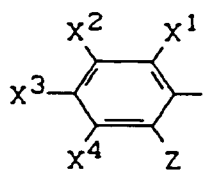

or

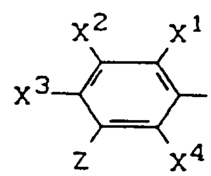

where

| | |
|---|---|
| Z | in each case represents hydroxyl, amino or N-methylamino and |
| $X^1$, $X^2$, $X^3$ and $X^4$ | have the meaning given in Claim 1, |

characterised in that azlactones of the formula (IX)

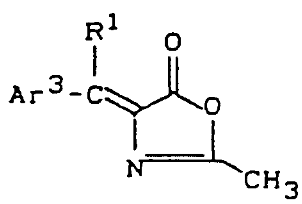

(IX)

in which

| | |
|---|---|
| $R^1$ | has the abovementioned meaning and |
| $Ar^3$ | represents one of the radicals |

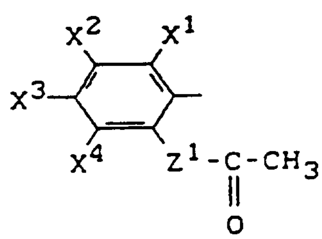

or

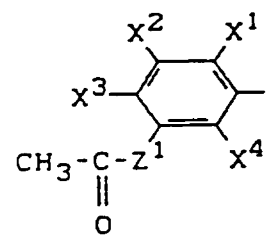

where

| | |
|---|---|
| $Z^1$ | in each case represents oxygen, an NH group or an |

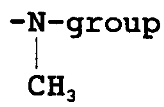

and

$X^1$, $X^2$, $X^3$ and $X^4$ have the abovementioned meaning,

are reacted with methanol in the presence of a base and subsequently in the presence of an acid at temperatures between 0 ° C and 50 ° C and the resulting acrylic ester derivatives of the formula (X)

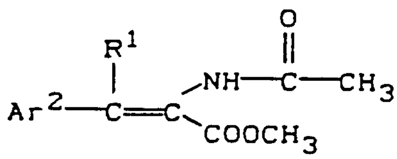

(X)

in which

$R^1$ has the abovementioned meaning and

$Ar^2$ represents one of the radicals

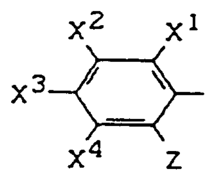

or

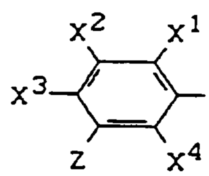

where

Z in each case represents hydroxyl, amino or N-methylamino and

$X^1$, $X^2$, $X^3$ and $X^4$ have the abovementioned meaning,

are subsequently reacted with an acid addition salt of O-methylhydroxylamine analogously to the procedure of process (a) according to the invention at temperatures between 20 ° C and 60 ° C, if appropriate in the presence of a diluent and if appropriate in the presence of a reaction auxiliary.

**Revendications**

**Revendications pour les Etats contractants suivants : BE, CH, DE, DK, FR, GB, GR, IT, LI, NL**

**1.** Esters 2-méthoximinocarboxyliques répondant à la formule générale (I)

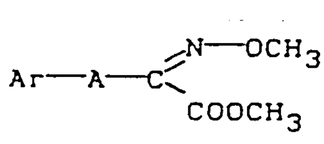

(I)

dans laquelle

Ar représente un des radicaux

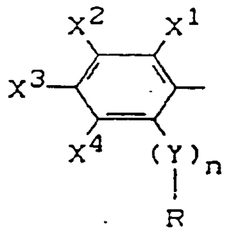 ; 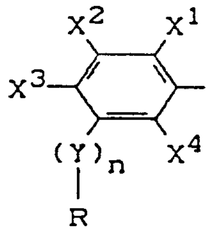 ; 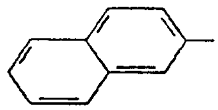 ou

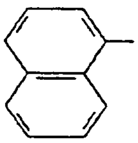

et

A représente un des radicaux $-CH_2-$;

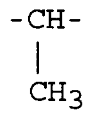 ou 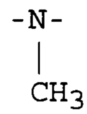

dans lesquels

Y représente un des radicaux

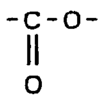 ; 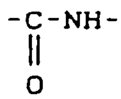 ; 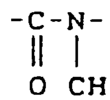 ; 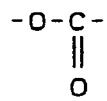 ; 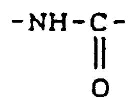 ;

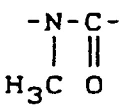 ;

$-O-CH_2-$ ; $-CH_2-O-$ ; $-S-CH_2-$;

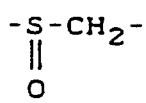 ;

$-SO_2-CH_2-$ ; -O- ; -CH = CH- ou $-CH_2-CH_2-$

n représente le nombre 0 ou 1,

R représente un groupe alkyle à chaîne droite ou ramifiée contenant de 1 à 8 atomes de carbone, un groupe halogénalkyle à chaîne droite ou ramifiée contenant de 1 à 8 atomes de carbone et de 1 à 9 atomes d'halogène identiques ou différents, un groupe alcényle à chaîne droite ou ramifiée contenant de 2 à 8 atomes de carbone, un groupe halogénalcényle à chaîne droite ou ramifiée contenant de 2 à 8 atomes de carbone et de 1 à 9 atomes d'halogène identiques ou différents, un groupe cycloalkyle contenant de 3 à 7 atomes de carbone, éventuellement substitué une ou plusieurs fois de manière identique ou différente par un atome d'halogène et/ou par un groupe alkyle à chaîne droite ou ramifiée contenant de 1 à 6 atomes de carbone, ou encore un groupe hétéroaryle

contenant de 2 à 9 atomes de carbone et de 1 à 5 hétéroatomes identiques ou différents - en particulier un atome d'azote, un atome d'oxygène et/ou un atome de soufre éventuellement substitué une ou plusieurs fois de manière identique ou différente, dans lequel, comme substituants du groupe hétéroaryle, on envisage respectivement : un atome d'halogène, ainsi qu'un groupe alkyle ou un groupe alcoxy contenant respectivement de 1 à 6 atomes de carbone, chacun de ces groupes étant à chaîne droite ou ramifiée;

R représente, en outre, un groupe aryle contenant de 6 à 10 atomes de carbone, éventuellement substitué une ou plusieurs fois de manière identique ou différente, dans lequel, comme substituants du groupe aryle, on envisage : un atome d'halogène, un groupe cyano, un groupe nitro; un groupe alkyle, un groupe alcoxy, un groupe alkylthio, un groupe alkylsulfinyle ou un groupe alkylsulfonyle contenant respectivement de 1 à 6 atomes de carbone, chacun de ces groupes étant à chaîne droite ou ramifiée; un groupe alcényle, un groupe alcynyle, un groupe alcényloxy, un groupe alcynyloxy, un groupe alcénylthio ou un groupe alcynylthio contenant respectivement de 2 à 6 atomes de carbone, chacun de ces groupes étant à chaîne droite ou ramifiée; un groupe halogénalkyle, un groupe halogénalcoxy, un groupe halogénalkylthio, un groupe halogénalkylsulfinyle, un groupe halogénalkylsulfonyle contenant respectivement de 1 à 6 atomes de carbone et de 1 à 13 atomes d'halogène identiques ou différents, chacun de ces groupes étant à chaîne droite ou ramifiée; un groupe halogénalcényle, un groupe halogénalcynyle, un groupe halogénalcényloxy, un groupe halogénalcynyloxy, un groupe halogénalcénylthio ou un groupe halogénalcynylthio contenant respectivement de 2 à 6 atomes de carbone et de 1 à 9 atomes d'halogène identiques ou différents, chacun de ces groupes étant à chaîne droite ou ramifiée; un groupe alkylcarbonyle, un groupe alcoxycarbonyle, un groupe alkylcarbonyloxy, un groupe alkylcarbonylamino, un groupe alkylcarbonyl(N-alkyl)-amino, un groupe aminocarbonyle, un groupe N-alkylaminocarbonyle, un groupe N,N-dialkylaminocarbonyle ou un groupe alcoximinoalkyle contenant respectivement de 1 à 6 atomes de carbone dans les fractions alkyle individuelles, chacun de ces groupes étant à chaîne droite ou ramifiée; ainsi qu'un groupe aryle, un groupe aryloxy, un groupe arylthio, un groupe arylcarbonyle, un groupe arylcarbonyloxy, un groupe arylcarbonylamino, un groupe arylcarbonyl(N-alkyl)amino, un groupe arylaminocarbonyle, un groupe N-aryl-N-alkylaminocarbonyle, un groupe arylalkyle ou un groupe arylalcényle contenant respectivement de 6 à 10 atomes de carbone dans la fraction aryle et éventuellement jusqu'à 6 atomes de carbone dans les fractions alkyle, respectivement alcényle individuelles, chacun de ces groupes étant éventuellement substitué une ou plusieurs fois de manière identique ou différente, dans lesquels, comme substituants dans les fractions aryle individuelles, on mentionnera respectivement :

un atome d'halogène, un groupe cyano, un groupe nitro; un groupe alkyle, un groupe alcoxy, un groupe alkylthio, un groupe alkylsulfinyle ou un groupe alkylsulfonyle contenant respectivement de 1 à 6 atomes de carbone, chacun de ces groupes étant à chaîne droite ou ramifiée; un groupe alcényle, un groupe alcynyle, un groupe alcényloxy, un groupe alcynyloxy, un groupe alcénylthio ou un groupe alcynylthio contenant respectivement de 2 à 6 atomes de carbone, chacun de ces groupes étant à chaîne droite ou ramifiée; un groupe halogénalkyle, un groupe halogénalcoxy, un groupe halogénalkylthio, un groupe halogénalkylsulfinyle, un groupe halogénalkylsulfonyle contenant respectivement de 1 à 6 atomes de carbone et de 1 à 13 atomes d'halogène identiques ou différents, chacun de ces groupes étant à chaîne droite ou ramifiée; un groupe halogénalcényle, un groupe halogénalcynyle, un groupe halogénalcényloxy, un groupe halogénalcynyloxy, un groupe halogénalcénylthio ou un groupe halogénalcynylthio contenant respectivement de 2 à 6 atomes de carbone et de 2 à 9 atomes d'halogène identiques ou différents, chacun de ces groupes étant à chaîne droite ou ramifiée; un groupe alkylcarbonyle, un groupe alcoxycarbonyle, un groupe alkylcarbonyloxy, un groupe alkylcarbonylamino, un groupe alkylcarbonyl(N-alkyl)amino, un groupe aminocarbonyle, un groupe N-alkylaminocarbonyle, un

groupe N,N-dialkylaminocarbonyle ou un groupe alcoximinoalkyle contenant respectivement de 1 à 6 atomes de carbone dans les fractions alkyle individuelles, chacun de ces groupes étant à chaîne droite ou ramifiée; ainsi qu'un groupe aryle, un groupe aryloxy, un groupe arylthio, un groupe arylcarbonyle, un groupe arylcarbonyloxy, un groupe arylcarbonylamino, un groupe arylcarbonyl(N-alkyl)-amino, un groupe arylaminocarbonyle, un groupe N-aryl-N-alkylaminocarbonyle, un groupe arylalkyle ou un groupe arylalcényle contenant respectivement de 6 à 10 atomes de carbone dans la fraction aryle et éventuellement jusqu'à 6 atomes de carbone dans les fractions alkyle, respectivement alcényle individuelles, chacun de ces groupes étant éventuellement substitué une ou plusieurs fois de manière identique ou différente, dans lesquels, comme substituants dans les fractions aryle individuelles, on envisage respectivement :
un atome d'halogène, ainsi qu'un groupe alkyle ou un groupe alcoxy contenant respectivement de 1 à 6 atomes de carbone, chacun de ces groupes étant à chaîne droite ou ramifiée;

R représente, en outre, pour le cas où n = 0, également un atome de fluor, un atome de chlore, un atome de brome ou un atome d'iode, et

$X^1$, $X^2$, $X^3$ et $X^4$ représentent, indépendamment l'un de l'autre, respectivement un atome d'hydrogène, un atome de fluor, un atome de chlore, un atome de brome, un atome d'iode, un groupe hydroxyle ou un groupe méthoxy, dans lesquels, toutefois, pour le cas où $X^2$ représente un atome de brome, $X^3$ ne représente pas simultanément un groupe hydroxyle ou un groupe méthoxy.

**2.** Esters 2-méthoximinocarboxyliques répondant à la formule générale (I) selon la revendication 1, dans laquelle

Ar représente un radical

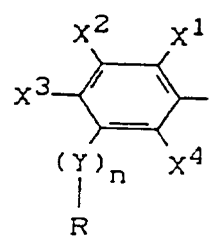

et

A représente un des radicaux $-CH_2-$;

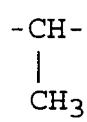 ou 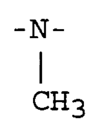

dans lesquels

Y représente un des radicaux

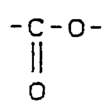 ; 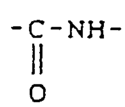 ; 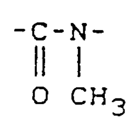 ; 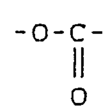 ; 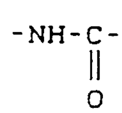 ;

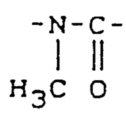 ;

-O-$CH_2$ ; -$CH_2$-O- ; -S-$CH_2$-;

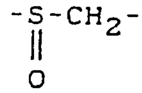 ;

-$SO_2$-$CH_2$- ; -O- ; -CH = CH-ou -$CH_2$-$CH_2$-

n représente le nombre 0 ou 1,

R représente un groupe alkyle à chaîne droite ou ramifiée contenant de 1 à 6 atomes de carbone, un groupe halogénalkyle à chaîne droite ou ramifiée contenant de 1 à 6 atomes de carbone et de 1 à 9 atomes d'halogène identiques ou différents, un groupe alcényle à chaîne droite ou ramifiée contenant de 2 à 6 atomes de carbone, un groupe halogénalcényle à chaîne droite ou ramifiée contenant de 2 à 6 atomes de carbone et de 1 à 9 atomes d'halogène identiques ou différents, un groupe cycloalkyle contenant de 3 à 7 atomes de carbone, éventuellement substitué de 1 à 5 fois de manière identique ou différente par un groupe alkyle à chaîne droite ou ramifiée contenant de 1 à 4 atomes de carbone et/ou par un atome d'halogène, ou représente un groupe hétéroaryle contenant de 2 à 9 atomes de carbone et de 1 à 4 hétéroatomes identiques ou différents - en particulier un atome d'azote, un atome d'oxygène et/ou un atome de soufre - éventuellement substitué de 1 à 3 fois de manière identique ou différente, dans lequel, comme substituants du groupe hétéroaryle, on envisage respectivement : un atome de fluor, un atome de chlore, un atome de brome, ainsi qu'un groupe alkyle ou un groupe alcoxy contenant respectivement de 1 à 4 atomes de carbone, chacun de ces groupes étant à chaîne droite ou ramifiée;

R représente, en outre, un groupe aryle contenant 6 ou 10 atomes de carbone, éventuellement substitué de 1 à 5 fois de manière identique ou différente, dans lequel, comme substituants du groupe aryle, on envisage respectivement : un atome de fluor, un atome de chlore, un atome de brome, un groupe cyano, un groupe nitro; un groupe alkyle, un groupe alcoxy, un groupe alkylthio, un groupe alkylsulfinyle ou un groupe alkylsulfonyle contenant respectivement de 1 à 4 atomes de carbone, chacun de ces groupes étant à chaîne droite ou ramifiée; un groupe alcényle, un groupe alcynyle, un groupe alcényloxy, un groupe alcynyloxy, un groupe alcénylthio ou un groupe alcynylthio contenant respectivement de 2 à 4 atomes de carbone, chacun de ces groupes étant à chaîne droite ou ramifiée; un groupe halogénalkyle, un groupe halogénalcoxy, un groupe halogénalkylthio, un groupe halogénalkylsulfinyle, un groupe halogénalkylsulfonyle contenant respectivement de 1 à 4 atomes de carbone et de 1 à 9 atomes d'halogène identiques ou différents, chacun de ces groupes étant à chaîne droite ou ramifiée; un groupe halogénalcényle, un groupe halogénalcynyle, un groupe halogénalcényloxy, un groupe halogénalcynyloxy, un groupe halogénalcénylthio ou un groupe halogénalcynylthio contenant respectivement de 2 à 4 atomes de carbone et de 1 à 7 atomes d'halogène identiques ou différents, chacun de ces groupes étant à chaîne droite ou ramifiée; un groupe alkylcarbonyle, un groupe alcoxycarbonyle, un groupe alkylcarbonyloxy, un groupe alkylcarbonylamino, un groupe alkylcarbonyl(N-alkyl)amino, un groupe aminocarbonyle, un groupe N-alkylaminocarbonyle, un groupe N,N-dialkylaminocarbonyle ou un groupe alcoximinoalkyle contenant respectivement de 1 à 4 atomes de carbone dans les fractions alkyle individuelles, chacun de ces groupes étant à chaîne droite ou ramifiée; ainsi qu'un groupe aryle, un groupe aryloxy, un groupe arylthio, un groupe arylcarbonyle, un groupe arylcarbonyloxy, un groupe arylcarbonylamino, un groupe arylcarbonyl(N-alkyl)amino, un groupe arylaminocarbonyle, un groupe N-aryl-N-alkylaminocarbonyle, un groupe arylalkyle ou un groupe arylalcényle contenant respectivement de 6 à 10 atomes de carbone dans la fraction aryle et éventuellement jusqu'à 4 atomes de carbone dans les fractions alkyle, respectivement alcényle individuelles, chacun de ces groupes étant éventuellement

substitué une ou plusieurs fois de manière identique ou différente, dans lesquels, comme substituants dans les fractions aryle individuelles, on envisage respectivement :

un atome de fluor, un atome de chlore, un atome de brome, un groupe cyano, un groupe nitro; un groupe alkyle, un groupe alcoxy, un groupe alkylthio, un groupe alkylsulfinyle ou un groupe alkylsulfonyle contenant respectivement de 1 à 4 atomes de carbone, chacun de ces groupes étant à chaîne droite ou ramifiée; un groupe alcényle, un groupe alcynyle, un groupe alcényloxy, un groupe alcynyloxy, un groupe alcénylthio ou un groupe alcynylthio contenant respectivement de 2 à 4 atomes de carbone, chacun de ces groupes étant à chaîne droite ou ramifiée; un groupe halogénalkyle, un groupe halogénalcoxy, un groupe halogénalkylthio, un groupe halogénalkylsulfinyle, un groupe halogénalkylsulfonyle contenant respectivement de 1 à 4 atomes de carbone et de 1 à 9 atomes d'halogène identiques ou différents, chacun de ces groupes étant à chaîne droite ou ramifiée; un groupe halogénalcényle, un groupe halogénalcynyle, un groupe halogénalcényloxy, un groupe halogénalcynyloxy, un groupe halogénalcénylthio ou un groupe halogénalcynylthio contenant respectivement de 2 à 4 atomes de carbone et de 1 à 7 atomes d'halogène identiques ou différents, chacun de ces groupes étant à chaîne droite ou ramifiée; un groupe alkylcarbonyle, un groupe alcoxycarbonyle, un groupe alkylcarbonyloxy, un groupe alkylcarbonylamino, un groupe alkylcarbonyl(N-alkyl)amino, un groupe aminocarbonyle, un groupe N-alkylaminocarbonyle, un groupe N,N-dialkylamino-carbonyle ou un groupe alcoximinoalkyle contenant respectivement de 1 à 4 atomes de carbone dans les fractions alkyle individuelles, chacun de ces groupes étant à chaîne droite ou ramifiée; ainsi qu'un groupe aryle, un groupe aryloxy, un groupe arylthio, un groupe arylcarbonyle, un groupe arylcarbonyloxy, un groupe arylcarbonylamino, un groupe arylcarbonyl(N-alkyl)amino, un groupe arylaminocarbonyle, un groupe N-aryl-N-alkylaminocarbonyle, un groupe arylalkyle ou un groupe arylalcényle contenant respectivement de 6 à 10 atomes de carbone dans la fraction aryle et éventuellement jusqu'à 4 atomes de carbone dans les fractions alkyle, respectivement alcényle individuelles, chacun de ces groupes étant éventuellement substitué une ou plusieurs fois de manière identique ou différente, dans lesquels, comme substituants dans les fractions aryle individuelles, on envisage respectivement :

un atome de fluor, un atome de chlore, un atome de brome, ainsi qu'un groupe alkyle ou un groupe alcoxy contenant respectivement de 1 à 4 atomes de carbone, chacun de ces groupes étant à chaîne droite ou ramifiée;

R représente, en outre, pour le cas où n = 0, également un atome de chlore ou un atome de brome, et

$X^1$, $X^2$, $X^3$ et $X^4$ représentent, indépendamment l'un de l'autre, respectivement un atome d'hydrogène, un atome de chlore, un atome de brome, un groupe hydroxyle ou un groupe méthoxy, dans lesquels, toutefois, pour le cas où $X^2$ représente un atome de brome, $X^3$ ne représente pas simultanément un groupe hydroxyle ou un groupe méthoxy.

**3.** Procédé pour la préparation d'esters 2-méthoximinocarboxyliques répondant à la formule générale (I)

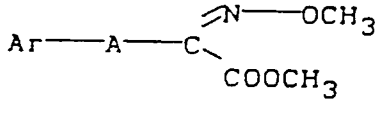

(I)

dans laquelle

Ar et A ont la signification indiquée à la revendication 1,

caractérisé en ce que

(a) pour obtenir des esters 2-méthoximinocarboxyliques répondant à la formule (Ia)

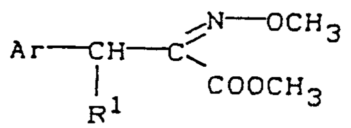

(Ia)

dans laquelle

$R^1$ représente un atome d'hydrogène ou un groupe méthyle et

Ar a la signification indiquée ci-dessus,

on fait réagir des dérivés d'ester acrylique de formule (II)

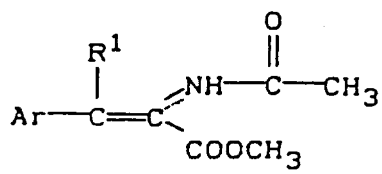

(II)

dans laquelle

$R^1$ et Ar ont la signification indiquée ci-dessus,

avec un sel d'addition d'acide de la O-méthylhydroxylamine, éventuellement en présence d'un diluant, ainsi qu'éventuellement en présence d'un adjuvant réactionnel;

ou bien en ce que

(b) pour obtenir des esters 2-méthoximinocarboxyliques de formule (Ib)

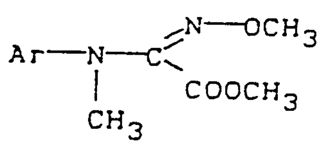

(Ib)

dans laquelle

Ar a la signification indiquée ci-dessus,

on fait réagir, d'abord dans une première étape, des amines aromatiques de formule (III)

$Ar\text{-}NH\text{-}CH_3$ (III)

dans laquelle

Ar a la signification indiquée ci-dessus,

avec l'ester méthylique de l'acide 2-chloro-2-hydroximinoacétique répondant à la formule (IV)

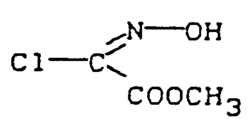

(IV)

éventuellement en présence d'un diluant et éventuellement en présence d'un agent neutralisateur d'acides et ensuite, dans une deuxième étape, on procède à une méthylation du groupe hydroximino avec un agent de méthylation, éventuellement en présence d'un agent neutralisateur d'acides;

ou bien en ce que

(c) pour obtenir des esters 2-méthoximinocarboxyliques de formule (Ic)

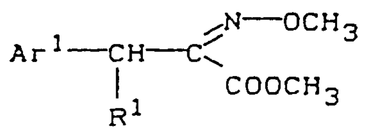

(Ic)

dans laquelle

R¹ représente un atome d'hydrogène ou un groupe méthyle et

$Ar^1$ représente un des radicaux

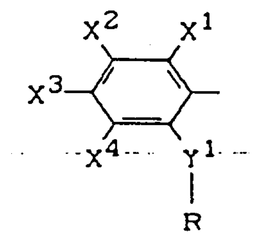

ou

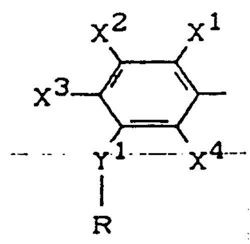

dans lesquels

$Y^1$ représente respectivement un des radicaux

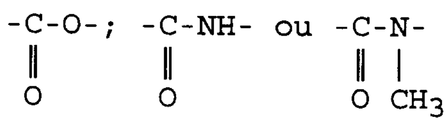

$X^1$, $X^2$, $X^3$, $X^4$ et R ont la signification indiquée ci-dessus,

on acyle des dérivés substitués de phénol ou d'aniline de formule (V)

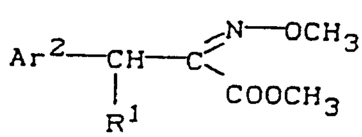

(V)

dans laquelle

$R^1$ représente un atome d'hydrogène ou un groupe méthyle et

$Ar^2$ représente un des radicaux

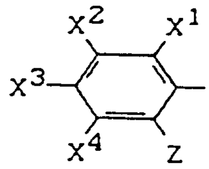

ou

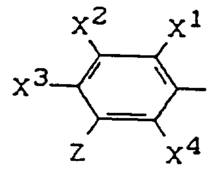

dans lesquels

Z représente respectivement un groupe hydroxyle, un groupe amino ou un groupe N-méthylamino, et

$X^1$, $X^2$, $X^3$ et $X^4$ ont la signification indiquée ci-dessus,

avec des chlorures d'acide de formule (VI)

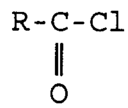

(VI)

dans laquelle

R a la signification indiquée ci-dessus,

éventuellement en présence d'un diluant et éventuellement en présence d'un agent neutralisateur d'acides.

**4.** Agents de lutte contre les parasites, caractérisés par une teneur en au moins un ester 2-méthoximinocarboxylique répondant à la formule générale (I) selon la revendication 1.

**5.** Procédé pour lutter contre des champignons phytopathogènes, caractérisé en ce qu'on laisse agir des esters 2-méthoximinocarboxyliques de formule générale (I) selon la revendication 1, sur des champignons phytopathogènes et/ou sur leur biotope.

**6.** Utilisation d'esters 2-méthoximinocarboxyliques répondant à la formule générale (I) selon la revendication 1, pour lutter contre des parasites, en particulier des champignons phytopathogènes.

**7.** Procédé pour la préparation d'agents de lutte contre les parasites, caractérisé en ce qu'on mélange des esters 2-méthoximinocarboxyliques répondant à la formule générale (I) selon la revendication 1, avec des diluants et/ou des agents tensioactifs.

**8.** Procédé pour la préparation d'agents fongicides, caractérisé en ce qu'on mélange des esters 2-méthoximinocarboxyliques répondant à la formule générale (I) selon la revendication 1, avec des diluants et/ou des agents tensioactifs.

**9.** Composés de formule (V)

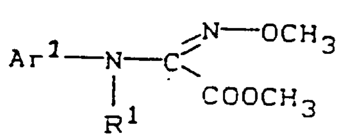

(V)

dans laquelle

$R^1$ représente un atome d'hydrogène ou un groupe méthyle et

$Ar^2$ représente un des radicaux

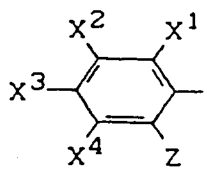

ou

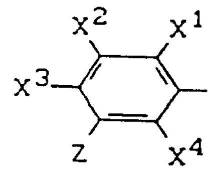

dans lesquels

Z représente respectivement un groupe hydroxyle, un groupe amino ou un groupe N-méthylamino, et

$X^1$, $X^2$, $X^3$ et $X^4$ ont la signification indiquée ci-dessus.

**Revendications pour l'Etat contractant suivant : ES**

**1.** Procédé pour la préparation d'esters 2-méthoximinocarboxyliques répondant à la formule générale (I)

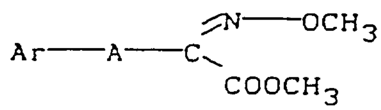

(I)

dans laquelle

Ar représente un des radicaux

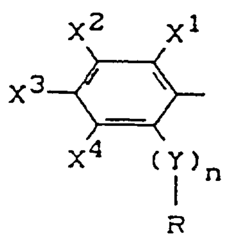

;

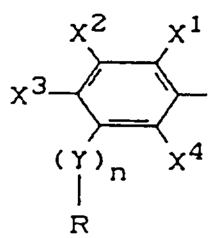

;

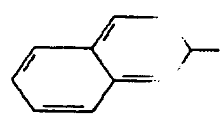

ou

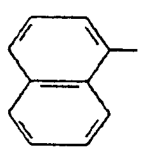

et

A représente un des radicaux $-CH_2-$;

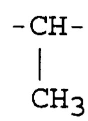

ou

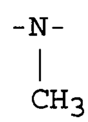

dans lesquels

Y représente un des radicaux

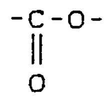 ; 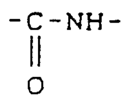 ; 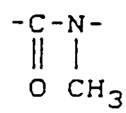 ; 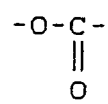 ; 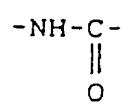 ;

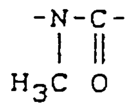 ;

$-O-CH_2$ ; $-CH_2-O-$ ; $-S-CH_2-$;

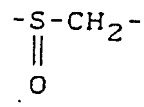

$-SO_2-CH_2-$ ; -O- ; -CH = CH- ou $-CH_2-CH_2-$

n représente le nombre 0 ou 1,

R représente un groupe alkyle à chaîne droite ou ramifiée contenant de 1 à 8 atomes de carbone, un groupe halogénalkyle à chaîne droite ou ramifiée contenant de 1 à 8 atomes de carbone et de 1 à 9 atomes d'halogène identiques ou différents, un groupe alcényle à chaîne droite ou ramifiée contenant de 2 à 8 atomes de carbone, un groupe halogénalcényle à chaîne droite ou ramifiée contenant de 2 à 8 atomes de carbone et de 1 à 9 atomes d'halogène identiques ou différents, un groupe cycloalkyle contenant de 3 à 7 atomes de carbone, éventuellement substitué une ou plusieurs fois de manière identique ou différente par un atome d'halogène et/ou par un groupe alkyle à chaîne droite ou ramifiée contenant de 1 à 6 atomes de carbone, ou encore un groupe hétéroaryle contenant de 2 à 9 atomes de carbone et de 1 à 5 hétéroatomes identiques ou différents - en particulier un atome d'azote, un atome d'oxygène et/ou un atome de soufre - éventuellement substitué une ou plusieurs fois de manière identique ou différente, dans lequel, comme substituants du groupe hétéroaryle, on envisage respectivement : un atome d'halogène, ainsi qu'un groupe alkyle ou un groupe alcoxy contenant respectivement de 1 à 6 atomes de carbone, chacun de ces groupes étant à chaîne droite ou ramifiée;

R représente, en outre, un groupe aryle contenant de 6 à 10 atomes de carbone, éventuellement substitué une ou plusieurs fois de manière identique ou différente, dans lequel, comme substituants du groupe aryle, on envisage : un atome d'halogène, un groupe cyano, un groupe nitro; un groupe alkyle, un groupe alcoxy, un groupe alkylthio, un groupe alkylsulfinyle ou un groupe alkylsulfonyle contenant respectivement de 1 à 6 atomes de carbone, chacun de ces groupes étant à chaîne droite ou ramifiée; un groupe alcényle, un groupe alcynyle, un groupe alcényloxy, un groupe alcynyloxy, un groupe alcénylthio ou un groupe alcynylthio contenant respectivement de 2 à 6 atomes de carbone, chacun de ces groupes étant à chaîne droite ou ramifiée; un groupe halogénalkyle, un groupe halogénalcoxy, un groupe halogénalkylthio, un groupe halogénalkylsulfinyle, un groupe halogénalkylsulfonyle contenant respectivement de 1 à 6 atomes de carbone et de 1 à 13 atomes d'halogène identiques ou différents, chacun de ces groupes étant à chaîne droite ou ramifiée; un groupe halogénalcényle, un groupe halogénalcynyle, un groupe halogénalcényloxy, un groupe halogénalcynyloxy, un groupe halogénalcénylthio ou un groupe halogénalcynylthio contenant respectivement de 2 à 6 atomes de carbone et de 1 à 9 atomes d'halogène identiques ou différents, chacun de ces groupes étant à chaîne droite ou ramifiée; un groupe alkylcarbonyle, un groupe alcoxycarbonyle, un groupe alkylcarbonyloxy, un groupe alkylcarbonylamino, un groupe alkylcarbonyl(N-alkyl)-amino, un groupe aminocarbonyle, un groupe N-alkylaminocarbonyle, un groupe N,N-dialkylaminocarbonyle ou un groupe alcoximinoalkyle contenant respectivement de 1 à 6 atomes de carbone dans les fractions alkyle individuelles, chacun de ces groupes étant à chaîne droite ou ramifiée; ainsi qu'un groupe aryle, un groupe aryloxy, un groupe arylthio, un groupe arylcarbonyle, un groupe arylcarbonyloxy, un groupe arylcarbonylamino, un groupe arylcarbonyl(N-alkyl)amino, un groupe arylaminocarbonyle, un groupe N-aryl-N-alkylaminocarbonyle, un groupe arylalkyle ou un groupe arylalcényle contenant respectivement de 6 à 10 atomes de carbone dans la fraction aryle et éventuellement jusqu'à 6 atomes de carbone dans les fractions alkyle, respectivement alcényle individuelles, chacun de ces groupes étant éventuellement substitué une ou plusieurs fois de manière identique ou différente, dans lesquels, comme substituants dans les fractions aryle individuelles, on mentionnera respectivement :

un atome d'halogène, un groupe cyano, un groupe nitro; un groupe alkyle, un groupe alcoxy, un groupe alkylthio, un groupe alkylsulfinyle ou un groupe alkylsulfonyle contenant respectivement de 1 à 6 atomes de carbone, chacun de ces groupes étant à chaîne droite ou ramifiée; un groupe alcényle, un groupe alcynyle, un groupe alcényloxy, un groupe alcynyloxy, un groupe alcénylthio ou un groupe alcynylthio contenant respectivement de 2 à 6 atomes de carbone, chacun de ces groupes étant à chaîne droite ou ramifiée; un groupe halogénalkyle, un groupe halogénalcoxy, un groupe halogénalkylthio, un groupe halogénalkylsulfinyle, un groupe halogénalkylsulfonyle contenant respectivement de 1 à 6 atomes de carbone et de 1 à 13 atomes d'halogène identiques ou différents, chacun de ces groupes étant à chaîne droite ou ramifiée; un groupe halogénalcényle, un groupe halogénalcynyle, un groupe halogénalcényloxy, un groupe halogénalcynyloxy, un groupe halogénalcénylthio ou un groupe halogénalcynylthio contenant respectivement de 2 à 6 atomes de carbone et de 2 à 9 atomes d'halogène identiques ou différents, chacun de ces groupes étant à chaîne droite ou ramifiée; un groupe alkylcarbonyle, un groupe alcoxycarbonyle, un groupe alkylcarbonyloxy, un groupe alkylcarbonylamino, un groupe alkylcarbonyl(N-alkyl)amino, un groupe aminocarbonyle, un groupe N-alkylaminocarbonyle, un groupe N,N-dialkylaminocarbonyle ou un groupe alcoximinoalkyle contenant respectivement de 1 à 6 atomes de carbone dans les fractions alkyle individuelles, chacun de ces groupes étant à chaîne droite ou ramifiée; ainsi qu'un groupe aryle, un groupe aryloxy, un groupe arylthio, un groupe arylcarbonyle, un groupe arylcarbonyloxy, un groupe arylcarbonylamino, un groupe arylcarbonyl(N-alkyl)-amino, un groupe arylaminocarbonyle, un groupe N-aryl-N-alkylaminocarbonyle, un groupe arylalkyle ou un groupe arylalcényle contenant respectivement de 6 à 10 atomes de carbone dans la fraction aryle et éventuellement jusqu'à 6 atomes de carbone dans les fractions alkyle, respectivement alcényle individuelles, chacun de ces groupes étant éventuellement substitué une ou plusieurs fois de manière identique ou différente, dans lesquels, comme substituants dans les fractions aryle individuelles, on envisage respectivement :

un atome d'halogène, ainsi qu'un groupe alkyle ou un groupe alcoxy contenant respectivement de 1 à 6 atomes de carbone, chacun de ces groupes étant à chaîne droite ou ramifiée;

R représente, en outre, pour le cas où n = 0, également un atome de fluor, un atome de chlore, un atome de brome ou un atome d'iode, et

$X^1$, $X^2$, $X^3$ et $X^4$ représentent, indépendamment l'un de l'autre, respectivement un atome d'hydrogène, un atome de fluor, un atome de chlore, un atome de brome, un atome d'iode, un groupe hydroxyle ou un groupe méthoxy, dans lesquels, toutefois, pour le cas où $X^2$ représente un atome de brome, $X^3$ ne représente pas simultanément un groupe hydroxyle ou un groupe méthoxy,

caractérisé en ce que

(a) pour obtenir des esters 2-méthoximinocarboxyliques répondant à la formule (Ia)

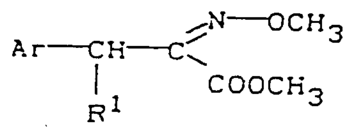

(Ia)

dans laquelle

R[1] représente un atome d'hydrogène ou un groupe méthyle et

Ar a la signification indiquée ci-dessus,

on fait réagir des dérivés d'ester acrylique de formule (II)

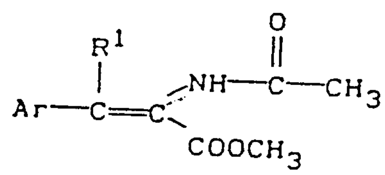

(II)

dans laquelle

R[1] et Ar ont la signification indiquée ci-dessus,

avec un sel d'addition d'acide de la O-méthylhydroxylamine, éventuellement en présence d'un diluant, ainsi qu'éventuellement en présence d'un adjuvant réactionnel;

ou bien en ce que

(b) pour obtenir des esters 2-méthoximinocarboxyliques de formule (Ib)

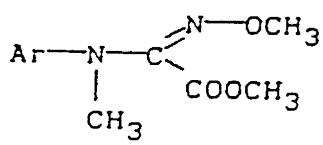

(Ib)

dans laquelle

Ar a la signification indiquée ci-dessus,

on fait réagir, d'abord dans une première étape, des amines aromatiques de formule (III)

$Ar\text{-}NH\text{-}CH_3$ (III)

dans laquelle

Ar a la signification indiquée ci-dessus,

avec l'ester méthylique de l'acide 2-chloro-2-hydroximinoacétique répondant à la formule (IV)

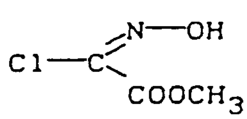

(IV)

éventuellement en présence d'un diluant et éventuellement en présence d'un agent neutralisateur d'acides et ensuite, dans une deuxième étape, on procède à une méthylation du groupe hydroximino avec un agent de méthylation, éventuellement en présence d'un agent neutralisateur d'acides;

ou bien en ce que

(c) pour obtenir des esters 2-méthoximinocarboxyliques de formule (Ic)

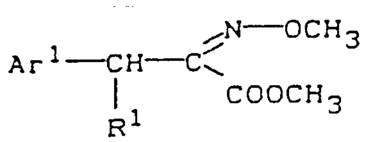

(Ic)

dans laquelle

$R^1$ représente un atome d'hydrogène ou un groupe méthyle et

$Ar^1$ représente un des radicaux

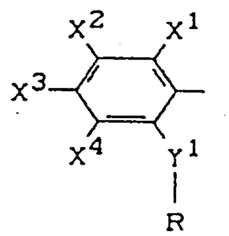

ou

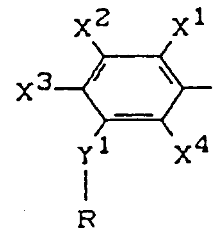

dans lesquels

$Y^1$ représente respectivement un des radicaux

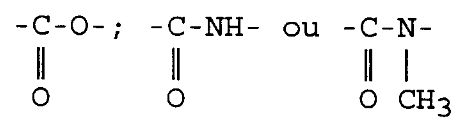

$X^1$, $X^2$, $X^3$, $X^4$ et R ont la signification indiquée ci-dessus,

on acyle des dérivés substitués de phénol ou d'aniline de formule (V)

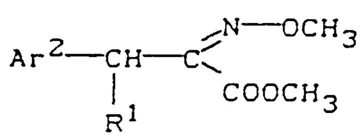

(V)

dans laquelle

$R^1$ représente un atome d'hydrogène ou un groupe méthyle et

$Ar^2$ représente un des radicaux

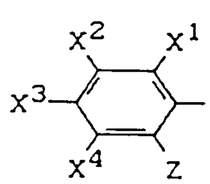

ou

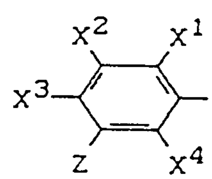

dans lesquels

Z représente respectivement un groupe hydroxyle, un groupe amino ou un groupe N-méthylamino, et

$X^1$, $X^2$, $X^3$ et $X^4$ ont la signification indiquée ci-dessus,

avec des chlorures d'acide de formule (VI)

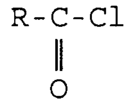 (VI)

dans laquelle

R a la signification indiquée ci-dessus,

éventuellement en présence d'un diluant et éventuellement en présence d'un agent neutralisateur d'acides.

**2.** Procédé selon la revendication 1, dans lequel

Ar représente un radical

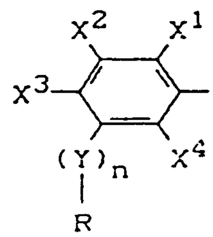

et

A représente un des radicaux $-CH_2-$;

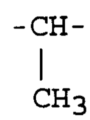 ou 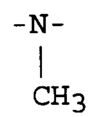

dans lesquels

Y représente un des radicaux

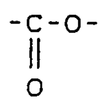 ; 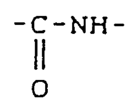 ; 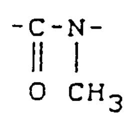 ; 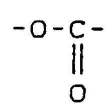 ; 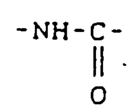 ;

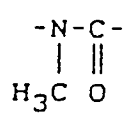 ;

$-O-CH_2$ ; $-CH_2-O-$ ; $-S-CH_2-$;

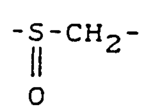

$-SO_2-CH_2-$ ; $-O-$ ; $-CH=CH-$ ou $-CH_2-CH_2-$

n représente le nombre 0 ou 1,

R représente un groupe alkyle à chaîne droite ou ramifiée contenant de 1 à 6 atomes de carbone, un groupe halogénalkyle à chaîne droite ou ramifiée contenant de 1 à 6 atomes de carbone et de 1 à 9 atomes d'halogène identiques ou

différents, un groupe alcényle à chaîne droite ou ramifiée contenant de 2 à 6 atomes de carbone, un groupe halogénalcényle à chaîne droite ou ramifiée contenant de 2 à 6 atomes de carbone et de 1 à 9 atomes d'halogène identiques ou différents, un groupe cycloalkyle contenant de 3 à 7 atomes de carbone, éventuellement substitué de 1 à 5 fois de manière identique ou différente par un groupe alkyle à chaîne droite ou ramifiée contenant de 1 à 4 atomes de carbone et/ou par un atome d'halogène, ou représente un groupe hétéroaryle contenant de 2 à 9 atomes de carbone et de 1 à 4 hétéroatomes identiques ou différents - en particulier un atome d'azote, un atome d'oxygène et/ou un atome de soufre - éventuellement substitué de 1 à 3 fois de manière identique ou différente, dans lequel, comme substituants du groupe hétéroaryle, on envisage respectivement : un atome de fluor, un atome de chlore, un atome de brome, ainsi qu'un groupe alkyle ou un groupe alcoxy contenant respectivement de 1 à 4 atomes de carbone, chacun de ces groupes étant à chaîne droite ou ramifiée;

R représente, en outre, un groupe aryle contenant 6 ou 10 atomes de carbone, éventuellement substitué de 1 à 5 fois de manière identique ou différente, dans lequel, comme substituants du groupe aryle, on envisage respectivement : un atome de fluor, un atome de chlore, un atome de brome, un groupe cyano, un groupe nitro; un groupe alkyle, un groupe alcoxy, un groupe alkylthio, un groupe alkylsulfinyle ou un groupe alkylsulfonyle contenant respectivement de 1 à 4 atomes de carbone, chacun de ces groupes étant à chaîne droite ou ramifiée; un groupe alcényle, un groupe alcynyle, un groupe alcényloxy, un groupe alcynyloxy, un groupe alcénylthio ou un groupe alcynylthio contenant respectivement de 2 à 4 atomes de carbone, chacun de ces groupes étant à chaîne droite ou ramifiée; un groupe halogénalkyle, un groupe halogénalcoxy, un groupe halogénalkylthio, un groupe halogénalkylsulfinyle, un groupe halogénalkylsulfonyle contenant respectivement de 1 à 4 atomes de carbone et de 1 à 9 atomes d'halogène identiques ou différents, chacun de ces groupes étant à chaîne droite ou ramifiée; un groupe halogénalcényle, un groupe halogénalcynyle, un groupe halogénalcényloxy, un groupe halogénalcynyloxy, un groupe halogénalcénylthio ou un groupe halogénalcynylthio contenant respectivement de 2 à 4 atomes de carbone et de 1 à 7 atomes d'halogène identiques ou différents, chacun de ces groupes étant à chaîne droite ou ramifiée; un groupe alkylcarbonyle, un groupe alcoxycarbonyle, un groupe alkylcarbonyloxy, un groupe alkylcarbonylamino, un groupe alkylcarbonyl(N-alkyl)amino, un groupe aminocarbonyle, un groupe N-alkylaminocarbonyle, un groupe N,N-dialkylaminocarbonyle ou un groupe alcoximinoalkyle contenant respectivement de 1 à 4 atomes de carbone dans les fractions alkyle individuelles, chacun de ces groupes étant à chaîne droite ou ramifiée; ainsi qu'un groupe aryle, un groupe aryloxy, un groupe arylthio, un groupe arylcarbonyle, un groupe arylcarbonyloxy, un groupe arylcarbonylamino, un groupe arylcarbonyl(N-alkyl)amino, un groupe arylaminocarbonyle, un groupe N-aryl-N-alkylaminocarbonyle, un groupe arylalkyle ou un groupe arylalcényle contenant respectivement de 6 à 10 atomes de carbone dans la fraction aryle et éventuellement jusqu'à 4 atomes de carbone dans les fractions alkyle, respectivement alcényle individuelles, chacun de ces groupes étant éventuellement substitué une ou plusieurs fois de manière identique ou différente, dans lesquels, comme substituants dans les fractions aryle individuelles, on envisage respectivement :

un atome de fluor, un atome de chlore, un atome de brome, un groupe cyano, un groupe nitro; un groupe alkyle, un groupe alcoxy, un groupe alkylthio, un groupe alkylsulfinyle ou un groupe alkylsulfonyle contenant respectivement de 1 à 4 atomes de carbone, chacun de ces groupes étant à chaîne droite ou ramifiée; un groupe alcényle, un groupe alcynyle, un groupe alcényloxy, un groupe alcynyloxy, un groupe alcénylthio ou un groupe alcynylthio contenant respectivement de 2 à 4 atomes de carbone, chacun de ces groupes étant à chaîne droite ou ramifiée; un groupe halogénalkyle, un groupe halogénalcoxy, un groupe halogénalkylthio, un groupe halogénalkylsulfinyle, un groupe halogénalkylsulfonyle contenant respectivement de 1 à 4 atomes de carbone et de 1 à 9 atomes

d'halogène identiques ou différents, chacun de ces groupes étant à chaîne droite ou ramifiée; un groupe halogénalcényle, un groupe halogénalcynyle, un groupe halogénalcényloxy, un groupe halogénalcynyloxy, un groupe halogénalcénylthio ou un groupe halogénalcynylthio contenant respectivement de 2 à 4 atomes de carbone et de 1 à 7 atomes d'halogène identiques ou différents, chacun de ces groupes étant à chaîne droite ou ramifiée; un groupe alkylcarbonyle, un groupe alcoxycarbonyle, un groupe alkylcarbonyloxy, un groupe alkylcarbonylamino, un groupe alkylcarbonyl(N-alkyl)amino, un groupe aminocarbonyle, un groupe N-alkylaminocarbonyle, un groupe N,N-dialkylaminocarbonyle ou un groupe alcoximinoalkyle contenant respectivement de 1 à 4 atomes de carbone dans les fractions alkyle individuelles, chacun de ces groupes étant à chaîne droite ou ramifiée; ainsi qu'un groupe aryle, un groupe aryloxy, un groupe arylthio, un groupe arylcarbonyle, un groupe arylcarbonyloxy, un groupe arylcarbonylamino, un groupe arylcarbonyl(N-alkyl)amino, un groupe arylaminocarbonyle, un groupe N-aryl-N-alkylaminocarbonyle, un groupe arylalkyle ou un groupe arylalcényle contenant respectivement de 6 à 10 atomes de carbone dans la fraction aryle et éventuellement jusqu'à 4 atomes de carbone dans les fractions alkyle, respectivement alcényle individuelles, chacun de ces groupes étant éventuellement substitué une ou plusieurs fois de manière identique ou différente, dans lesquels, comme substituants dans les fractions aryle individuelles, on envisage respectivement :

un atome de fluor, un atome de chlore, un atome de brome, ainsi qu'un groupe alkyle ou un groupe alcoxy contenant chacun de 1 à 4 atomes de carbone, chacun de ces groupes étant à chaîne droite ou ramifiée;

R représente, en outre, pour le cas où n = 0, également un atome de chlore ou un atome de brome, et

$X^1$, $X^2$, $X^3$ et $X^4$ représentent, indépendamment l'un de l'autre, respectivement un atome d'hydrogène, un atome de chlore, un atome de brome, un groupe hydroxyle ou un groupe méthoxy, dans lesquels, toutefois, pour le cas où $X^2$ représente un atome de brome, $X^3$ ne représente pas simultanément un groupe hydroxyle ou un groupe méthoxy.

**3.** Agents de lutte contre les parasites, caractérisés par une teneur en au moins un ester 2-méthoximinocarboxylique répondant à la formule générale (I) selon la revendication 1.

**4.** Procédé pour lutter contre des champignons phytopathogènes, caractérisé en ce qu'on laisse agir des esters 2-méthoximinocarboxyliques de formule générale (I) selon la revendication 1, sur des champignons phytopathogènes et/ou sur leur biotope.

**5.** Utilisation d'esters 2-méthoximinocarboxyliques répondant à la formule générale (I) selon la revendication 1, pour lutter contre des parasites, en particulier des champignons phytopathogènes.

**6.** Procédé pour la préparation d'agents de lutte contre les parasites, caractérisé en ce qu'on mélange des esters 2-méthoximinocarboxyliques répondant à la formule générale (I) selon la revendication 1, avec des diluants et/ou des agents tensioactifs.

**7.** Procédé pour la préparation d'agents fongicides, caractérisé en ce qu'on mélange des esters 2-méthoximinocarboxyliques répondant à la formule générale (I) selon la revendication 1, avec des diluants et/ou des agents tensioactifs.

**8.** Procédé pour la préparation de composés de formule (V)

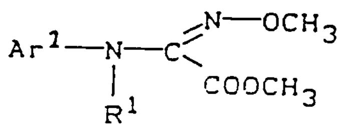

(V)

dans laquelle

$R^1$ représente un atome d'hydrogène ou un groupe méthyle et

$Ar^2$ représente un des radicaux

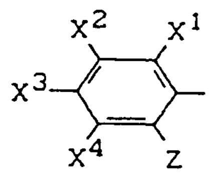

ou

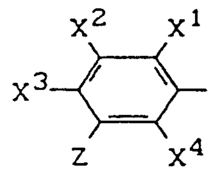

dans lesquels

Z représente respectivement un groupe hydroxyle, un groupe amino ou un groupe N-méthylamino, et

$X^1$, $X^2$, $X^3$ et $X^4$ ont la signification indiquée à la revendication 1,

caractérisé en ce qu'on fait réagir des azlactones de formule (IX)

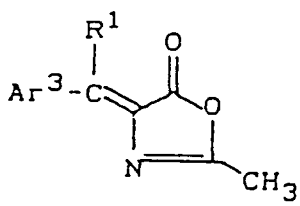

(IX)

dans laquelle

$R^1$ a la signification indiquée ci-dessus et

$Ar_3$ représente un des radicaux

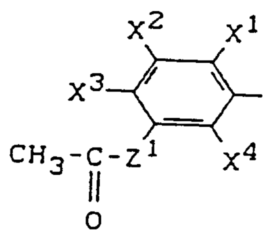

ou

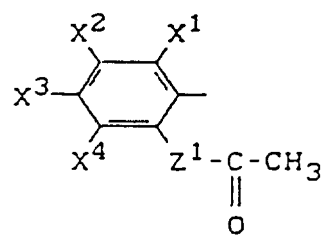

dans lesquels

$Z^1$ représente respectivement un atome d'oxygène, un groupe NH ou un groupe

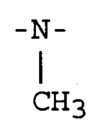

et

$X^1$, $X^2$, $X^3$ et $X^4$ ont la signification indiquée ci-dessus,

avec du méthanol en présence d'une base et ensuite, en présence d'un acide à des températures entre 0 °C et 50 °C, puis on fait réagir les dérivés d'ester acrylique que l'on obtient de cette manière, répondant à la formule (X)

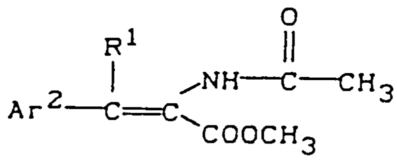

(X)

dans laquelle

$R^1$ a la signification indiquée ci-dessus et

$Ar^2$ représente un des radicaux

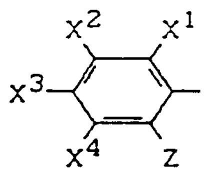

ou

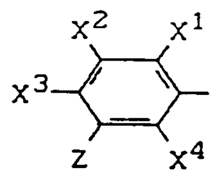

dans lesquels

Z représente respectivement un groupe hydroxyle, un groupe amino ou un groupe N-méthylamino et

$X^1$, $X^2$, $X^3$ et $X^4$ ont la signification indiquée ci-dessus,

avec un sel d'addition d'acide de la O-méthylhydroxylamine, éventuellement en présence d'un diluant et éventuellement en présence d'un adjuvant réactionnel par analogie à la mise en oeuvre du procédé (a) selon l'invention à des températures entre 20 ° C et 60 ° C.